(19) Europäisches Patentamt
European Patent Office
Office européen des brevets

(11) EP 3 486 244 A1

(12) # EUROPEAN PATENT APPLICATION
published in accordance with Art. 153(4) EPC

(43) Date of publication:
**22.05.2019 Bulletin 2019/21**

(21) Application number: **17812596.9**

(22) Date of filing: **05.06.2017**

(51) Int Cl.:
***C07D 487/04*** *(2006.01)* ***A61K 31/519*** *(2006.01)*
***A61K 31/5377*** *(2006.01)* ***A61P 35/00*** *(2006.01)*

(86) International application number:
**PCT/CN2017/087222**

(87) International publication number:
**WO 2017/215485 (21.12.2017 Gazette 2017/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **16.06.2016 CN 201610428765**

(71) Applicant: **Shanghai Institute of Materia Medica, Chinese Academy of Sciences**
**Shanghai 201203 (CN)**

(72) Inventors:
- **DUAN, Wenhu**
  **Shanghai 201203 (CN)**
- **GENG, Meiyu**
  **Shanghai 201203 (CN)**
- **WANG, Yuming**
  **Shanghai 201203 (CN)**
- **AI, Jing**
  **Shanghai 201203 (CN)**
- **FAN, Jun**
  **Shanghai 201203 (CN)**
- **DAI, Yang**
  **Shanghai 201203 (CN)**
- **DING, Jian**
  **Shanghai 201203 (CN)**

(74) Representative: **Predazzi, Valentina**
**Società Italiana Brevetti S.p.A.**
**Piazza di Pietra, 39**
**00186 Roma (IT)**

(54) **NEW COMPOUND HAVING FGFR INHIBITORY ACTIVITY AND PREPARATION AND APPLICATION THEREOF**

(57) The present invention relates to a new compound having an FGFR inhibitory activity and preparation and application thereof. In particular, the compound according to the present invention has a structure as shown in formula I, wherein each group and substituent are as defined in the description. Also disclosed in the present invention are a preparation method for the compound and a use thereof in preparation of a drug for treating and/or preventing a tumor-related disease and/or an FGFR-related disease.

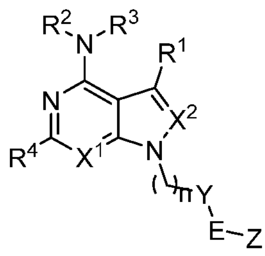
I

EP 3 486 244 A1

## Description

### TECHNICAL FIELD

The present invention relates to the field of medicine, and in particular to a novel compound having FGFR inhibitory activity and preparation and application thereof.

### BACKGROUND ART

Protein kinase is a protein (enzyme) that regulates various cellular functions by phosphorylation of specific amino acids on proteins. Proteins regulate activity and ability to bind to other components by changing their conformation. The activity of a protein kinase refers to the rate at which a kinase binds a phosphate group to a substrate, and the rate can be determined by detecting the amount of substrate converted to a product over a period of time. Phosphorylation of the substrate occurs at activation sites of the protein kinase.
Tyrosine kinase is a protein enzyme that catalyzes the transfer of adenosine triphosphate to tyrosine residue of a protein. These kinases play an important role in growth factor-induced cell proliferation, differentiation and migration.
Fibroblast growth factor (FGF) has been identified to have important regulatory roles in many physiological processes, such as organogenesis and angiogenesis. It is known that there are more than 25 subtypes in the FGF family, and the fibroblast growth factor receptor (FGFR) family contains four subtypes (FGFR1-4), all of which are glycoproteins, including extracellular immunoglobulin-like region, transmembrane hydrophobic region and cytoplasmic tyrosine kinase region. Binding of FGF initiates FGFR dimerization, which in turn leads to autophosphorylation of the receptor and activation of downstream signaling pathways. Certain specific components of the downstream signaling pathway play a very important role in cell growth, metabolism and survival. Therefore, the FGFR signaling pathway plays a pleiotropic and important physiological role in tumor cell reproduction, migration, infiltration and angiogenesis.
Currently, there are evidences indicating that the FGF signaling pathway is directly associated with human cancer. Different FGF overexpression phenomena have been reported in different types of cancer cells (bladder cancer, kidney cancer, prostate cancer, etc.). Therefore, the FGF signaling pathway is a promising therapeutic target.
In summary, there is an urgent need in the art to develop new FGFR inhibitors.

### SUMMARY OF THE INVENTION

The object of the present invention is to provide a novel compound having FGFR inhibitory activity and its preparation and application.
In the first aspect of the invention, a compound of formula I or a stereoisomer, a geometric isomer, a tautomer thereof, a pharmaceutically acceptable salt thereof, a prodrug thereof and a hydrate or solvate thereof is provided,

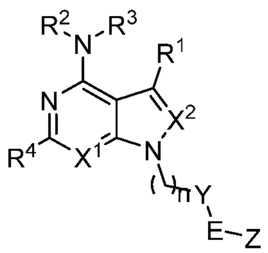
I

wherein $R^1$ is selected from the group consisting of substituted or unsubstituted 5-14 membered heteroaryl containing 1-3 heteroatoms selected from S, O, N and Se and substituted or unsubstituted 6-14 membered aryl, and the "substituted" refers to being substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halocycloalkyl), -$NR^6R^7$, halogen, -(C1-C6 alkylene)-L1, C(=O)$R^8$; and when $R^1$ is a N-containing 5-14 membered heteroaryl, $R^1$ is not a group selected from the group consisting of unsubstituted quinolyl and unsubstituted isoquinolyl;
$R^2$ and $R^3$ may be the same or different and are respectively independently selected from: H, substituted or unsubstituted C1-C6 alkyl, substituted or unsubstituted C3-C8 cycloalkyl, C(=O)$R^8$ and $S(=O)_2R^9$;
$R^4$ is selected from: H, substituted or unsubstituted C1-C6 alkyl, substituted or unsubstituted C3-C8 cycloalkyl, $NR^6R^7$, halogen, hydroxy, cyano, substituted or unsubstituted C1-C6 alkoxy and substituted or unsubstituted C1-C6 alkylthio;

$X^1$ and $X^2$ may be the same or different and are respectively independently selected from: N and $CR^{10}$; $R^{10}$ is selected from: H, substituted or unsubstituted C1-C6 alkyl, substituted or unsubstituted C3-C8 cycloalkyl, halogen, hydroxy, cyano, substituted or unsubstituted C1-C6 alkoxy and substituted or unsubstituted C1-C6 alkylthio;
n is 0, 1, 2, 3, 4 or 5;
Y is selected from: substituted or unsubstituted 3-10 membered heterocyclyl containing 1-3 heteroatoms selected from N, O or S, $-NR^6R^7$, substituted or unsubstituted C3-C8 cycloalkyl and -L2-(substituted or unsubstituted C6-C10 aryl)-, and the "substituted" refers to being independently substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halogenated cycloalkyl), $NR^{11}R^{12}$, halogen, 4-10 membered heterocyclyl containing 1-3 heteroatoms selected from N, O or S, -(C1-C6 alkylene)-L1, $C(=O)R^8$ and -Boc,
E is selected from the group consisting of none, C(=O), $S(=O)_2$, C(=S) and S(=O);
Z is selected from the group consisting of none, substituted or unsubstituted C2-C6 alkenyl, substituted or unsubstituted C2-C6 alkynyl, substituted or unsubstituted C1-C6 alkyl, substituted or unsubstituted C3-C8 cycloalkyl, $-(R^{13})-N(R^{11})-(R^{14})$-(substituted or unsubstituted C1-C6 alkoxy);
$R^6$ and $R^7$ may be the same or different and are respectively independently selected from H, substituted or unsubstituted C1-C6 alkyl, substituted or unsubstituted C3-C8 cycloalkyl, $C(=O)R^8$, -(C1-C6 alkylene)-L1, -(C1-C6 alkylene)-(substituted or unsubstituted 4-8 membered heterocyclyl containing 1-3 heteroatoms selected from N, O or S), -CN, halogen, -OH, -(C1-C6 alkylene)-(substituted or unsubstituted C4-C8 cycloalkyl), or -(C1-C6 alkylene)-L2-(C1-C6 alkylene)-(substituted or unsubstituted C1-C6 alkoxy);
$R^8$ and $R^9$ may be the same or different and are respectively independently selected from: H, hydroxy, substituted or unsubstituted C1-C6 alkyl, substituted or unsubstituted C3-C8 cycloalkyl, substituted or unsubstituted C1-C6 alkoxy, substituted or unsubstituted C2-C4 alkenyl, substituted or unsubstituted C2-C4 alkynyl, -L2-(C1-C6 alkylene)-L1 and $-NR^{11}R^{12}$,
L1 is selected from -OH, C1-C4 alkoxy, $-NR^{11}R^{12}$, or a 4-7 membered heterocyclyl having 1 or 2 N atoms;
L2 is selected from the group consisting of $-NR^{11}-$ and -N (substituted or unsubstituted C3-C6 cycloalkyl);
for $R^2$ to $R^{10}$, $X^1$, $X^2$, E, Z and L2, the "substituted" refers to being independently substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halogenated cycloalkyl), $NR^{11}R^{12}$, halogen, -CN, 4-10 membered heterocyclyl containing 1-3 heteroatoms selected from N, O or S, -(C1-C6 alkylene)-L1 and $C(=O)R^8$; wherein the 4-10 membered heterocyclyl may optionally have 1 to 3 substituents selected from the group consisting of C1-C3 alkyl, halogen, -OH, C1-C3 haloalkyl and C3-C4 cycloalkyl;
$R^{11}$ and $R^{12}$ are independently selected from H, C1-C6 alkyl, C1-C6 haloalkyl, C3-C6 cycloalkyl, C3-C6 halocycloalkyl, -CO(C2-C4 alkenyl), or -CO(C2-C4 alkynyl); or $R^{11}$ and $R^{12}$ together with an adjacent N constitute a 4-7 membered heterocyclyl containing 1-2 N atoms and 0-2 O or S atoms;
$R^{13}$ and $R^{14}$ are independently selected from substituted or unsubstituted C1-C6 alkylene or substituted or unsubstituted C2-C6 alkenylene.

In another preferred embodiment, when Z is a C2-C6 alkenyl having 1, 2 or 3 substituents AA, wherein the substituent AA is selected from the group consisting of $NR^{11}R^{12}$, -(C1-C6 alkylene)-L1, C3-C8 cycloalkyl, halogenated C3-C8 cycloalkyl, -CN, halogen.

In another preferred embodiment, when Z is a C2-C4 alkenyl substituted with 1-2 substituents AA, wherein the substituent AA is selected from the group consisting of $NR^{11}R^{12}$, -(C1-C6 alkylene)-L1 , C3-C8 cycloalkyl, -CN, halogen.

In another preferred embodiment, when Z has the following structure:

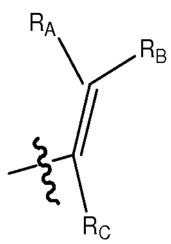

wherein $R_A$, $R_B$ and $R_C$ are independently selected from the group consisting of H, the above substituent AA; and, not all of $R_A$, $R_B$ and $R_C$ are H.

In another preferred embodiment, $R_A$ or $R_B$ is a substituent AA.

In another preferred embodiment, $R_C$ is a substituent AA.

In another preferred embodiment, $R^1$ is a bicyclic group having the following formula:

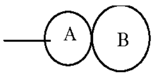 or 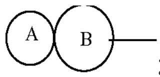 ;

wherein ring A is a substituted or unsubstituted 5-membered heteroaryl ring; and ring B is a substituted or unsubstituted 6-membered heteroaryl ring or a substituted or unsubstituted phenyl.

In another preferred embodiment, $R^1$ is a bicyclic group having the following formula:

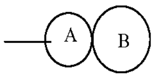.

In another preferred embodiment, the ring A is a 5-membered heteroaryl ring containing S. In another preferred embodiment, $R^1$ is a bicyclic group having the following formula:

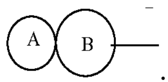.

In another preferred embodiment, the ring A is a 5-membered heteroaryl ring containing one or two N atoms.

In another preferred embodiment, $R^1$ is a substituted 5-14 membered heteroaryl or substituted 6-14 membered aryl.

In another preferred embodiment, $R^1$ is a substituted 5-14 membered heteroaryl or substituted 6-14 membered aryl, and the "substituted" refers to having one or more substituents selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halocycloalkyl), $-NR^6R^7$, halogen, $C(=O)R^8$.

In another preferred embodiment, $R^1$ is a substituted 5-14 membered heteroaryl or substituted 6-14 membered aryl, and the "substituted" refers to having one or more substitutions selected from the group consisting of C1-C6 alkyl, hydroxy, C1-C6 alkoxy, halogenated C1-C6 alkoxy, $-NH_2$, halogen.

In another preferred embodiment, for R1, the "substituted" refers to having at least one substituent selected from the group consisting of C1-C6 alkoxy, halogenated C1-C6 alkoxy.

In another preferred embodiment, for R1, the "substituted" refers to having at least one substituent selected from the group consisting of C1-C6 alkoxy, halogenated C1-C6 alkoxy, hydroxyl and $-NH_2$; and having at least one substituent selected from the group consisting of C1-C6 alkyl, C3-C8 cycloalkyl and halogen.

In another preferred embodiment, $R^1$ is a substituted group selected from the group consisting of benzothienyl, benzofuranyl, indolyl, benzimidazolyl, benzoselenophenyl, indazolyl and benzothiazolyl.

In another preferred embodiment, R1 is a substituted benzothienyl.

In another preferred embodiment, R1 is a substituted benzothienyl, and has a substituent selected from the group consisting of C1-C6 alkoxy, halogenated C1-C6 alkoxy, hydroxyl and $-NH_2$ at position 7.

In another preferred embodiment, the compound of formula I is selected from the compounds listed in Table 1.

In the second aspect of the present invention, a pharmaceutical composition is provided, comprising a therapeutically effective amount of one or more of the compound or a stereoisomer, a geometric isomer, a tautomer thereof, a pharmaceutically acceptable salt, a prodrug thereof and a hydrate or solvate thereof according to the first aspect of the present invention and optional a pharmaceutically acceptable carrier.

In another preferred embodiment, the pharmaceutical composition is a pharmaceutical composition for preventing and/or treating cancer, or a pharmaceutical composition for preventing and/or treating a FGFR-related disease.

In another preferred embodiment, the FGFR is selected from the group consisting of FGFR1, FGFR2, FGFR3, FGFR4 and a combination thereof.

In another preferred embodiment, the pharmaceutical composition is used for preventing and/or treating a disease associated with abnormal expression of the FGF/FGFR signaling pathway.

In another preferred embodiment, the dosage form of the pharmaceutical composition is selected from the group consisting of an oral dosage form, a lyophilized preparation and an injection.

In the third aspect of the invention, a use of the compound or a stereoisomer, a geometric isomer, a tautomer thereof, a pharmaceutically acceptable salt thereof, a prodrug thereof and a hydrate or solvate thereof according to the first aspect of the invention or the pharmaceutical composition according to the second aspect of the invention is provided,

for the preparation of a medicament for preventing and/or treating a disease selected from the group consisting of:

a) tumor-related diseases; and
b) diseases associated with protein tyrosine kinase activity.

In another preferred embodiment, the tumor-related disease is selected from the group consisting of breast cancer, lung cancer, bladder cancer, gastric cancer, pancreatic cancer, prostate cancer, colon cancer, multiple myeloma AML, liver cancer, melanoma, head and neck cancer, thyroid cancer, renal cell carcinoma, glioblastoma and testicular cancer.

In another preferred embodiment, the lung cancer is non-small cell lung cancer.

In another preferred embodiment, the disease associated with protein tyrosine kinase activity is selected from the group consisting of FGFR-related diseases.

In the fourth aspect of the invention, a FGFR inhibitor is provided, comprising an inhibitorically effective amount of one or more of the compound or a stereoisomer, a geometric isomer, a tautomer thereof, a pharmaceutically acceptable salt thereof, a prodrug thereof, and a hydrate or solvate thereof according to the first aspect of the invention.

In another preferred embodiment, the FGFR inhibitor is a FGFR1 inhibitor and a FGFR4 inhibitor.

In the fifth aspect of the present invention, a preparation method for the compound or a stereoisomer, a geometric isomer, a tautomer thereof, a pharmaceutically acceptable salt thereof, a prodrug thereof and a hydrate or solvate thereof according to the first aspect of the present invention is provided, comprising the following steps:
(i) in an inert solvent, subjecting a compound of formula 4 or a salt thereof and an acyl halide compound or an acid of formula 15 to a condensation reaction to form a compound of formula I;

| | | |
|---|---|---|
| Z-E-X 15 | + | 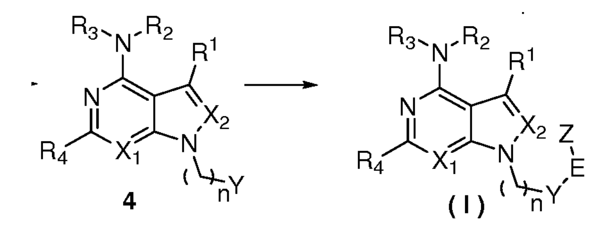 4 (I) |

in various formulas, $R^1$, $R^2$, $R^3$, $R^4$, n, $X_1$, $X_2$, Y, E and Z are as defined above; X is selected from the group consisting of halogen and hydroxyl.

In another preferred embodiment, the reaction in step (i) is carried out under basic conditions, preferably in the presence of triethylamine, pyridine, N,N-diisopropylethylamine, 4-dimethylaminopyridine or N-methylmorpholine and the like.

In another preferred embodiment, the acyl halide compound is preferably an acyl chloride.

In another preferred embodiment, the acid is a carboxylic acid.

In the sixth aspect of the invention, a preparation method for the pharmaceutical composition according to the second aspect of the invention is provided, comprising the steps of: mixing pharmaceutically acceptable carrier and the compound or a stereoisomer, a geometric isomer, a tautomer thereof, a pharmaceutically acceptable salt thereof, a prodrug thereof, and a hydrate or solvate thereof according to the first aspect of the invention to form the pharmaceutical composition.

In the seventh aspect of the invention, a method for non-diagnostically, non-therapeutically inhibiting FGFR activity is provided, comprising administering to a patient in need thereof an inhibitory effective amount of the compound of the first aspect of the invention or a stereoisomer, a geometric isomer, a tautomer thereof, a pharmaceutically acceptable salt thereof, a prodrug thereof, and a hydrate or solvate thereof or the pharmaceutical composition according to the second aspect of the invention.

It should be understood that within the scope of the present invention, the above various technical features of the present invention and the technical features specifically described in the following (as in the embodiment) may be combined with each other to constitute a new or preferred technical solution which will not redundantly be described one by one herein.

## DETAILED DESCRIPTION OF INVENTION

Through extensive and intensive long research, the inventors have unexpectedly prepared a compound of formula I which is novel in structure and has a significant FGFR inhibitory effect. Compared to existing FGFR inhibitors, the FGFR inhibitor prepared with the compound of the present invention can achieve significant inhibition on activities

of FGFR1-4 enzyme at nM level, and the inhibitor also has a significant inhibitory effect on various cancer cell proliferation induced by FGFR1-4 at the cellular level, which is of great significance for the development of new anti-tumor drugs. On this basis, the inventors have completed the present invention.

## TERMS

In this context, unless otherwise stated, the term "substituted" means that one or more hydrogen atoms on a group is replaced with a substituent selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halocycloalkyl), halogen, 4-10 membered heterocyclyl containing 1-3 heteroatoms selected from S, O, N and Se, amino, phenyl, cyano, C2-C6 alkenyl, C2-C6 alkynyl; and the phenyl comprises unsubstituted phenyl or substituted phenyl having 1-3 substituents selected from: halogen, C1-C6 alkyl, C3-C8 cycloalkyl, C1-C6 alkoxy, OH, cyano, nitro, amino.

Unless otherwise stated, among all compounds of the present invention, each chiral carbon atom may be optionally R configuration or S configuration, or a mixture of R configuration and S configuration.

The term "5-14 membered heteroaryl" refers to a group formed by the loss of one hydrogen atom from a 5-14 membered aryl group having from 1 to 3 heteroatoms selected from the group consisting of N, S, O, Se, wherein ring systems of every heteroaryl may be monocyclic or polycyclic; for example, benzothienyl, benzofuranyl, indolyl, naphthyl, benzimidazolyl, benzoselenophenyl, pyridyl, furanyl, phenyl, indazolyl, thienyl, pyrrolyl, imidazolyl, pyrazolyl, pyrazinyl, oxazolyl, thiazolyl, benzothiazolyl, or the like.

The term "6-14 membered aryl" refers to a group formed by the loss of one hydrogen atom from a 6-14 membered aryl; for example, phenyl, naphthyl, or the like.

The term "C1-C6 alkyl" refers to a straight or branched alkyl having from 1 to 6 carbon atoms, such as methyl, ethyl, propyl, isopropyl, butyl, isobutyl, sec-butyl, tert-butyl, or the like.

The term "C1-C6 alkoxy" means a straight or branched alkoxy having from 1 to 6 carbon atoms, such as methoxy, ethoxy, propoxy, isopropoxy, butoxy, isobutoxy, sec-butoxy, tert-butoxy, or the like.

The term "C2-C6 alkenyl" refers to a group formed by the loss of one or two hydrogen atoms of an olefin having 2 to 6 carbon atoms, which may be a monoolefin, a diene or a triene, such as $-CH=CH_2$, $-C_2H_4=CH_2$, $-CH=C_2H_4$, or the like.

The term "halogen" refers to F, Cl, Br and I.

In addition, in the present invention, the term "alkyl" includes saturated or unsaturated, linear, branched, cyclic all carbon alkyl having 1-10 carbon atoms or alkyl wherein 1-3 carbon atoms thereof are substituted with a hetero atom such as oxygen, nitrogen or sulfur, and an aralkyl bonded through one or more carbon atoms. Further, the alkyl is unsubstituted or substituted.

As used herein, the term "aryl" includes fused or non-fused aryl, usually containing 6-30 carbon atoms, and representative aryl includes phenyl, naphthyl, or aromatic groups containing heteroatoms such as oxygen, nitrogen, sulfur.

Unless otherwise indicated, the structural formulae described herein are intended to include all isomeric forms (e.g., enantiomers, diastereoisomers and geometric isomers (or conformational isomers): for example, the R, S configuration having asymmetric center, the (Z), (E) isomers of the double bond, and the conformational isomers of (Z) and (E). Thus, a single stereochemical isomer or a mixture of enantiomers, diastereoisomers or geometric isomers (or conformational isomers) thereof of the compound of the invention is within the scope of the invention.

The term "tautomer" means that structural isomers with different energies can exceed the low energy barrier, thus transforming into each other. For example, proton tautomers (i.e. proton shifts) include interconversion by proton transfer, such as 1H-indazole and 2H-indazole, 1H-benzo[d]imidazole and 3H-benzo[d]imidazole, and valency tautomers include interconversion through some bonding electron recombination.

## COMPOUND OF FORMULA I

The present inventors designed and synthesized a series of FGFR inhibitor compounds having novel structures, and through structural optimization, found small molecule FGFR inhibitors with excellent activity in enzymes, cells and animals and with strongly inhibiting effect on FGFR1-4. This series of compounds is expected to be used clinically to treat diseases caused by abnormal expression of FGF/FGFR signaling pathways, such as cancer.

Specifically, the present invention provides a compound of formula I:

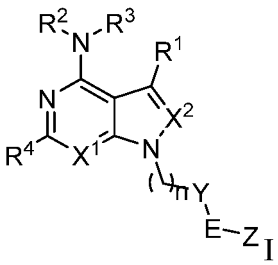

In another preferred embodiment, any one of $R^1$, $R^2$, $R^3$, $R^4$, $R^6$, $R^7$, $R^8$, $R^9$, $R^{10}$, $R^{11}$, $R^{12}$, $R^{13}$, $R^{14}$, $X^1$, $X^2$, L1, L2, Y, E, Z is respectively the corresponding group in each of the specific compounds in the examples.

In another preferred embodiment, the compound is preferably a compound prepared in the examples.

In another preferred embodiment, the compounds NO. 1 - NO. 56 are the compounds obtained in Examples 1-56, respectively.

In another preferred embodiment, the compound is selected from the Compounds listed in Table 1.

Table 1

| | | | |
|---|---|---|---|
| NO.1 | 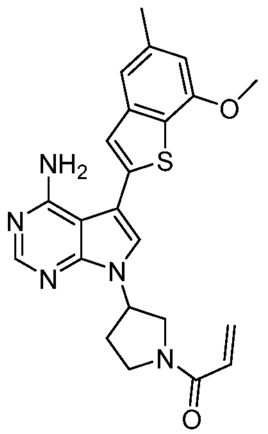 | NO.2 | 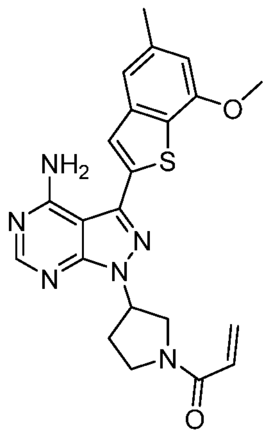 |
| NO.3 | 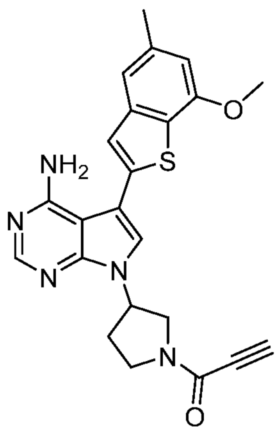 | NO.4 | 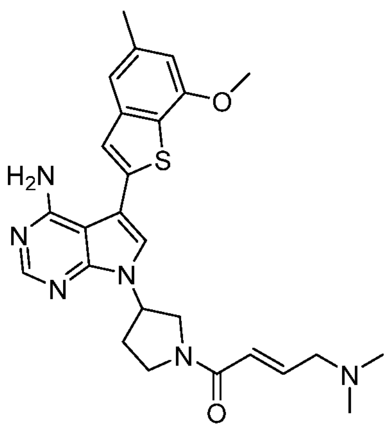 |

(continued)

| | | | |
|---|---|---|---|
| NO.5 | 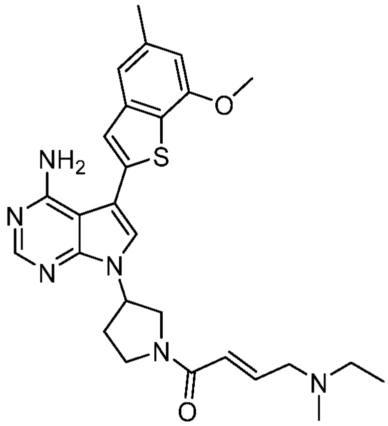 | NO.6 | 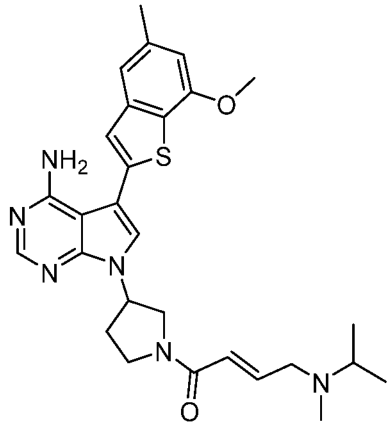 |
| NO.7 | 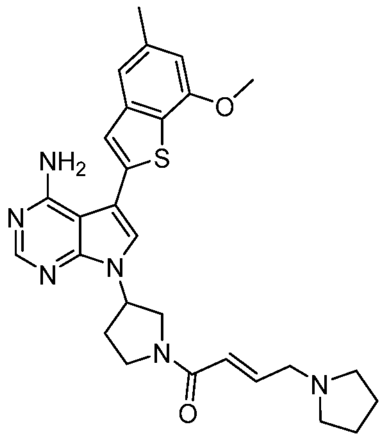 | NO.8 | 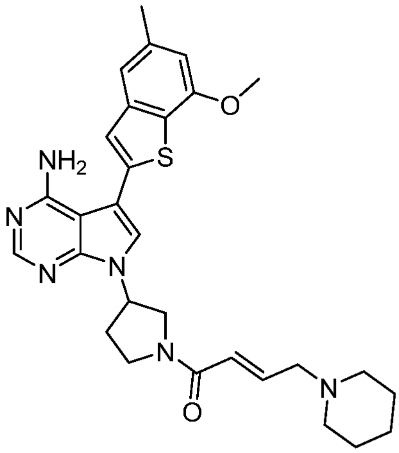 |
| NO.9 | 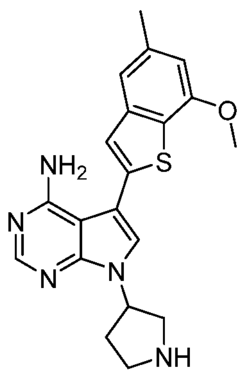 | NO.10 | 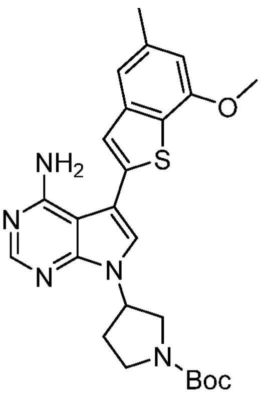 |

(continued)

| | | | |
|---|---|---|---|
| NO.11 | 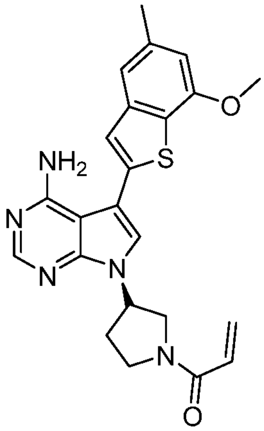 | NO.12 | 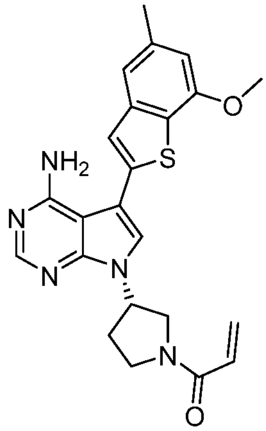 |
| NO.13 | 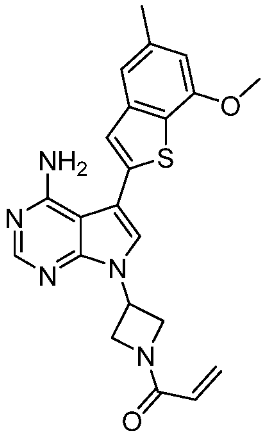 | NO.14 | 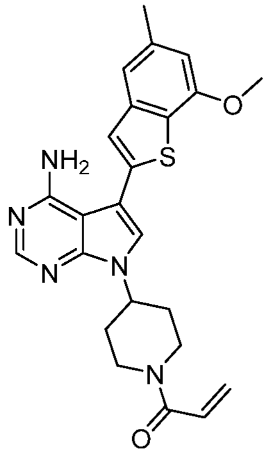 |
| NO.15 | 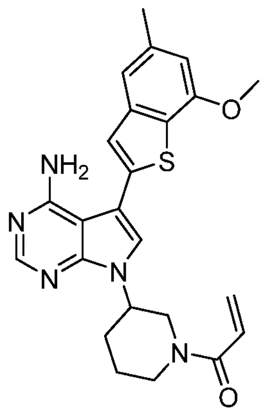 | NO.16 | 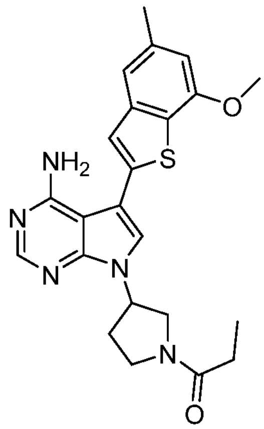 |

(continued)

| | | | |
|---|---|---|---|
| NO.17 | 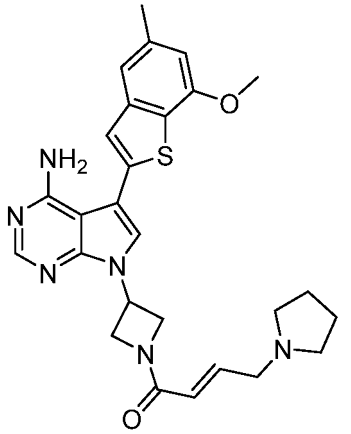 | NO.18 | 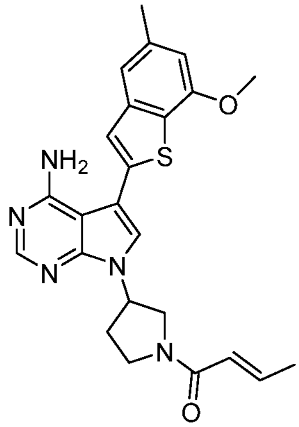 |
| NO.19 | 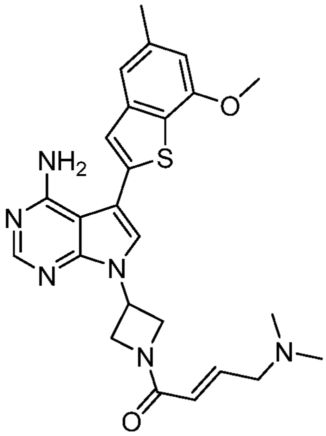 | NO.20 | 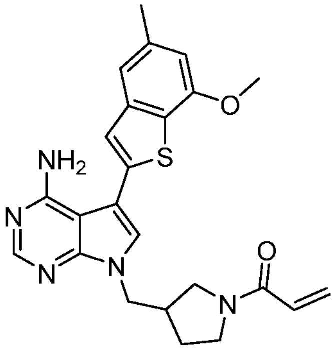 |
| NO.21 | 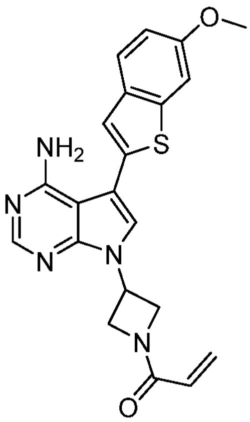 | NO.22 | 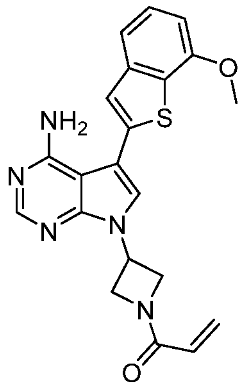 |

(continued)

| | | | |
|---|---|---|---|
| NO.23 | 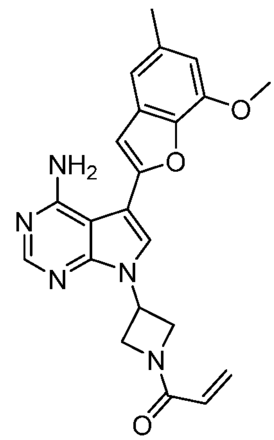 | NO.24 | 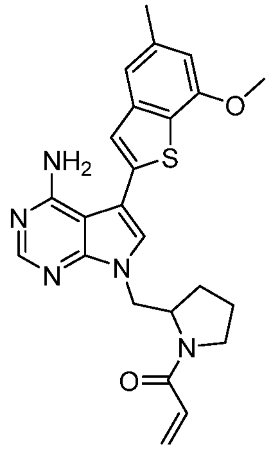 |
| NO.25 | 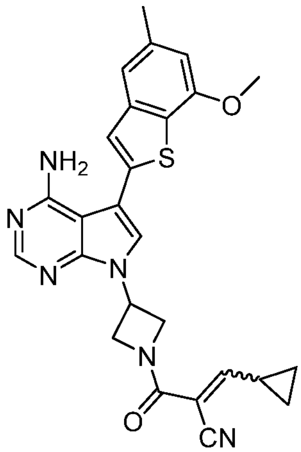 | NO.26 | 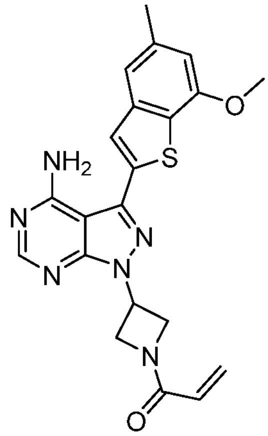 |
| NO.27 | 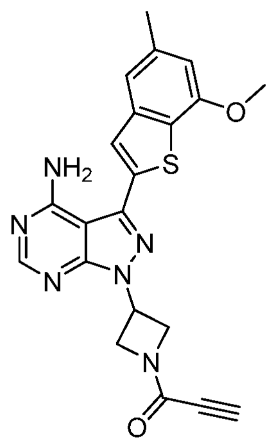 | NO.28 | 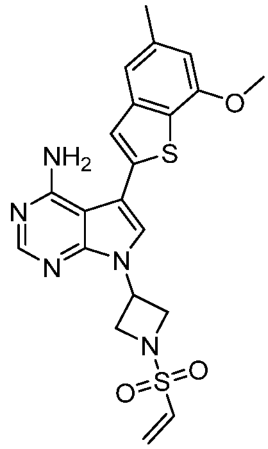 |

(continued)

| | | | |
|---|---|---|---|
| NO.29 | 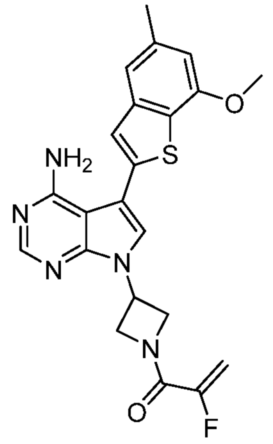 | NO.30 | 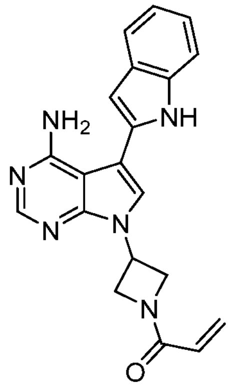 |
| NO.31 | 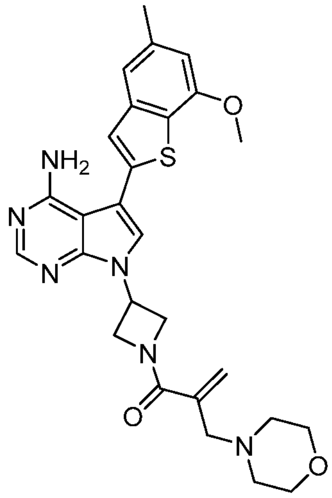 | NO.32 | 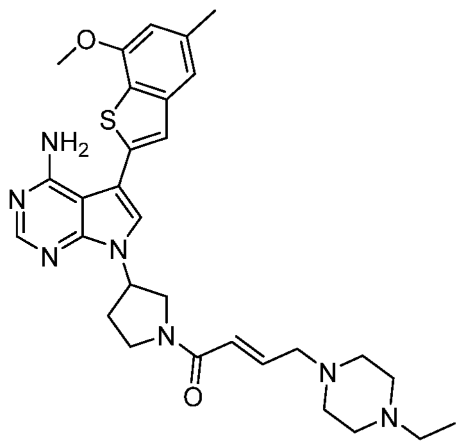 |
| NO.33 | 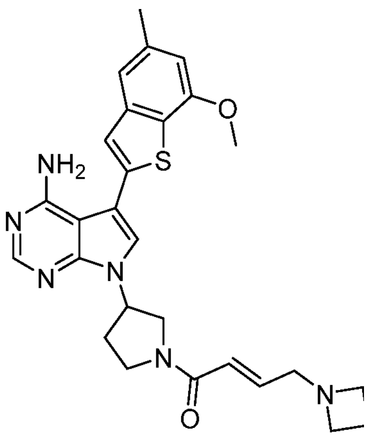 | NO.34 | 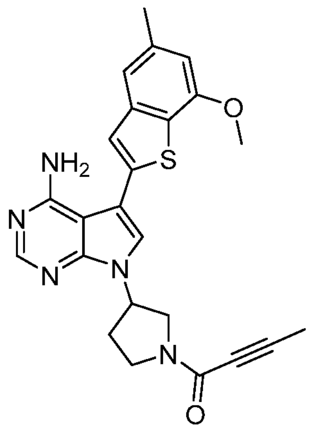 |

(continued)

| | | | |
|---|---|---|---|
| NO.35 | 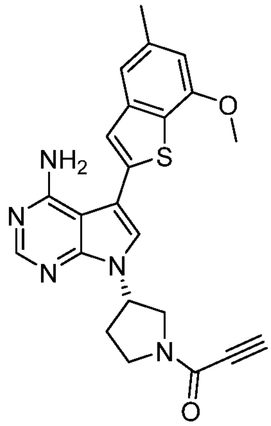 | NO.36 | 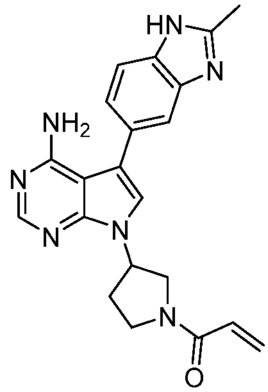 |
| NO.37 | 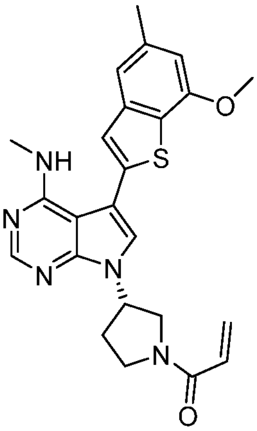 | NO.38 | 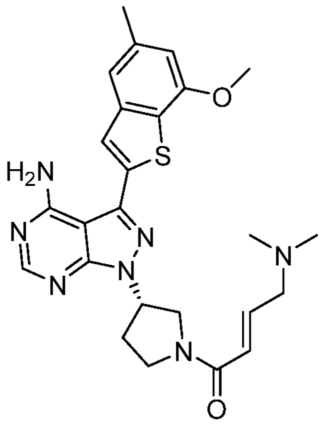 |
| NO.39 | 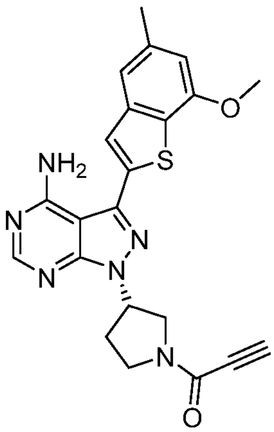 | NO.40 | 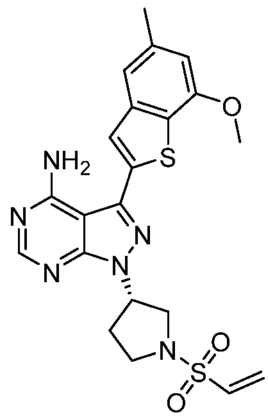 |

(continued)

| | | | |
|---|---|---|---|
| NO.41 | 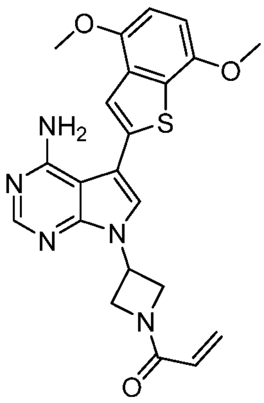 | NO.42 | 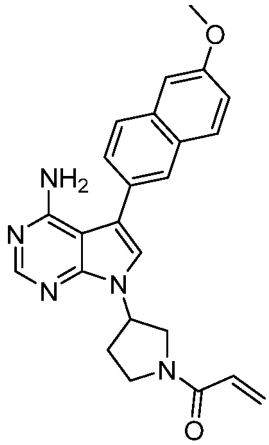 |
| NO.43 | 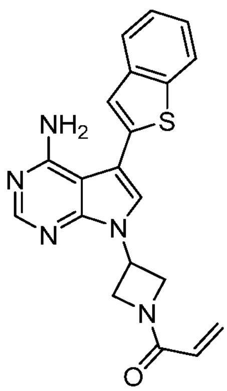 | NO.44 | 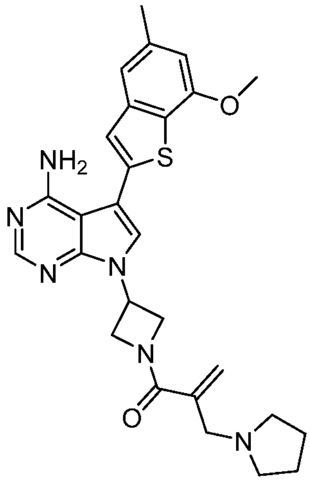 |
| NO.45 | 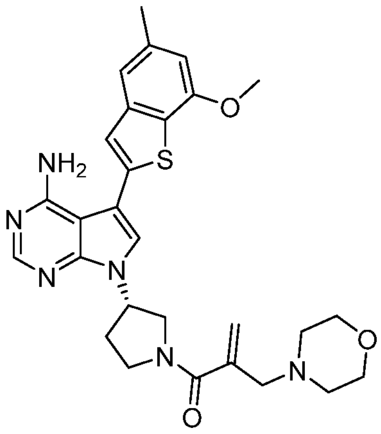 | NO.46 | 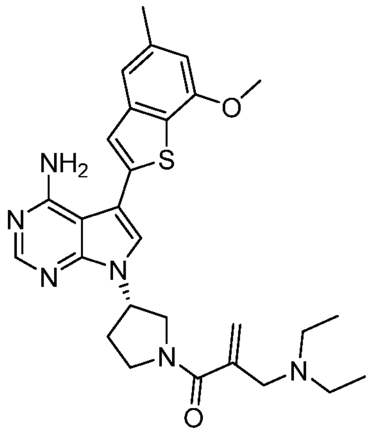 |

(continued)

| | | | |
|---|---|---|---|
| NO.47 | 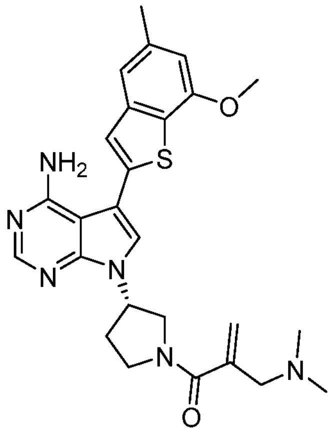 | NO.48 | 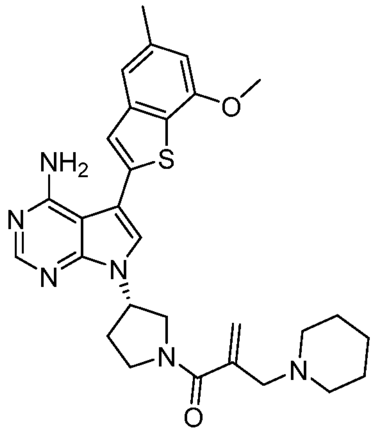 |
| NO.49 | 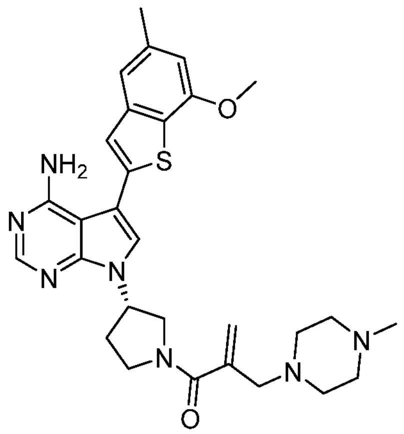 | NO.50 | 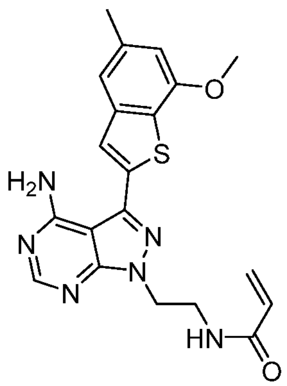 |
| NO.51 | 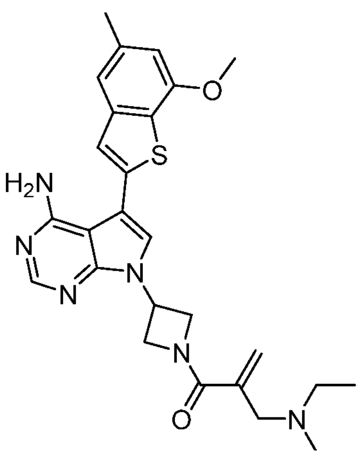 | NO.52 | 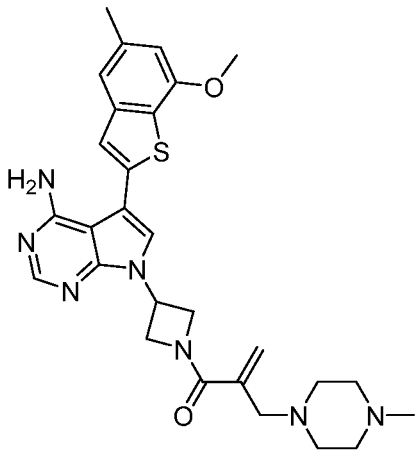 |

(continued)

| | | | |
|---|---|---|---|
| NO.53 | 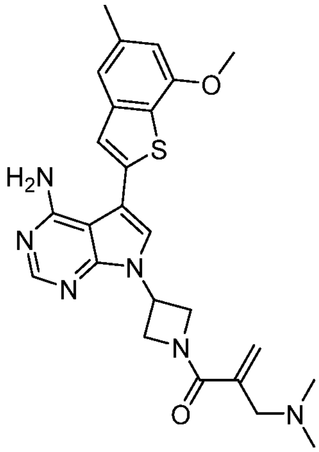 | NO.54 | 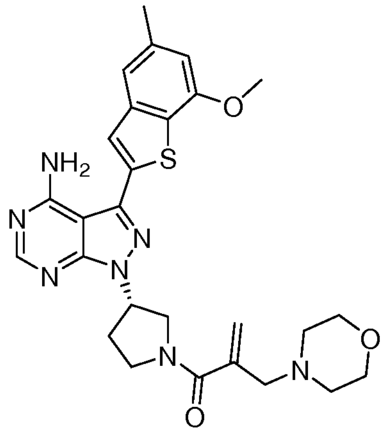 |
| NO.55 | 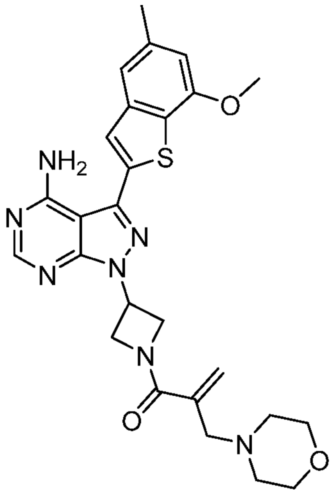 | NO.56 | 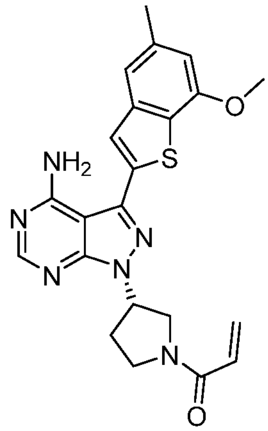 |
| NO.57 | 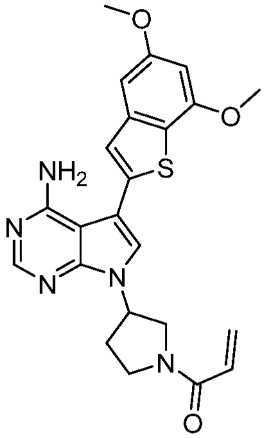 | NO.58 | 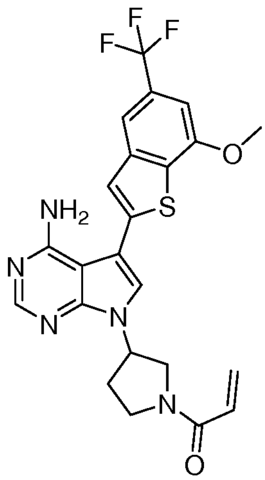 |

(continued)

| | | | |
|---|---|---|---|
| NO.59 | 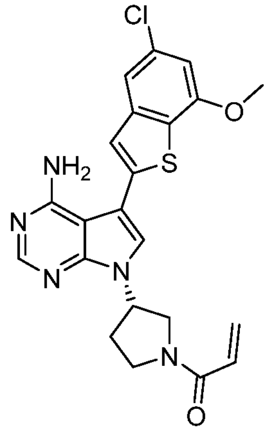 | NO.60 | 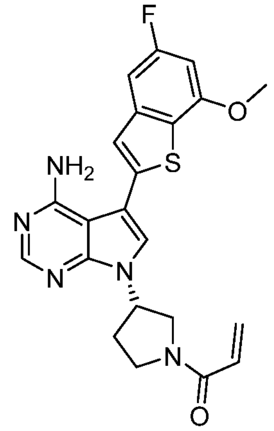 |
| NO.61 | 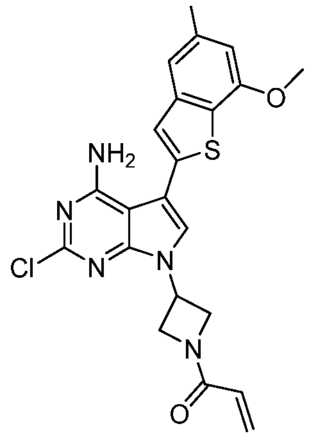 | NO.62 | 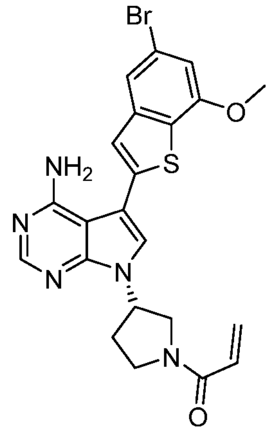 |
| NO.63 | 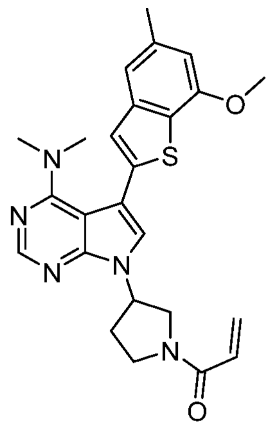 | NO.64 | 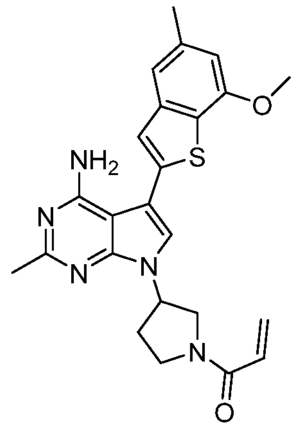 |

(continued)

| | | | |
|---|---|---|---|
| NO.65 | 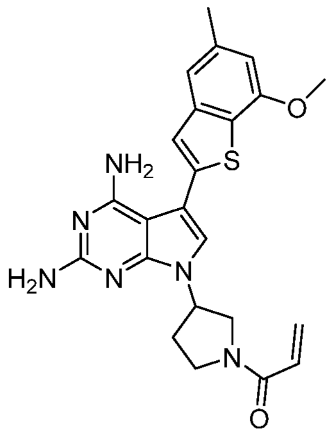 | NO.66 | 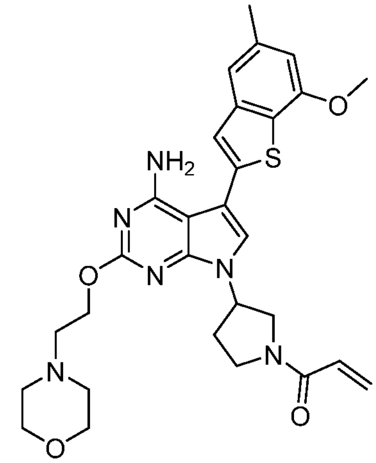 |
| NO.67 | 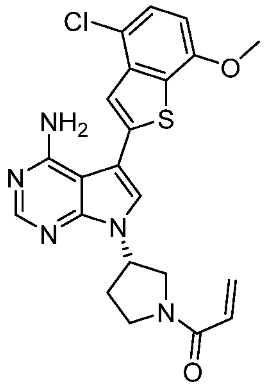 | NO.68 | 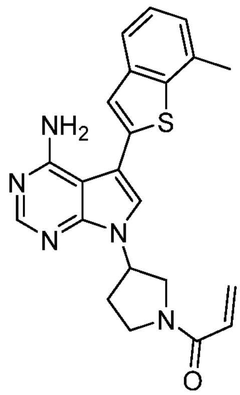 |
| NO.69 | 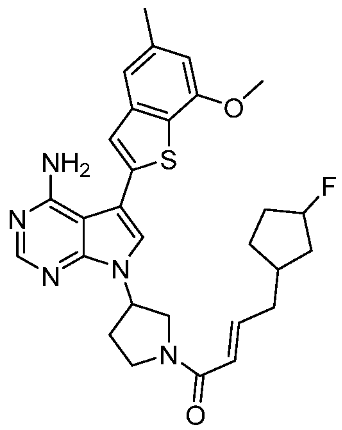 | NO.70 | 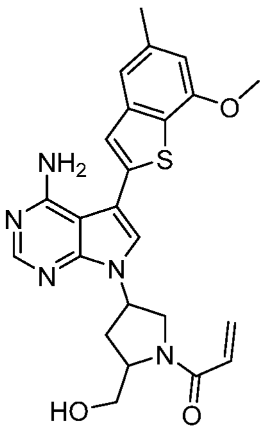 |

(continued)

| | | | |
|---|---|---|---|
| NO.71 | 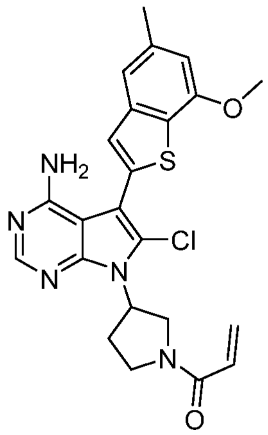 | NO.72 | 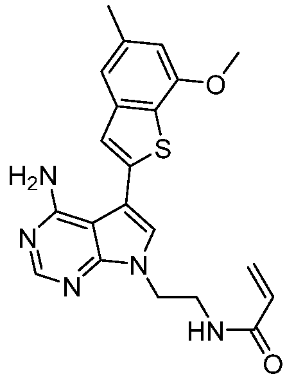 |
| NO.73 | 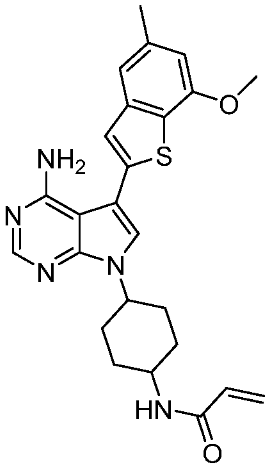 | NO.74 | 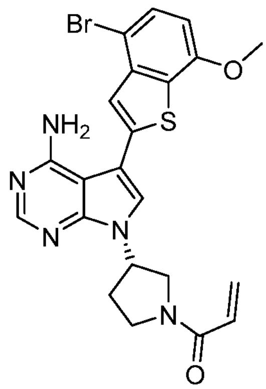 |
| NO.75 | 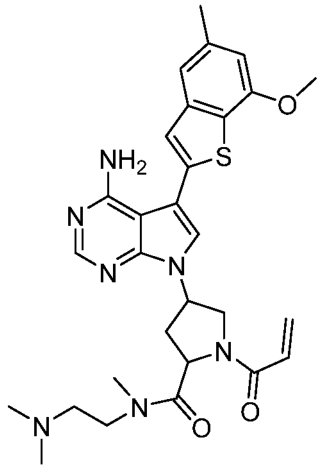 | NO.76 | 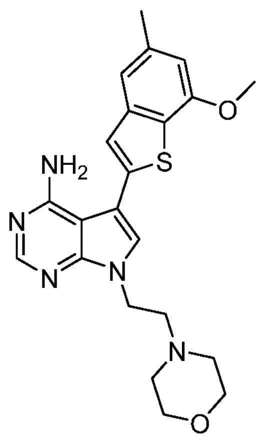 |

(continued)

| | | | |
|---|---|---|---|
| NO.77 | 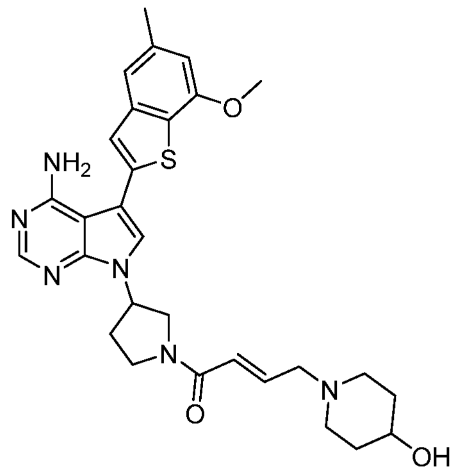 | NO.78 | 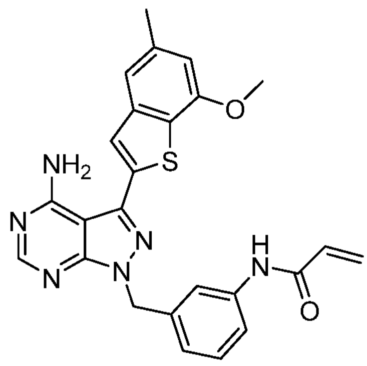 |
| NO.79 | 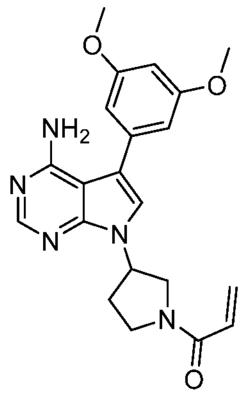 | NO.80 | 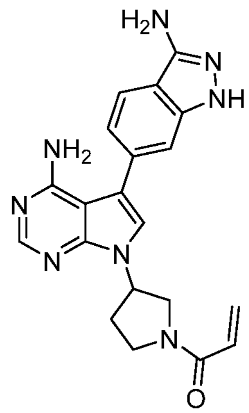 |
| NO.81 | 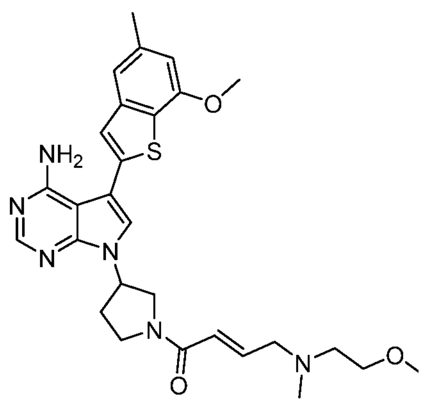 | NO.82 | 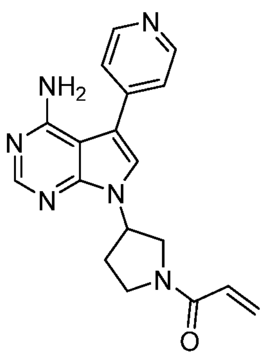 |

(continued)

| | | | |
|---|---|---|---|
| NO.83 | 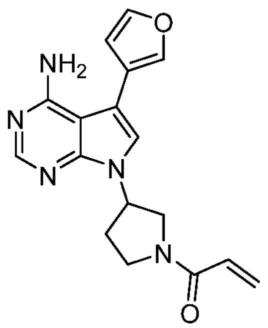 | NO.84 | 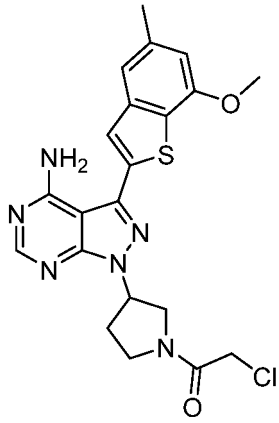 |
| NO.85 | 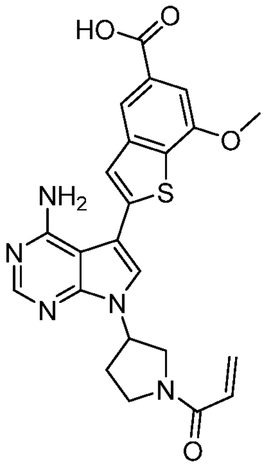 | NO.86 | 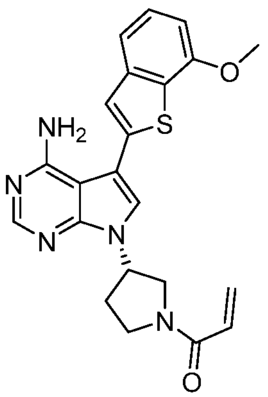 |

In another preferred embodiment, the compound has significant FGFR inhibitory activity.

In another preferred embodiment, the compound is substantially free of EGFR inhibitory activity.

As used herein, the term "pharmaceutically acceptable salt" refers to a salt of the compound of the invention formed with an acid or base which is suitable for use as a medicament. The pharmaceutically acceptable salts include inorganic salts and organic salts. A preferred class of salts is the salts of the compounds of the invention formed with acids. Suitable acids for forming salts include, but are not limited to, inorganic acids such as hydrochloric acid, hydrobromic acid, hydrofluoric acid, sulfuric acid, nitric acid, phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methylsulfonic acid, ethanesulfonic acid, methanesulfonic acid, trifluoromethanesulfonic acid, ethanesulfonic acid, p-toluenesulfonic acid, benzenesulfonic acid, naphthalenesulfonic acid; and amino acids such as proline, phenylalanine, aspartic acid and glutamic acid.

Another preferred class of salts are salts of the compounds of the invention formed with bases, such as alkali metal salts (for example sodium or potassium salts), alkaline earth metal salts (for example magnesium or calcium salts), ammonium salts (such as lower grades alkanol ammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trishydroxyethylamine salt, and an amine salt formed from morpholine, piperazine, and lysine, respectively.

The term "solvate"refers to a complex of the compound of the invention coordinated to a solvent molecule at a particular ratio. "Hydrate" refers to a complex formed by the coordination of the compound of the invention with water.

The term "prodrug" includes compounds which are biologically active or inactive, which, when administered by an appropriate manner, are metabolized or chemically reacted in the human body to form a compound of formula I, or salts or solution consisted of a compound of formula I. The prodrug includes, but is not limited to, a carboxylic acid ester, a carbonate, a phosphate, a nitrate, a sulfate, a sulfone ester, a sulfoxide ester, an amino compound, a carbamate, an azo compound, phosphoramide, glucoside, ether, acetal of the compound and the like.

## PREPARATION METHOD OF THE COMPOUND OF FORMULA I

The preparation method of the formula I structural compound of the present invention is more specifically described below, but these specific methods do not constitute any limitation to the present invention. The compounds of the present invention may also be conveniently prepared by optionally combining various synthetic methods described in the specification or known in the art, and such combinations are readily made by those skilled in the art to which the present invention pertains.

Typically, the preparation process of the compounds of the present invention is as follows, wherein the starting materials and reagents used are commercially available unless otherwise specified.

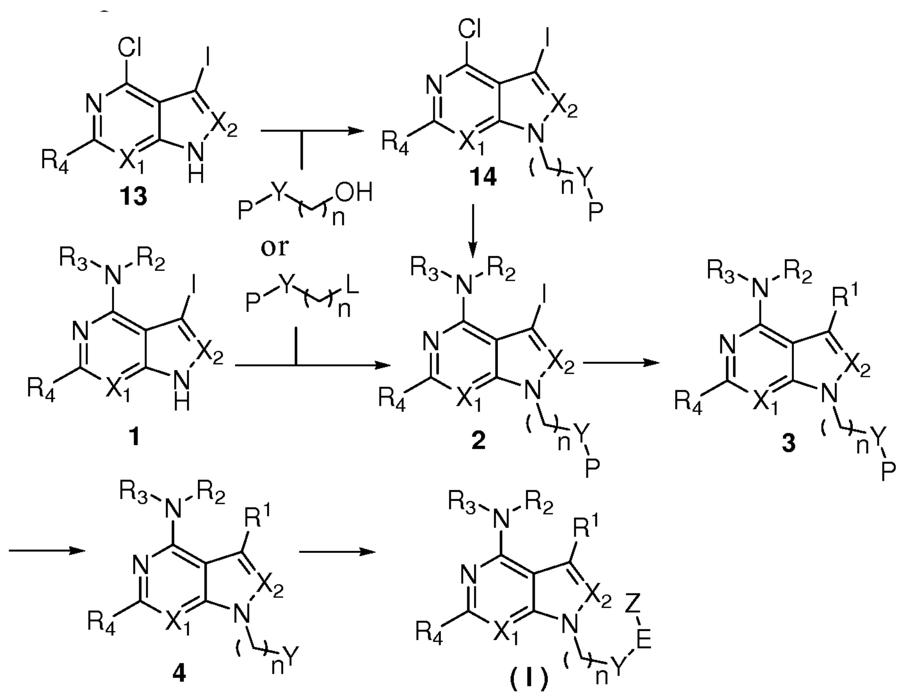

13 14 1 2 3 4 (I)

wherein $R^1$, $R^2$, $R^3$, $R^4$, n, $X_1$, $X_2$, Y, E and Z are as defined above, P is any group that can serve as an amino protecting group, and L is the leaving group when nucleophilic substitution occurs.

Starting from compound 1, compound 2 is prepared by Mitsunobu reaction or nucleophilic substitution connection, and then coupled, and the target product is obtained through deprotecting the protecting group and condensation.

Specifically, the preparation method includes the following steps:

1. compound 2 is formed by Mitsunobu reaction or nucleophilic substitution from compound 1, or compound 14 is formed by Mitsunobu reaction or nucleophilic substitution from compound 13, and compound 2 is formed by the reaction of compound 14 with $NHR_2R_3$ or a salt thereof;
2. compound 2 is coupled with the corresponding boric acid under the action of a palladium catalyst to give compound 3;
3. compound 3 is deprotected under acidic conditions to give compound 4;
4. compound 4 is condensed with the corresponding acyl chloride compound or carboxylic acid to give the target product.

wherein, the intermediate 1 can be prepared by a conventional method in the art, or can be obtained by a commercially available route. In a preferred embodiment of the invention, the compound (1a) is prepared by the following method:

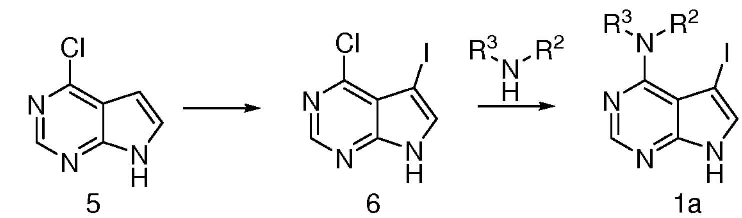

5 6 1a

wherein $R^2$ and $R^3$ are as defined above.

Starting from compound 5, an iodide is made and then aminated to give intermediate (1a).

Specifically, the preparation method includes the following steps:

1. compound 5 produces the corresponding iodide 6 under N-iodosuccinimide conditions;
2. compound 6 is heated or microwaved with a suitable amine (ammonia) in a suitable solvent to give intermediate (1a);

In another preferred embodiment of the invention, the compound (1b) is prepared by the following method:

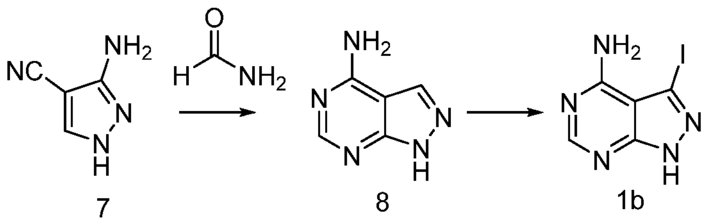

7 8 1b

starting from compound 7, an iodide is made after cyclization to give intermediate (1b).

Specifically, the preparation method includes the following steps:

1. compound 7 is cyclized at 180 degrees in a formamide solvent to form the corresponding 8;
2. compound 8 is reacted with N-iodosuccinimide to give intermediate (1b);

The corresponding boronic acid required in the process for preparing the structure of Formula I can be prepared by conventional methods in the art or is commercially available. In a preferred embodiment of the invention, it is prepared by the following method:

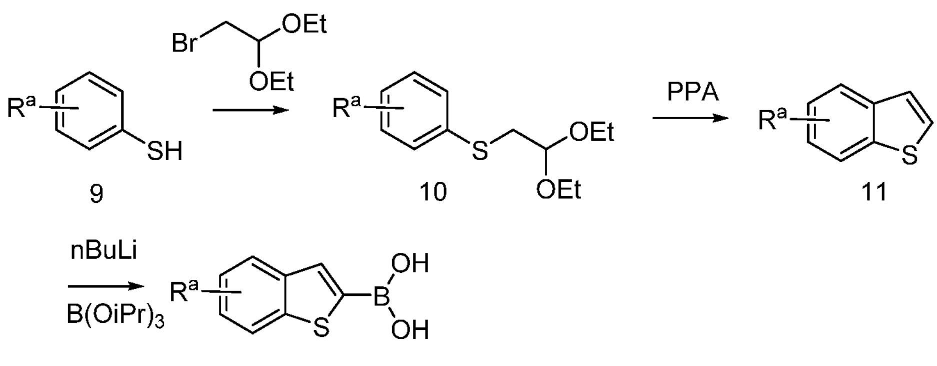


9 10 11

12

$R^a$ is a substituent of the above $R^1$, and the definition thereof is as described above. Starting from compound 9, compound 10 is formed by nucleophilic substitution connection, and then compound 11 is obtained in chlorobenzene under polyphosphoric acid conditions, and the corresponding boric acid is prepared under conventional reaction conditions (n-butyl lithium and triisopropyl borate) from 11.

Specifically, the preparation method includes the following steps:

1. compound 9 produces compound 10 by nucleophilic substitution;
2. compound 10 is refluxed with polyphosphoric acid in a suitable solvent such as chlorobenzene to give compound 11;
3. compound 11 gives boric acid compounds under the condition of n-butyl lithium and triisopropyl borate.

The preparation method for the compound of the invention has the advantages of mild reaction conditions, abundant raw materials, easy operation and post-treatment.

**Pharmaceutical composition and preparation method thereof**

The present invention also provides a pharmaceutical composition comprising a therapeutically effective amount

of one or more of the compound or a stereoisomer, a geometric isomer, a tautomer thereof, a pharmaceutically acceptable salt, a prodrug thereof and a hydrate or solvate thereof and optional a pharmaceutically acceptable carrier.

In another preferred embodiment, the pharmaceutical composition is a pharmaceutical composition for preventing and/or treating cancer, or a pharmaceutical composition for preventing and/or treating a FGFR-related disease.

In another preferred embodiment, the FGFR is selected from the group consisting of FGFR1, FGFR2, FGFR3, FGFR4, or a combination thereof.

In another preferred embodiment, the pharmaceutical composition is used for preventing and/or treating a disease associated with abnormal expression of the FGF/FGFR signaling pathway.

In another preferred embodiment, the dosage form of the pharmaceutical composition is selected from the group consisting of an oral dosage form, a lyophilized preparation and an injection.

Because the compounds of the present invention have excellent inhibitory activity against FGFR kinases such as FGFR1 and FGFR4, the compounds of the present invention and various crystal forms thereof, pharmaceutically acceptable inorganic or organic salts, hydrates or solvates and a pharmaceutical composition comprising the compound of the present invention as a main active ingredient can be used in the treatment, prevention and alleviation of diseases associated with FGFR activity or expression, such as prevention and/or treatment of diseases associated with abnormal expression of the FGF/FGFR signaling pathway. According to the prior art, the compounds of the present invention can be used to treat cancer, and the cancer includes breast cancer, lung cancer, bladder cancer, gastric cancer, pancreatic cancer, prostate cancer, colon cancer, multiple myeloma AML, liver cancer, melanoma, head and neck cancer, thyroid cancer, renal cell carcinoma, glioblastoma and testicular cancer. More specifically, these cancers are selected from breast cancer, non-small cell lung cancer, bladder cancer, gastric cancer, pancreatic cancer, prostate cancer, colon cancer, multiple myeloma, liver cancer, melanoma, head and neck cancer, thyroid cancer, renal cell carcinoma, glioblastoma and testicular cancer. Most particularly, the cancer is non-small cell lung cancer, gastric cancer or multiple myeloma.

The pharmaceutical composition of the present invention comprises a safe and effective amount of a compound of the present invention or a pharmacologically acceptable salt thereof, and a pharmacologically acceptable excipient or carrier. Wherein "safe and effective amount" refers to: the amount of the compound is sufficient to significantly improve the condition, but will not have serious side effects. Generally, the pharmaceutical composition contains 1-2000 mg of the compound of the invention per dose, more preferably 5-200 mg of the compound of the invention per dose. Preferably, the one dose is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginate, gelatin, polyvinylpyrrolidone, sucrose and arabic gum; (c) humectants, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, calcium stearate, magnesium stearate, solid polyethylene glycol, sodium lauryl sulfate, or the mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain any conventional inert diluent known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the mixture thereof etc..

In addition to these inert diluents, the compositions may contain adjuvants such as wetting agents, emulsifying and suspending agents, sweetening agents, flavoring agents and perfume.

In addition to the active compound, the suspension may contain suspending agent, for example, ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, propellants and inhalants. The active ingredient is mixed under sterile conditions with a physiologically acceptable carrier and any preservatives, buffers, or propellants which may be required if necessary.

The compounds of the invention may be administered alone or in combination with other therapeutic means and/or other therapeutic agents.

When a pharmaceutical composition is used, a safe and effective amount of a compound of the invention is applied to a mammal in need of treatment (e.g., human), wherein the dosage is a pharmaceutically acceptable effective dosage for administration. To a human having a weight of 60 kg, the daily dose is usually from 1 to 2000 mg, preferably from 5 to 500 mg. Of course, specific doses should also consider factors such as the administration route, the health of the patient, etc., which are within the skill of the skilled physician.

Furthermore, the present invention also provides a preparation method for the pharmaceutical composition, comprising the steps of: mixing pharmaceutically acceptable carrier and the compound or a stereoisomer, a geometric isomer, a tautomer thereof, a pharmaceutically acceptable salt thereof, a prodrug thereof, and a hydrate or solvate thereof to form the pharmaceutical composition.

**Use**

The present invention also provides a use of the compound or the pharmaceutical composition for the preparation of a medicament for the prevention and/or treatment of a disease selected from the group consisting of:

a) tumor-related diseases;
b) diseases associated with protein tyrosine kinase activity.

In another preferred embodiment, the tumor-related disease is selected from the group consisting of breast cancer, lung cancer, bladder cancer, gastric cancer, pancreatic cancer, prostate cancer, colon cancer, multiple myeloma AML, liver cancer, melanoma, head and neck cancer, thyroid cancer, renal cell carcinoma, glioblastoma and testicular cancer.

In another preferred embodiment, the lung cancer is non-small cell lung cancer.

In another preferred embodiment, the disease associated with protein tyrosine kinase activity is selected from the group consisting of FGFR-related diseases.

Additionally, the present invention provides a FGFR inhibitor comprising an inhibitory effective amount of one or more of the compound or a stereoisomer, a geometric isomer, a tautomer thereof, a pharmaceutically acceptable salt thereof, a prodrug thereof, and a hydrate or solvate thereof.

In another preferred embodiment, the FGFR inhibitor is a FGFR1 inhibitor and a FGFR4 inhibitor.

**Treatment method**

The invention also provides a method for treating FGFR-associated disease, comprising administering to a patient in need thereof an inhibitory effective amount of the compound or a stereoisomer, geometric isomer, tautomer thereof, a pharmaceutically acceptable salt, a prodrug thereof, and a hydrate or solvate thereof or the pharmaceutical composition.

The invention also provides a method for non-diagnostically, non-therapeutically inhibiting FGFR activity, comprising administering to a patient in need thereof an inhibitory effective amount of the compound or a stereoisomer, a geometric isomer, a tautomer thereof, a pharmaceutically acceptable salt thereof, a prodrug thereof and a hydrate or solvate thereof or the pharmaceutical composition.

Compared with the prior art, the present invention has the following main advantages:

(1) the inhibitor prepared by the compound has a significant inhibitory effect on FGFR enzyme activity;
(2) the compound can achieve significant inhibition of FGFR1-4 enzyme activity at nM level;
(3) the compound also has a significant inhibitory effect on FGFR1-4-induced proliferation of various cancer cells at cellular level.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, parts and percentage are calculated by weight.

Unless otherwise defined, all professional and scientific terminology used in the text have the same meanings as known to the skilled in the art. In addition, any methods and materials similar or equal with the record content can apply to the methods of the invention. The method of the preferred embodiment described herein and the material are only for demonstration purposes.

**Example 1:**

**1-(3-(4-amino-5-(7-methoxy-5-methylbenzothiophen-2-yl)-7H-pyrrole[2,3-d]pyrimidine-7-yl)pyrrolidine-1-yl)prop-2-en-1-one**

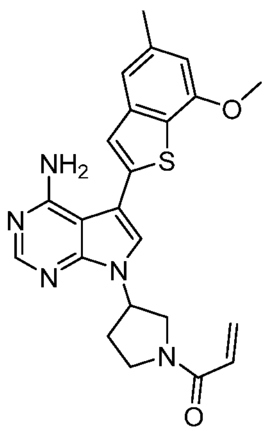

Step 1: Preparation of 2-methoxy-4-methylnitrobenzene

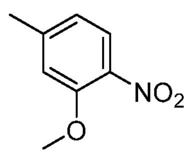

7.5g of 5-methyl-2-nitrophenol and 10.2g of potassium carbonate were suspended in 80 ml of acetone, and then 8.3g of methyl iodide was added. After heating and refluxing for 5 hours, the heating was stopped, and diatomite was used to filter the insoluble. The filtrate was evaperated to dryness *in vacuo* to give 7.7g of orange crystals which were directly used in the next step.

$^1$H NMR (300 MHz, DMSO-$d_6$) $\delta$ 7.76 (d, $J$ = 8.2 Hz,1H), 6.86 (s, 1H), 6.79 (d, $J$ = 8.2 Hz, 1H), 3.92 (s, 3H), 2.39 (s, 3H).

Step 2: Preparation of 2-methoxy-4-methylaniline

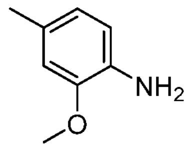

7.7 g of 2-methoxy-4-methylnitrobenzene and 32 g of stannous chloride dihydrate were dissolved in 250 ml of methanol, after heating and refluxing for 3 hours, the reaction was quenched. Most of methanol was evaporated, and the reaction liquid was diluted with ethyl acetate. the reaction mixture was neutralized with a saturated solution of sodium hydrogencarbonate, then the insoluble was filtered and the filtrate was extracted with ethyl acetate. After liquid separation, the organic phase was washed with saturated aqueous solution of sodium chloride and then dried over anhydrous sodium sulfate, filtered and concentrated to give 5.2 g of bronzing oily matter which was directly used in the next step.
$^1$H NMR (300 MHz, DMSO-$d_6$) δ 6.45-6.63 (m, 3H), 4.46 (s, 2H), 3.72 (s, 3H), 2.16 (s, 3H).

Step 3: Preparation of 2-methoxy-4-methylthiophenol

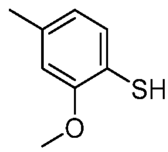

5.2 g of 2-methoxy-4-methylaniline was dissolved in 32 ml of water and 11.4 ml of concentrated hydrochloric acid, cooled to 0-5 °C in an ice bath, and 2.88 g of sodium nitrite dissolved in 9 ml of water was added dropwise to the above cooled solution. The mixture was stirred for 10 minutes in an ice bath and then 5.6 g of sodium acetate was added. The above reaction liquid was added dropwise to a hot (about 75 °C) solution wherein 11 g of potassium ethyl xanthate was dissolved in 51 ml of water, stirred for another 1 hour, cooled to room temperature, and extracted with ethyl acetate. After liquid separation, the organic phase was washed with saturated aqueous solution of sodium chloride and then dried over anhydrous sodium sulfate, filtered, concentrated, and then dissolved in a solution of 1.3 N potassium hydroxide in ethanol, and then added with 3 g of glucose and refluxed for 3 hours. The reaction mixture was concentrated and the pH was adjusted to about 1 with 6 N sulfuric acid in ice bath, and then 5.7 g of zinc powder was added and heated and stirred at 50 °C for 30 minutes. The insoluble was filtered off and the filtrate was extracted with ethyl acetate. After liquid separation, the organic phase was washed with saturated aqueous solution of sodium chloride and dried over anhydrous sodium sulfate, filtered and concentrated, and the residue was separated by column chromatography (100% petroleum ether) to afford 4.15 g of oily matter, yield 71%.
$^1$H NMR (300 MHz, DMSO-$d_6$) δ 7.17 (s, 1H), 6.81 (s, 1H), 6.66 (s, 1H), 4.63 (s, 1H), 3.80 (s, 3H), 2.26 (s, 3H).

Step 4: Preparation of (2,2-diethoxyethyl)(2-methoxy-4-methylphenyl)thioether

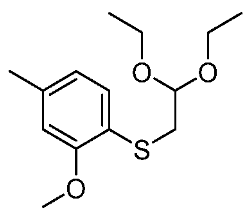

4.15 g of 2-methoxy-4-methylthiophenol and 5.91 g of 2-bromo-1,1-diethoxyethane were dissolved in 40 ml of N,N-dimethylformamide. 15.8 g of cesium carbonate was added, and then the mixture was stirred at room temperature overnight. Water was added, and the mixture was extracted with ethyl acetate. The organic phase was washed with

saturated aqueous solution of sodium chloride and then dried over anhydrous sodium sulfate and filtered and evaporated. The residue was separated by column chromatography (100% petroleum ether) to afford 6.6 g of oily matter, yield 90%.
$^1H$ NMR (300 MHz, DMSO-$d_6$) δ 7.16 (s, 1H), 6.82 (s, 1H), 6.73 (s, 1H), 4.55 (t, *J* = 4.86 Hz, 1H), 3.80 (s, 3H), 3.52-3.64 (m, 2H) 3.39-3.51 (m, 2H) 2.96 (m, 2H), 2.33 (s, 3H), 1.09 (t, *j* = 7.0 Hz, 6H).

Step 5: Preparation of 7-methoxy-5-methylbenzothiophene

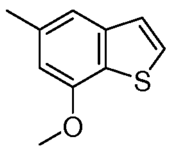

17 g of polyphosphoric acid was dissolved in 150 ml of chlorobenzene and refluxed, and 6.6 g of (2,2-diethoxyethyl)(2-methoxy-4-methylphenyl)thioether was added dropwise into the reaction mixture and refluxed overnight, and the solution was poured out at the next day. The residue was washed with ethyl acetate and then the organic phases were combined, concentrated and separated by column chromatography (100% petroleum ether) to afford 1.85 g of oily matter, yield 42%.
$^1H$ NMR (300 MHz, DMSO-$d_6$) δ 7.68 (s, 1H), 7.34 (s, 1H), 7.28 (s, 1H), 6.78 (s, 1H), 3.93 (s, 3H), 2.43 (s, 3H).

Step 6: Preparation of (7-methoxy-5-methylbenzothiophen-2-yl)boronic acid

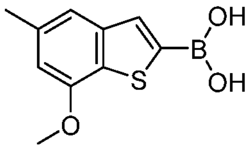

1.5 g of 7-methoxy-5-methylbenzothiophene was dissolved in 20 ml of dry tetrahydrofuran, cooled to -70 °C, and then 1.5 equivalents of n-butyllithium solution was added dropwise, and after stirring at -70 °C for 1 hour, 3 equivalents of triisopropyl borate were added dropwise, and the mixture was stirred at -70 °C for 1 hour, then slowly warmed to room temperature. The reaction was quenched with saturated aqueous solution of ammonium chloride, stirred at room temperature for half an hour and then extracted with ethyl acetate. After washed with saturated aqueous solution of sodium chloride, the organic phase was dried over anhydrous sodium sulfate, filtered and concentrated, and the residue was separated by column chromatography (dichloromethane : methanol = 98:2) to afford 1.7 g of beige solids, yield 94%.

Step 7: Preparation of 4-chloro-5-iodo-7H-rrole [2,3-d]pyrimidine

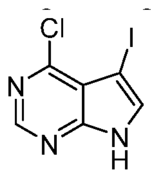

1.53 g of 4-chloro-7H-pyrrole [2,3-d]pyrimidine and 2.7 g of N-iodosuccinimide were dissolved in 35 ml of dichloromethane, stirred at room temperature for 1 hour and then washed with saturated sodium thiosulfate solution and extracted with ethyl acetate. The organic phase was washed with saturated aqueous solution of sodium chloride and then dried over anhydrous sodium sulfate, filtered, concentrated and dried to afford 2.5g of off-white solids which were used directly in the next step.
$^1H$ NMR (300 MHz, DMSO-$d_6$) δ 12.95 (brs, 1 H), 8.60 (s, 1 H), 7.91 (d, *J* = 2.5 Hz, 1 H).

Step 8: Preparation of tert-butyl 3-(4-chloro-5-iodo-7H-pyrrole[2,3-d]pyrimidine-7-yl)pyrrolidine-1-formate

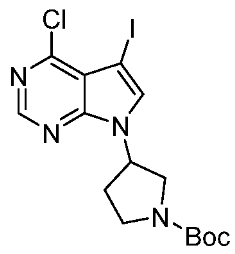

In an argon atmosphere, 500 mg of 4-chloro-5-iodo-7H-pyrrole[2,3-d]pyrimidine, 505 mg of tert-butyl 3-hydroxypyrrolidine-1-formate and 945 mg of triphenylphosphine was dissolved in a dry tetrahydrofuran solution, and 0.7 ml of diisopropyl azodicarboxylate was added dropwise thereto under ice cooling. Upon addition, the mixture was stirred at room temperature for 2 hours, and the reaction mixture was concentrated to dryness, and the residue was isolated by column chromatography (ethyl acetate: petroleum ether = 10: 90) to obtain 1.05 g of pale yellow solids.

Step 9: Preparation of tert-butyl 3-(4-amino-5-iodo-7H-pyrrole[2,3-d]pyrimidine-7-yl)pyrrolidine-1-formate

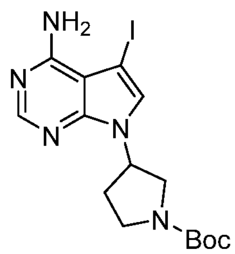

120 mg of tert-butyl 3-(4-chloro-5-iodo-7H-pyrrole[2,3-d]pyrimidine-7-yl)pyrrolidine-1-formate was dissolved in a mixed solution of 2 ml of 1,4 dioxane and 1 ml of aqueous ammonia, the tube was sealed, and the reaction was heated at 100 °C for 16 hours, cooled to room temperature, and then water was added, and the mixture was extracted with ethyl acetate. The organic phase was washed with saturated aqueous sodium chloride and then dried over anhydrous sodium sulfate, filtered and concentrated, and the residue was isolated by column chromatography (dichloromethane: methanol= 98:2) to obtain 113 mg of beige solids, yield 80%.

Step 10: Preparation of tert-butyl 3-(4-amino-5-(7-methoxy-5-methylbenzothiophene-2-yl)-7H-pyrrole[2,3-d]pyrimidine-7-yl) pyrrolidine-1-formate

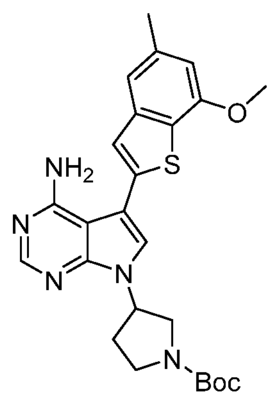

178 mg of tert-butyl 3-(4-amino-5-iodo-7H-pyrrole[2,3-d]pyrimidine-7-yl)pyrrolidine-1-formate, 95 mg of (7-methoxy-5-methylbenzothiophene-2-yl)boronic acid, 90 mg of sodium carbonate and 25 mg of tetratriphenylphosphine palladium were dissolved in a mixed solution of 4 ml of 1,4-dioxane and 1 ml of water, after the replacement of argon gas, the reaction was heated at 80 °C for 5 hours, and the reaction liquid was concentrated to dryness, and the residue was isolated by column chromatography (dichloromethane : methanol = 98:2) to yield 200 mg of pale yellow solids.
$^1$H NMR (400 MHz, $CDCl_3$) δ 8.34 (s, 1H), 7.24 (s, 1H), 7.23 (s, 1H), 7.16 (s, 1H), 6.65 (s, 1H), 5.68 (s, 2H), 5.52 - 5.43 (m, 1H), 4.01 (s, 3H), 3.99 - 3.91 (m, 1H), 3.81 - 3.53 (m, 3H), 2.50 (s, 3H), 2.48 - 2.38 (m, 1H), 2.35 - 2.27 (m, 1H), 1.49 (s, 9H).

Step 11 : Preparation of 5-(7-methoxy-5-methylbenzothiophene-2-yl)-7-(pyrrolidine-3-yl)-7H-pyrrole[2,3-d]pyrimidine-4-amino hydrochloride

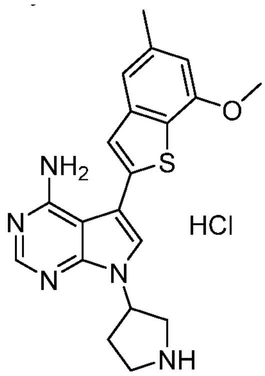

95 mg of tert-butyl3-(4-amino-5-(7-methoxy-5-methylbenzothiophene-2-yl)-7H-pyrrole[2,3-d]pyrimidine-7-yl)pyrrolidine-1-formate was dissolved in a solution of 4 N hydrochloric acid in 1,4-dioxane, stirred at room temperature for 2 hours, and the reaction mixture was concentrated to dryness and directly used in the next step.

Step 12: Preparation of 1-(3-(4-amino-5-(7-methoxy-5-methylbenzothiophene-2-yl)-7H-pyrrole[2,3-d]pyrimidine -7-yl)pyrrolidine-1-yl)prop-2-en-1-one

5-(7-methoxy-5-methylbenzothiophene-2-yl)-7-(pyrrolidine-3-yl)-7H-pyrrole [2,3-d]pyrimidine-4-amino hydrochloride obtained in Step 11 was suspended in 2 ml of dry dichloromethane, 0.15 ml of triethylamine was added, and 17 μl of acryloyl chloride was added dropwise under ice cooling, and upon addition, the mixture was stirred at room temperature overnight. The reaction mixture was concentrated to dryness, and the residue was isolated by column chromatography (dichloromethane: methanol=97: 3) to obtain 59 mg of white solids in 70% yield.
$^1$H NMR (400 MHz, $CDCl_3$) δ 8.33 (s, 1H), 7.23 - 7.19 (m, 2H), 7.11 (d, J = 14.3 Hz, 1H), 6.64 (s, 1H), 6.51 - 6.39 (m, 2H), 5.79 - 5.69 (m, 1H), 5.50 (m, 3H), 4.22 - 4.09 (m, 1H), 4.04 - 3.90 (m, 1H), 3.99 (s, 3H), 3.86 - 3.75 (m, 2H), 2.68 - 2.53 (m, 1H), 2.48 (s, 3H), 2.43 (m, 1H).

**Example 2:**

**1-(3-(4-amino-3-(7-methoxy-5-methylbenzothiophene-2-yl)-1H-pyrazole[3,4-d]pyrimidi ne-1-yl)pyrrolidine-1-yl)prop-2-en-1-one**

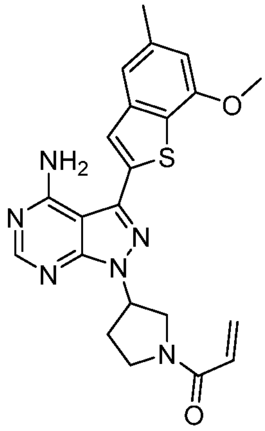

Step 1: Preparation of 1H-pyrazole[3,4-d]pyrimidine-4-amine

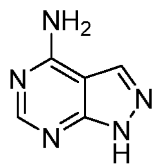

1 g of 3-amino-4-cyano pyrazole was dissolved in formamide, heated to 170 °C under argon for 5 hours, cooled to room temperature, poured into 30 ml of water and beaten. After filtration, the cake was dried to give 1.15 g of brown solids.
$^1$H NMR (300 MHz, DMSO-$d_6$) δ 13.34 (s, 1H), 8.13 (s, 1H), 8.07 (s, 1H), 7.61 (s, 2H).

Step 2: Preparation of 3-iodo-1H-pyrazole [3,4-d]pyrimidine-4-amine

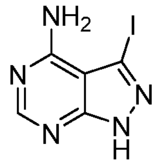

500 mg 1H-pyrazole [3,4-d]pyrimidine-4-amine and 1.25 g of N-iodosuccinimide were dissolved in 5 ml of N,N-dimethylformamide, under argon, the mixture was heated to 80 °C for 18 hours, and after cooled to room temperature, it was poured into 20 ml of saturated aqueous solution of sodium thiosulfate and then beated and filtered. The cake was dried to give 1.2 g of brown solid.
$^1$H NMR (300 MHz, DMSO-$d_6$) δ 13.81 (s, 1H), 8.13 (s, 1H), 8.17 (s, 1H).

Step 3: Preparation of tert-butyl 3-((methylsulfonyl)oxy)pyrrolidine-1-formate

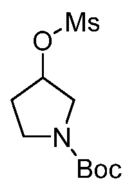

136 mg of tert-butyl 3-hydroxypyrrolidine-1-formate was dissolved in 1 ml of dichloromethane, 0.15 ml of triethylamine was added, and 67 μl of methanesulfonyl chloride was added dropwise under ice cooling. Upon addition, the mixture was stirred at room temperature for 2 hours and then the reaction was quenched. Water was added, and then the mixture was extracted with EtOAc, and the organic phase was washed with a saturated aqueous solution of sodium chloride and dried over anhydrous sodium sulfate. After filtration and concentration, 190 mg of oil matter was obtained.
$^1H$ NMR (300 MHz, $CDCl_3$) δ 5.25-5.28 (m, 1H), 3.49-3.68 (m, 4H), 3.05 (s, 3H), 2.12-2.28 (m, 2H), 1.47 (s, 9H).

Step 4: Preparation of tert-butyl 3-(4-amino-3-iodo-1H-pyrazole [3,4-d]pyrimidine-1-yl)pyrrolidine-1-formate

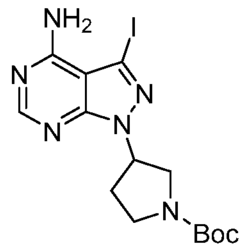

110 mg of 3-iodo-1H-pyrazole [3,4-d]pyrimidine-4-amine and 110 mg of tert-butyl 3-((methylsulfonyl)oxy)pyrrolidine-1-formate were dissolved in 2 ml of N,N-dimethylformamide, then 270 mg of cesium carbonate was added, and heated to 90 °C overnight under argon atmosphere. The next day, water was added and the mixture was extracted with ethyl acetate. The organic phase was washed with saturated aqueous sodium chloride solution and dried over anhydrous sodium sulfate, filtered and evaporated. The residue was isolated by column chromatography (dichloromethane: methanol= 97:3) to obtain 100 mg of yellow solids in a yield of 57%.
$^1H$ NMR (300 MHz, $CDCl_3$) δ 8.32 (s, 1H), 6.05 (s, 2H), 5.40-5.39 (m, 1H), 3.90-3.68 (m, 3H), 3.58-3.47 (m, 1H), 2.58-2.26 (m, 2H), 1.48 (s, 9H).

Step 5: Preparation of tert-butyl 3-(4-amino-3-(7-methoxy-5-methylbenzothiophene-2-yl)-1H-pyrazole [3,4-d]pyrimidine-1 -yl)pyrrolidine-1-formate

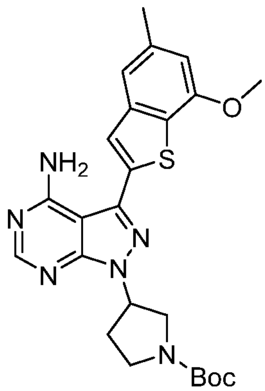

tert-butyl 3-(4-amino-5-iodo-7H-pyrrole[2,3-d]pyrimidine-7-yl)pyrrolidine-1-formate was replaced with tert-butyl 3-(4-amino-3-iodo-1H-pyrazole [3,4-d]pyrimidine-1-yl)pyrrolidine-1-formate, and the remaining required raw materials, reagents and preparation methods were the same as those of Step 10 in Example 1, thereby obtaining 3-(4-amino-3-(7-methoxy-5-methylbenzothiophene-2-yl)-1H-pyrazole[3,4-d]pyrimidine-1-yl)pyrr olidine-1-tert-butyformate.

Step 6: Preparation of 3-(7-methoxy-5-methylbenzothiophene-2-yl)-1-(pyrrolidine-3-yl)-1H-pyrazole[3,4-d]pyrimidine-4 -amine hydrochloride

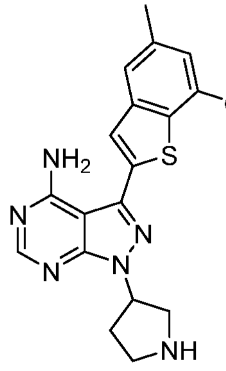

HCl

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-3-(7-methoxy-5-methylbenzothiophene-2-yl)-1H-pyrazole [3,4-d] pyrimidine-1-yl)pyrrolidine-1-formate, and the remaining required materials, reagents and preparation methods were the same as those of Step 11 in Example 1, thereby obtaining 3-(7-methoxy-5-methylbenzothiophene-2-yl)-1-(pyrrolidine-3-yl)-1H-pyrazole[3,4-d]pyrimid ine-4-amine hydrochloride.

Step 7: Preparation of 1-(3-(4-amino-3-(7-methoxy-5-methylbenzothiophene-2-yl)-1H-pyrazole [3,4-d]pyrimidine-1-yl)pyrrolidine-1-yl)propyl-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 3-(7-methoxy-5-methylbenzothiophene-2-yl)-1-(pyrrolidine-3-yl)-1H-pyrazole[3,4-d]pyrimid ine-4-amine hydrochloride, and the remaining required materials, reagents and preparation methods were the same as those of Step 12 in Example 1, thereby obtaining 1-(3-(4-amino-3-(7-methoxy-5-methylbenzothiophene-2-yl)-1H-pyrazole[3,4-d]pyrimidine-1 -yl)pyrrolidine-1-yl)prop-2-en-1-one, yield 53%.
$^1$H NMR (300 MHz, DMSO-$d_6$) $\delta$ 8.29 (s, 1H), 7.67 (s, 1H), 7.34 (s, 1H), 6.85 (s, 1H), 6.74 - 6.51 (m, 1H), 6.17 (d, J = 16.4 Hz, 1H), 5.68 (dd, J = 16.9, 11.0 Hz, 1H), 5.51 (d, J = 21.5 Hz, 1H), 4.18 - 4.05 (m, 1H), 3.95 (s, 3H), 3.84 (m, 3H), 2.44 (s, 3H), 2.39 (m, 2H).

**Example 3:**

**1-(3-(4-amino-5-(7-methoxy-5-methylbenzothiophene-2-yl)-7H-pyrrole[2,3-d]pyrimidine -7-yl)pyrrolidine-1-yl)prop-2-yn-1-one**

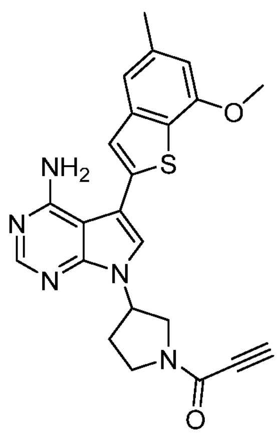

100 mg of 5-(7-methoxy-5-methylbenzothiophene-2-yl)-7-(pyrrolidine-3-yl)-7H-pyrrole[2,3-d]pyrimidi ne -4-amino hydrochloric acid, 20 μl of propiolic acid and 145 mg of 2-(7-azobenzotriazole)-N,N,N',N'-tetramethyluronium hexafluorophosphate were dissolved in 2 ml of N N-dimethylformamide, 0.2 ml of N,N-diisopropylethylamine was added dropwise, and the mixture was stirred at room temperature for 8 hours to quench the reaction. Water was added, and then the mixture was extracted with ethyl acetate, and the organic phase was washed with saturated aqueous sodium

chloride solution and dried over anhydrous sodium sulfate, filtered and concentrated, the residue was isolated by column chromatography (dichloromethane: methanol = 97:3) to 48 mg of yellow solids, yield 44%.
$^1$H NMR (300 MHz, $CDCl_3$) δ 8.33 (s, 1H), 7.22 (s, 2H), 7.11 (d, *J* = 9.3 Hz, 1H), 6.65 (s, 1H), 5.69 (s, 2H), 5.59 - 5.40 (m, 1H), 4.37 - 3.57 (m, 4H), 4.00 (s, 3H), 3.09 (d, *J* = 15.2 Hz, 1H), 2.64 - 2.24 (m, 2H), 2.49 (s, 3H).

**Example** 4**:**

**(E) 1-(3-(4-amino-5-(7-methoxy-5-methylbenzothiophene-2-yl)-7H-pyrrole[2,3-d]pyrimidine-7-yl)pyrrolidine-1-yl)-4-(dimethylamino)but-2-en-1-one**

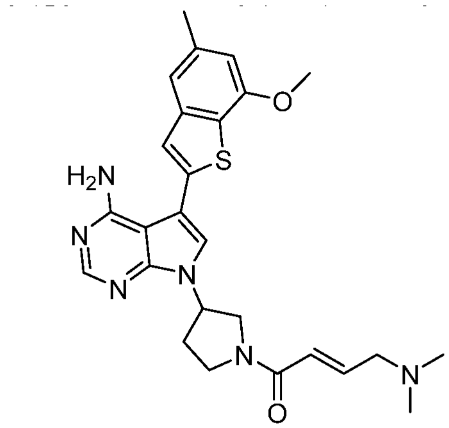

propynoic acid was replaced with trans 4-dimethylamino crotonate hydrochloride, and the remaining required raw materials, reagents and preparation methods were the same as those in Example 3, thereby obtaining (E) 1-(3-(4 -amino-5-(7-methoxy-5-methylbenzothiophene-2-yl)-7H-pyrrole[2,3-d]pyrimidine-7-yl)pyrro lidine-1-yl)-4-(dimethylamino)but-2-en-1-one, yield 43%.
$^1$H NMR (300 MHz, $CDCl_3$) δ 8.32 (s, 1H), 7.22 (s, 2H), 7.11 (d, *J* = 11.4 Hz, 1H), 7.03 - 6.86 (m, 1H), 6.78 (d, *J* = 15.9 Hz, 0.5H), 6.61 (d, *J* = 17.7 Hz, 1.5H), 5.57 (s, 2H), 5.53 - 5.39 (m, 1H), 4.33 - 3.81 (m, 4H), 3.99 (s, 3H), 3.52 (m, 2H), 2.63 (s, 3H), 2.57 (s, 3H), 2.53 - 2.44 (m, 5H).

**Example 5:**

**(E) 1-(3-(4-amino-5-(7-methoxy-5-methylbenzothiophene-2-yl)-7H-pyrrole[2,3-d]pyrimidine-7-yl)pyrrolidine-1-yl)-4-(ethyl(methyl)amino)but-2-en-1-one**

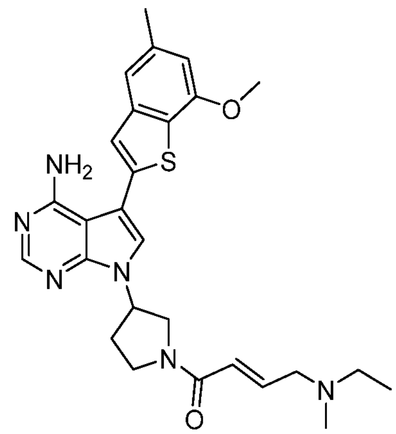

propynoic acid was replaced with trans -4-(ethyl(methyl)amino) crotonate hydrochloride, and the remaining

required raw materials, reagents and preparation methods were the same as those in Example 3, thereby obtaining (E)1-(3-(4-amino-5-(7-methoxy-5-methylbenzothiophene-2-yl)-7H-pyrrole[2,3-d]pyrimidine-7-yl)pyrrolidine-1-yl)-4-(ethyl(methyl)amino)but-2-en-1-one, yield 45%.
$^1$H NMR (300 MHz, $CDCl_3$) δ 8.32 (s, 1H), 7.22 (s, 2H), 7.11 (d, *J* = 12.3 Hz, 1H), 6.99 - 6.71 (m, 2H), 6.64 (s, 1H), 5.61 (s, 2H), 5.56 - 5.38 (m, 1H), 4.40 - 3.88 (m, 4H), 3.99 (s, 3H), 3.81 - 3.62 (m, 2H), 2.75 - 2.59 (m, 3H), 2.48 (s, 3H), 2.62 - 2.34 (m, 2H), 1.52 - 1.33 (m, 3H).

**Example 6:**

**(E)1-(3-(4-amino-5-(7-methoxy-5-methylbenzothiophene-2-yl)-7H-pyrrole[2,3-d]pyrimi dine-7-yl)pyrrolidine-1-yl)-4-(isopropyl(methyl)amino)but-2-en-1-one**

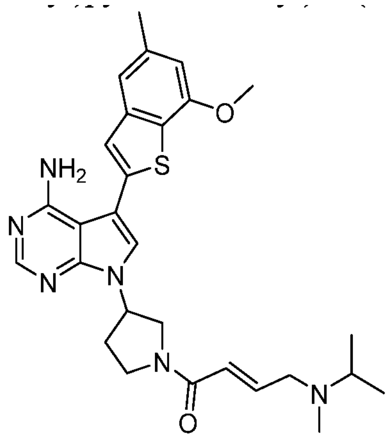

propynoic acid was replaced with trans -4-(isopropyl(methyl)amino) crotonate hydrochloride, and the remaining required raw materials, reagents and preparation methods were the same as those in Example 3, thereby obtaining (E)1-(3-(4-amino-5-(7-methoxy-5-methylbenzothiophene-2-yl)-7H-pyrrole[2,3-d]pyrimidine-7-yl)pyrrolidine-1-yl)-4-(isopropyl(methyl)amino)but-2-en-1-one, yield 42%.
$^1$H NMR (300 MHz, $CDCl_3$) δ 8.32 (s, 1H), 7.21 (s, 2H), 7.11 (d, *J* = 10.8 Hz, 1H), 6.98 - 6.85 (m, 1.5H), 6.78 - 6.67 (m, 0.5H), 6.64 (s, 1H), 5.54 (s, 2H), 5.50 - 5.38 (m, 1H), 4.33 - 3.84 (m, 4H), 3.99 (s, 3H), 3.71 - 3.52 (m, 2H), 3.45 - 3.22 (m, 1H), 2.54 (d, *J* = 16.3 Hz, 3H), 2.48 (s, 3H), 1.36 - 1.21 (m, 6H).

**Example 7:**

**(E)1-(3-(4-amino-5-(7-methoxy-5-methylbenzothiophene-2-yl)-7H-pyrrole[2,3-d]pyrimi dine-7-yl)pyrrolidine-1-yl)-4-(pyrrolidine-1-yl)but-2-en-1-one**

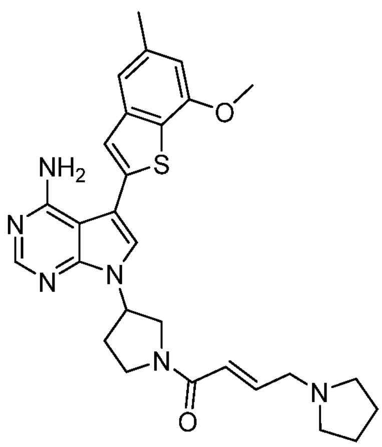

propynoic acid was replaced with trans -4-(pyrrolidine-1-yl)crotonate hydrochloride, and the remaining required raw materials, reagents and preparation methods were the same as those in Example 3, thereby obtaining (E)1-(3 -(4-amino-5-(7-methoxy-5-methylbenzothiophene-2-yl)-7H-pyrrole[2,3-d]pyrimidine-7-yl)py rrolidine-1-yl)-4-(pyrrolidine-1-yl)but-2-en-1-one, yield 43%.
$^1$H NMR (300 MHz, $CDCl_3$) δ 8.32 (s, 1H), 7.22 (s, 2H), 7.11 (d, *J* = 11.1 Hz, 1H), 6.92 (m, 1H), 6.79 (d, *J* = 17.6 Hz, 0.5H), 6.61 (d, *J* = 12.3 Hz, 1.5H), 5.54 - 5.40 (m, 3H), 4.32 - 3.81 (m, 4H), 3.99 (s, 3H), 3.61 (dd, *J* = 19.1, 6.0 Hz, 2H), 3.11 - 2.85 (m, 4H), 2.53 - 2.40 (m, 5H), 2.11 - 1.77 (m, 4H).

**Example 8:**

**(E) 1-(3-(4-amino-5-(7-methoxy-5-methylbenzothiophene-2-yl)-7H-pyrrole[2,3-d]pyrimidine-7-yl)pyrrolidine-1-yl)-4-(piperidine-1-yl)but-2-en-1-one**

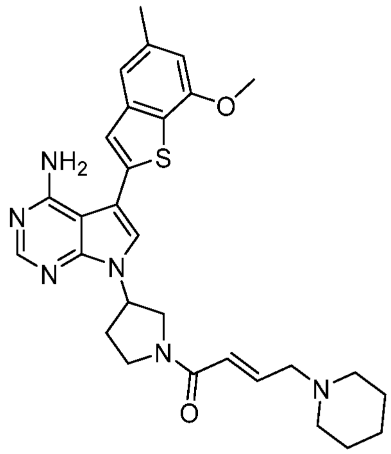

propynoic acid was replaced with trans -4-(piperidine-1-yl)crotonate hydrochloride, and the remaining required raw materials, reagents and preparation methods were the same as those in Example 3, thereby obtaining (E)1-(3 -(4-amino-5-(7-methoxy-5-methylbenzothiophene-2-yl)-7H-pyrrole[2,3-d]pyrimidine-7-yl)py rrolidine-1-yl)-4-(piperidine-1-yl)but-2-en-1-one, yield 44%.
$^1$H NMR (300 MHz, DMSO-d6) δ 8.19 (s, 1H), 7.48 (s, 1H), 7.31 (d, J = 6.6 Hz, 1H), 7.27 (s, 1H), 6.80 (s, 1H), 6.48 (s, 2H), 5.46-5.35 (m, 2H), 5.29-5.26 (m, 1H), 4.05 - 3.62 (m, 4H), 3.94 (s, 3H), 3.40 - 3.20 (m, 6H), 2.43 (s, 3H), 2.42(s, 2H), 1.42 - 1.18(m, 6H).

**Example 9:**

**5-(7-methoxy-5-methylbenzothiophene-2-yl)-7-(pyrrolidine-3-yl)-7H-pyrrole[2,3-d]pyri midine-4-amine**

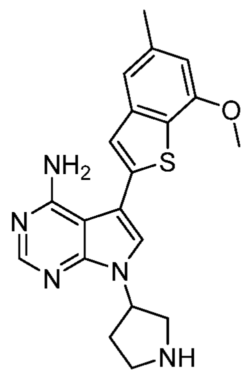

95 mg of tert-butyl 3-(4-amino-5-(7-methoxy-5-methylbenzothiophene-2-yl)-7H-pyrrole[2,3-d]pyrimidine-7-yl)pyrrolidine-1-formate was dissolved in a solution of 4 N hydrochloric acid in 1,4-dioxane, stirred at room temperature for 2 hours. The reaction mixture was concentrated to dryness and washed with a saturated aqueous solution of sodium bicarbonate and extracted by adding ethyl acetate. The organic phase was washed with saturated aqueous sodium chloride solution and then dried over anhydrous sodium sulfate, filtered and concentrated, and the residue was isolated by column chromatography (dichloromethane:methanol (containing 1% ammonia) = 95:5) to obtain 48 mg of yellow solid, yield 44%.

$^1$H NMR (300 MHz, DMSO-d6) δ 8.20 (s, 1H), 7.59 (d, J = 17.3 Hz, 1H), 7.32 (s, 1H), 7.27 (s, 1H), 6.79 (s, 1H), 6.49 (s, 2H), 5.46 - 5.26 (m, 1H), 4.19 - 4.06 (m, 0.5H), 3.94 (s, 3H), 3.85-4.0(m, 1H), 3.80 - 3.68 (m, 1.5H), 3.58-3.45 (m, 1H), 2.43 (s, 3H), 2.43 - 2.34 (m, 2H).

**Example 10:**

**tert-butyl 3-(4-amino-5-(7-methoxy-5-methylbenzothiophene-2-yl)-7H-pyrrole[2,3-d]pyrimidine-7-yl) pyrrolidine-1-formate**

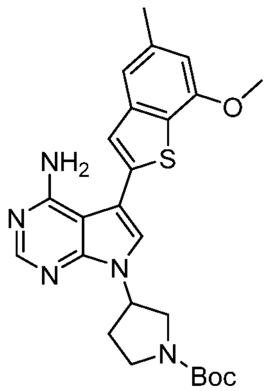

Preparation was the same as step 10 in Example 1.

$^1$H NMR (300 MHz, DMSO-d6) δ 8.20 (s, 1H), 7.59 (d, J = 17.3 Hz, 1H), 7.32 (s, 1H), 7.27 (s, 1H), 6.79 (s, 1H), 6.49 (s, 2H), 5.46 - 5.26 (m, 1H), 4.19 - 4.06 (m, 0.5H), 3.94 (s, 3H), 3.85-4.0(m, 1H), 3.80 - 3.68 (m, 1.5H), 3.58-3.45 (m, 1H), 2.43 (s, 3H), 2.43 - 2.34 (m, 2H),1.48(s, 9H).

**Example 11:**

**(R)-1-(3-(4-amino-5-(7-methoxy-5-methylbenzothiophene-2-yl)-7H-pyrrole[2,3-d]pyrimi dine-7-yl)pyrrolidine-1-yl)prop-2-en-1-one**

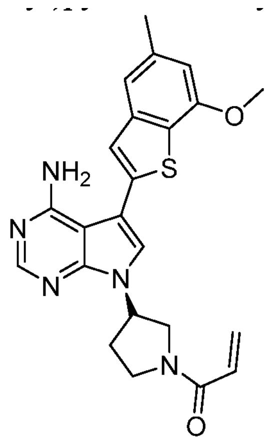

Step 1: Preparation of tert-butyl (R)-3-(4-chloro-5-iodo-7H-pyrrole[2,3-d]pyrimidine-7-yl)pyrrolidine-1-formate

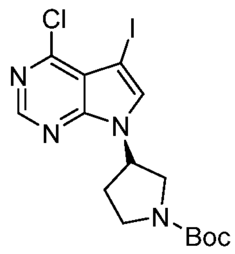

tert-butyl 3-hydroxypyrrolidine-1-formate was replaced with tert-butyl (S)-3-hydroxypyrrolidine-1-formate, and the remaining required raw materials, reagents and preparation methods were the same as those of Step 8 in Example 1, thereby obtaining (R)-3-(4-chloro-5-iodo-7H-pyrrole[2,3-d]pyrimidine-7-yl)pyrrolidine-1-tert-butyl formate.

Step 2: Preparation of tert-butyl (R)-3-(4-amino-5-iodo-7H-pyrrole[2,3-d]pyrimidine-7-yl)pyrrolidine-1-formate

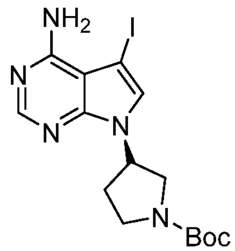

tert-butyl 3-(4-chloro-5-iodo-7H-pyrrole[2,3-d]pyrimidine-7-yl)pyrrolidine-1-formate was replaced with tert-butyl (R)-3-(4 -chloro-5-iodo-7H-pyrrole[2,3-d]pyrimidine-7-yl)pyrrolidine-1-formate, and the remaining required raw materials, reagents and preparation methods were the same as those of Step 9 in Example 1, thereby obtaining (R)-3-(4-amino-5-iodo-7H-pyrrole[2,3-d]pyrimidine-7-yl)pyrrolidine-1-tert-butyl formate.

Step 3: Preparation of tert-butyl (R) 3-(4-amino-5-(7-methoxy-5-methylbenzothiophene-2-yl)-7H-pyrrole[2,3-d]pyrimidine-7-yl) pyrrolidine-1-formate

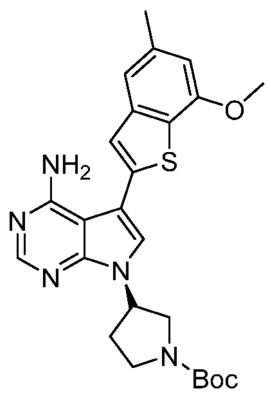

tert-butyl 3-(4-amino-5-iodo-7H-pyrrole[2,3-d]pyrimidine-7-yl)pyrrolidine-1-formate was replaced with tert-butyl (R)-3-(4-amino-5-iodo-7H-pyrrole[2,3-d]pyrimidine-7-yl)pyrrolidine-1-formate, and the remaining required raw materials, reagents and preparation methods were the same as those of Step 10 in Example 1, thereby obtaining tert-butyl (R) 3-(4-amino-5-(7-methoxy-5-methylbenzothiophene-2-yl)-7H-pyrrole[2,3-d]pyrimidine-7-yl)pyrrol idine-1-formate.

Step 4: Preparation of (R)-5-(7-methoxy-5-methylbenzothiophene-2-yl)-7-(pyrrolidine-3-yl)-7H-pyrrole[2,3-d]pyrimidin e-4-amino hydrochloride

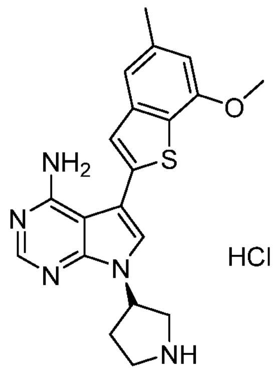

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl (S)-3-(4-amino-5-(7-methoxy-5-methylbenzothiophene-2-yl)-7H-pyrrole[2,3 -d]pyrimidine-7-yl)pyrrolidine-1-formate, and the remaining required raw materials, reagents and preparation methods were the same as those of Step 11 in Example 1, thereby obtaining (R)-5-(7-methoxy -5-methylbenzothiophene-2-yl)-7-(pyrrolidine-3-yl)-7H-pyrrole[2,3-d]pyrimidine-4-amino hydrochloride.

Step 5: Preparation of (R)-1-(3-(4-amino-5-(7-methoxy-5-methylbenzothiophene-2-yl)-7H-pyrrole[2,3-d]pyrimidin e-7-yl)pyrrolidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with (R)-5-(7-methoxy-5-methylbenzothiophene-2-yl)-7-(pyrrolidine-3-yl)-7H-pyrrole[2,3-d]pyri midine-4-amino hydrochloride, and the remaining required raw materials, reagents and preparation methods were the same as those of Step 12 in Example 1, thereby obtaining (R)-1-(3-(4-amino-5-(7-methoxy-5-methylbenzothiophene-2-yl)-7H-pyrrole[2,3-d]pyrimidin e-7-yl)pyrrolidine-1-yl)prop-2-en-1-one, yield 51%.
$^1$H NMR (400 MHz, $CDCl_3$) δ 8.33 (s, 1H), 7.23 - 7.19 (m, 2H), 7.11 (d, J = 14.3 Hz, 1H), 6.64 (s, 1H), 6.51 - 6.39 (m, 2H), 5.79 - 5.69 (m, 1H), 5.50 (m, 3H), 4.22 - 4.09 (m, 1H), 4.04 - 3.90 (m, 1H), 3.99 (s, 3H), 3.86 - 3.75 (m, 2H), 2.68 - 2.53 (m, 1H), 2.48 (s, 3H), 2.43 (m, 1H).

**Example 12:**

**(S)-1-(3-(4-amino-5-(7-methoxy-5-methylbenzothiophene-2-yl)-7H-pyrrole[2,3-d]pyrimi dine-7-yl)pyrrolidine-1-yl)prop-2-en-1-one**

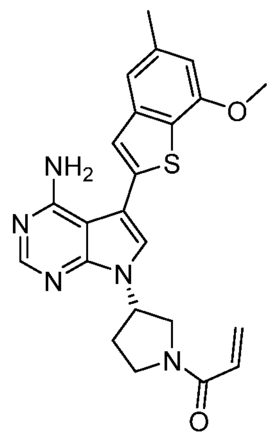

Step 1: Preparation of tert-butyl (S)-3-(4-chloro-5-iodo-7H-pyrrole[2,3-d]pyrimidine-7-yl)pyrrolidine-1-formate

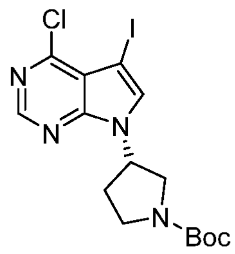

tert-butyl 3-hydroxypyrrolidine-1-formate was replaced with tert-butyl (R)-3-hydroxypyrrolidine-1- formate, and the remaining required raw materials, reagents and preparation methods were the same as those of Step 8 in Example 1, thereby obtaining tert-butyl (S)-3-(4-chloro-5-iodo-7H-pyrrole[2,3-d]pyrimidine-7-yl)pyrrolidine-1-formate.

Step 2: Preparation of tert-butyl (S)-3-(4-amino-5-iodo-7H-pyrrole[2,3-d]pyrimidine-7-yl)pyrrolidine-1-formate

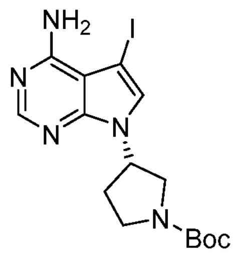

tert-butyl 3-(4-chloro-5-iodo-7H-pyrrole[2,3-d]pyrimidine-7-yl)pyrrolidine-1-formate was replaced with tert-butyl (S)-3-(4 -chloro-5-iodo-7H-pyrrole[2,3-d]pyrimidine-7-yl)pyrrolidine-1-formate, and the remaining required raw materials, reagents and preparation methods were the same as those of Step 9 in Example 1, thereby obtaining tert-butyl (S)-3-(4-amino-5-iodo-7H-pyrrole[2,3-d]pyrimidine-7-yl)pyrrolidine-1-formate.

Step 3: Preparation of tert-butyl (S)3-(4-amino-5-(7-methoxy-5-methylbenzothiophene-2-yl)-7H-pyrrole[2,3-d]pyrimidine -7-yl)pyrrolidine-1-formate

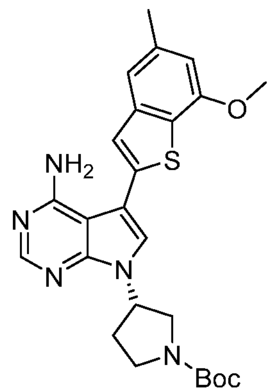

tert-butyl 3-(4-amino-5-iodo-7H-pyrrole[2,3-d]pyrimidine-7-yl)pyrrolidine-1-formate was replaced with tert-butyl (S)-3-(4-amino-5-iodo-7H-pyrrole[2,3-d]pyrimidine-7-yl)pyrrolidine-1-formate, and the remaining required raw materials, reagents and preparation methods were the same as those of Step 10 in Example 1, thereby obtaining tert-butyl (S)3-(4-amino-5-(7-methoxy-5-methylbenzothiophene-2-yl)-7H-pyrrole[2,3-d]pyrimidine-7-yl)pyr rolidine-1-formate.

Step 4: Preparation of (S)-5-(7-methoxy-5-methylbenzothiophene-2-yl)-7-(pyrrolidine-3-yl)-7H-pyrrole[2,3-d]pyrimidine -4-amino hydrochloride

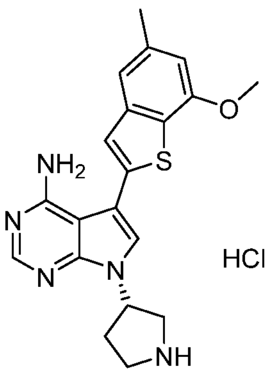

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl (S)-3-(4-amino-5-(7-methoxy-5-methylbenzothiophene-2-yl)-7H-pyrrole[2,3 -d]pyrimidine-7-yl)pyrrolidine-1-formate, and the remaining required raw materials, reagents and preparation methods were the same as those of Step 11 in Example 1, thereby obtaining (S)-5-(7-methoxy-5-methylbenzothiophene-2-yl)-7-(pyrrolidine-3-yl)-7H-pyrrole[2,3-d]pyri midine-4-amino hydrochloride.

Step 5: Preparation of (S)-1-(3-(4-amino-5-(7-methoxy-5-methylbenzothiophene-2-yl)-7H-pyrrole[2,3-d]pyrimidine -7-yl)pyrrolidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with (S)-5-(7-methoxy-5-methylbenzothiophene-2-yl)-7-(pyrrolidine-3-yl)-7H-pyrrole[2,3-d]pyri midine-4-amino hydrochloride, and the remaining required raw materials, reagents and preparation methods were the same as those of Step 12 in Example 1, thereby obtaining (S)-1-(3-(4-amino-5-(7-methoxy-5-methylbenzothiophene-2-yl)-7H-pyrrole[2,3-d]pyrimidine -7-yl)pyrrolidine-1-yl)prop-2-en-1-one, yield 53%.
$^1$H NMR (400 MHz, $CDCl_3$) δ 8.33 (s, 1H), 7.23 - 7.19 (m, 2H), 7.11 (d, J = 14.3 Hz, 1H), 6.64 (s, 1H), 6.51 - 6.39 (m, 2H), 5.79 - 5.69 (m, 1H), 5.50 (m, 3H), 4.22 - 4.09 (m, 1H), 4.04 - 3.90 (m, 1H), 3.99 (s, 3H), 3.86 - 3.75 (m, 2H), 2.68 - 2.53 (m, 1H), 2.48 (s, 3H), 2.43 (m, 1H).

**Example 13:**

**1-(3-(4-amino-5-(7-methoxy-5-methylbenzothiophene-2-yl)-7H-pyrrole[2,3-d]pyrimidine -7-yl)azetidin-1-yl)prop-2-en-1-one**

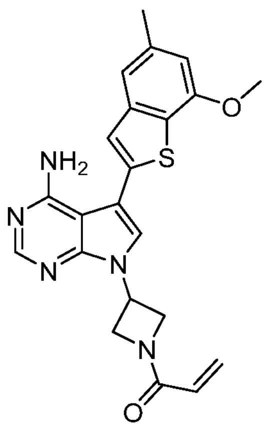

Step 1: Preparation of tert-butyl 3-(4-chloro-5-iodo-7H-pyrrole[2,3-d]pyrimidine-7-yl)azetidin-1-formate

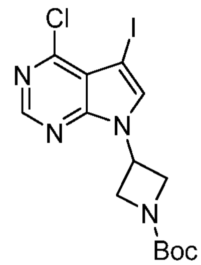

tert-butyl 3-hydroxypyrrolidine-1-formate was replaced with tert-butyl 3-hydroxyazetidin-1-formate, and the remaining required raw materials, reagents and preparation methods were the same as those of Step 8 in Example 1, thereby obtaining tert-butyl 3-(4-chloro-5-iodo-7H-pyrrole[2,3-d]pyrimidine-7-yl)azetidin-1-formate.

Step 2: Preparation of tert-butyl 3-(4-amino-5-iodo-7H-pyrrole[2,3-d]pyrimidine-7-yl)azetidine-1-formate

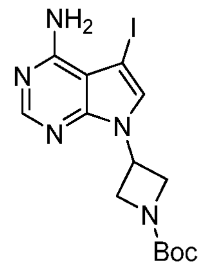

tert-butyl 3-(4-chloro-5-iodo-7H-pyrrole[2,3-d]pyrimidine-7-yl)pyrrolidine-1-formate was replacing with tert-butyl 3-(4-chloro-5-iodo-7H-pyrrole [2,3-d]pyrimidine-7-yl)azetidin-1-formate, and the remaining required raw materials, reagents and preparation methods were the same as those of Step 9 in Example 1, thereby obtaining tert-butyl 3-(4-amino-5-iodo-7H-pyrrole[2,3-d]pyrimidine-7-yl)azetidin-1-formate.
$^1$H NMR (300 MHz, $CDCl_3$) δ 8.22 (d, J = 1.7 Hz, 1H), 7.35 (d, J = 1.3 Hz, 1H), 6.02 - 5.86 (m, 2H), 5.50 (d, J = 4.0 Hz, 1H), 4.45 (m, 2H), 4.15 (m, 2H), 1.47 (s, 9H).

Step 3: Preparation of tert-butyl 3-(4-amino-5-(7-methoxy-5-methylbenzothiophene-2-yl)-7H-pyrrole[2,3-d]pyrimidine-7-yl)azetidi n-1-formate

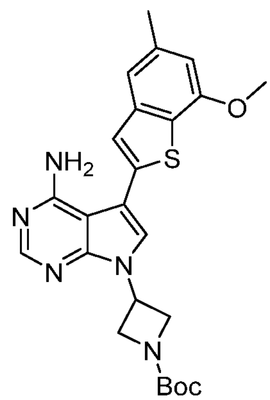

tert-butyl 3-(4-amino-5-iodo-7H-pyrrole[2,3-d]pyrimidine-7-yl)pyrrolidine-1-formate was replaced with tert-butyl 3-(4-amino-5-iodo-7H-pyrrole[2,3-d]pyrimidine-7-yl)azetidin-1-formate, and the remaining required raw materials, reagents and preparation methods were the same as those of Step 10 in Example 1, thereby obtaining tert-butyl 3-(4-amino-5-(7-methoxy-5-methylbenzothiophene-2-yl)-7H-pyrrole[2,3-d]pyrimidine-7-yl)azetidi n-1-formate.

Step 4: Preparation of 5-(7-methoxy-5-methylbenzothiophene-2-yl)-7-(azetidin-3-yl)-7H-pyrrole[2,3-d]pyrimidine-4-ami ne hydrochloride

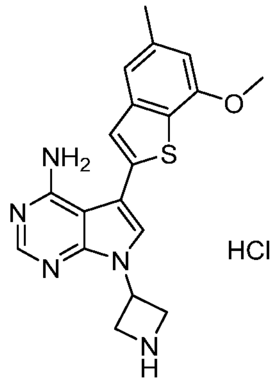

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-5-(7-methoxy-5-methylbenzothiophene-2-yl)-7H-pyrrole[2,3-d]pyrimidine -7-yl)azetidin-1-formate, and the remaining required raw materials, reagents and preparation methods were the same as those of Step 11 in Example 1, thereby obtaining 5-(7-methoxy-5-methylbenzothiophene-2-yl)-7-(azetidin-3-yl)-7H-pyrrole[2,3-d]pyrimidine-4-amine hydrochloride.

Step 5: Preparation of 1-(3-(4-amino-5-(7-methoxy-5-methylbenzothiophene-2-yl)-7H-pyrrole[2,3-d]pyrimidine -7-yl)azetidin-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxy-5-methylbenzothiophene-2-yl)-7-(azetidin-3-yl)-7H-pyrrole[2,3-d]pyrimidine-4-amino hydrochloride, and the remaining required raw materials, reagents and preparation methods were the same as those of Step 12 in Example 1, thereby obtaining 1-(3-(4-amino-5-(7-methoxy-5-methylbenzothiophene-2-yl)-7H-pyrrole[2,3-d]pyrimidine-7-yl)azetidin-1-yl)prop-2-en-1-one, yield 61%.
$^1$H NMR (300 MHz, $CDCl_3$) δ8.30 (s, 1H), 7.35 (s, 1H), 7.23 (d, J = 2.8 Hz, 2H), 6.64 (s, 1H), 6.47 - 6.37 (m, 1H), 6.24 (dd, J = 17.0, 10.1 Hz, 1H), 5.71 (dd, J = 25.2, 12.1 Hz, 4H), 4.78 (s, 1H), 4.66 (s, 1H), 4.54 (d, J = 14.2 Hz, 2H), 3.99 (s, 3H), 2.48 (s, 3H)..

**Example 14:**

**1-(4-(4-amino-5-(7-methoxy-5-methylbenzothiophene-2-yl)-7H-pyrrole[2,3-d]pyrimidine -7-yl)piperidine-1-yl)prop-2-en-1-one**

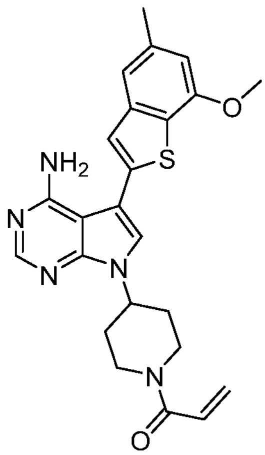

Step 1: Preparation of tert-butyl 4-(4-chloro-5-iodo-7H-pyrrole[2,3-d]pyrimidine-7-yl)piperidine-1-formate

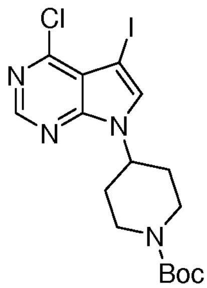

tert-butyl 3-hydroxypyrrolidine-1- formate was replaced with tert-butyl 4-hydroxypiperidine-1-formate, and the remaining required raw materials, reagents and preparation methods were the same as those of Step 8 in Example 1, thereby obtaining tert-butyl 4-(4-chloro-5-iodo-7H-pyrrole[2,3-d]pyrimidine-7-yl)piperidine-1-formate.

Step 2: Preparation of tert-butyl 4-(4-amino-5-iodo-7H-pyrrole[2,3-d]pyrimidine-7-yl)piperidine-1-formate

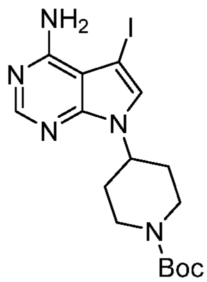

tert-butyl 3-(4-chloro-5-iodo-7H-pyrrole[2,3-d]pyrimidine-7-yl)pyrrolidine-1-formate was replaced with tert-butyl 4-(4-chloro-5-iodo-7H-pyrrole [2,3-d]pyrimidine-7-yl)piperidine-1-formate, and the remaining required raw materials, reagents and preparation methods were the same as those of Step 9 in Example 1, thereby obtaining tert-butyl 4-(4-amino-5-iodo-7H-pyrrole[2,3-d]pyrimidin-7-yl)piperidine-1-formate.
$^1$H NMR (300 MHz, $CDCl_3$) δ 8.26 (s, 1H), 7.09 (s, 1H), 5.64 (s, 2H), 4.75-4.85 (m, 1H), 4.25-4.35 (m, 2H), 2.85-2.95 (m, 2H), 1.98-2.04 (m, 2H), 1.78-1.90 (m, 2H), 1.48 (s, 9H).

Step 3: Preparation of tert-butyl 4-(4-amino-5-(7-methoxy-5-methylbenzothiophene-2-yl)-7H-pyrrole[2,3-d]pyrimidine-7-yl)piperi dine-1-formate

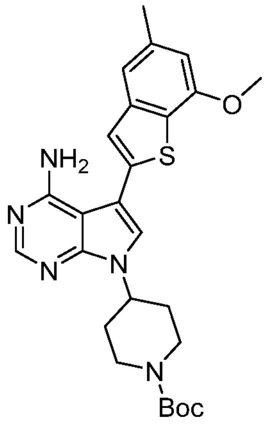

tert-butyl 3-(4-amino-5-iodo-7H-pyrrole[2,3-d]pyrimidine-7-yl)pyrrolidine-1-formate was replaced with tert-butyl 4-(4-amino-5-iodo-7H-pyrrole[2,3-d]pyrimidine-7-yl)piperidine-1-formate, and the remaining required raw materials, reagents and preparation methods were the same as those of Step 10 in Example 1, thereby obtaining tert-butyl 4-(4-amino-5-(7-methoxy-5-methylbenzothiophene-2-yl)-7H-pyrrole[2,3-d]pyrimidine-7-yl)piperi dine-1-formate.
$^1$H NMR (300 MHz, $CDCl_3$) δ 8.32 (s, 1H), 7.20 (s, 2H), 7.16 (s, 1H), 6.63 (s, 1H), 5.63 (s, 2H), 4.82-4.92 (m, 1H), 4.25-4.40 (m, 2H), 3.99 (s, 3H), 2.88-3.02 (m, 2H), 2.48 (s, 3H), 2.15-1.80 (m, 4H), 1.48 (s, 9H).

Step 4: Preparation of 5-(7-methoxy-5-methylbenzothiophene-2-yl)-7-(piperidine-4-yl)-7H-pyrrole [2,3-d]pyrimidine-4-amino hydrochloride

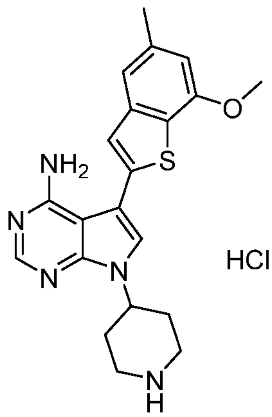

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 4-(4-amino-5-(7-methoxy-5-methylbenzothiophene-2-yl)-7H-pyrrole[2,3-d]pyrimidine -7-yl)piperidine-1-formate, and the remaining required raw materials, reagents and preparation methods were the same as those of Step 11 in Example 1, thereby obtaining 5-(7-methoxy-5-methylbenzothiophene-2-yl)-7-(piperidine-4-yl)-7H-pyrrole[2,3-d]pyrimidin e-4-amino hydrochloride.

Step 5: Preparation of 1-(4-(4-amino-5-(7-methoxy-5-methylbenzothiophene-2-yl)-7H-pyrrole[2,3-d]pyrimidine -7-yl)piperidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxy-5-methylbenzothiophene-2-yl)-7-(piperidine-4-yl)-7H-pyrrole [2,3-d]pyrimidine-4-amino hydrochloride, and the remaining required raw materials, reagents and preparation methods were the same as those of Step 12 in Example 1, thereby obtaining 1-(4-(4-amino-5-(7-methoxy-5-methylbenzothiophene-2-yl)-7H-pyrrole[2,3-d]pyrimidine-7-yl)piperidine-1-yl)prop-2-en-1-one, yield 56%.
$^1$H NMR (300 MHz, $CDCl_3$) δ 8.32 (s, 1H), 7.20 (d, J = 4.1 Hz, 2H), 7.14 (s, 1H), 6.62 (m, 2H), 6.32 (m, 1H), 5.73 (m, 1H), 5.62 (s, 2H), 4.97 (m, 2H), 4.18 (d, J = 11.4 Hz, 1H), 3.99 (s, 3H), 3.32 (s, 1H), 2.85 (s, 1H), 2.48 (s, 3H), 1.99 (m, 4H).

**Example 15:**

**1-(3-(4-amino-5-(7-methoxy-5-methylbenzothiophene-2-yl)-7H-pyrrole[2,3-d]pyrimidine -7-yl)piperidine-1-yl)prop-2-en-1-one**

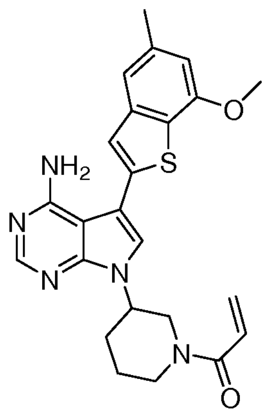

Step 1: Preparation of tert-butyl 3-(4-chloro-5-iodo-7H-pyrrole[2,3-d]pyrimidine-7-yl)piperidine-1-formate

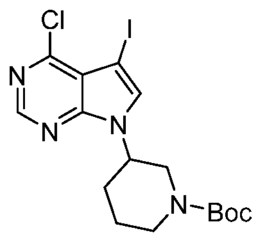

tert-butyl 3-hydroxypyrrolidine-1-formate was replaced with tert-butyl 3-hydroxypiperidine-1-formate, and the remaining required raw materials, reagents and preparation methods were the same as those of Step 8 in Example 1, thereby obtaining tert-butyl 3-(4-chloro-5-iodo-7H-pyrrole[2,3-d]pyrimidine-7-yl)piperidine-1-formate.

Step 2: Preparation of tert-butyl 3-(4-amino-5-iodo-7H-pyrrole[2,3-d]pyrimidine-7-yl)piperidine-1-formate

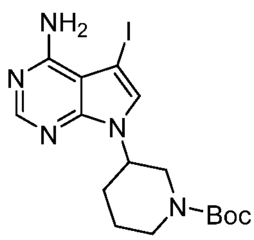

tert-butyl 3-(4-chloro-5-iodo-7H-pyrrole[2,3-d]pyrimidine-7-yl)pyrrolidine-1-formate was replaced with tert-butyl 3-(4-chloro-5-iodo-7H-pyrrole [2,3-d]pyrimidine-7-yl)piperidine-1-formate, and the remaining required raw materials, reagents and preparation methods were the same as those of Step 9 in Example 1, thereby obtaining 3-(4-amino-5-iodo-7H-pyrrole[2,3-d]pyrimidin-7-yl)piperidine-1-tert-butyl formate.

Step 3: Preparation of tert-butyl 3-(4-amino-5-(7-methoxy-5-methylbenzothiophene-2-yl)-7H-pyrrole[2,3-d]pyrimidine-7-yl)piperi dine-1-formate

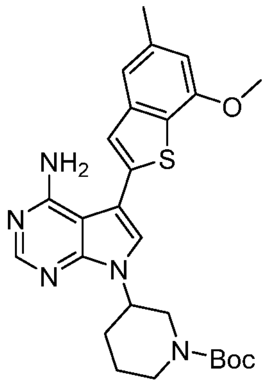

tert-butyl 3-(4-amino-5-iodo-7H-pyrrole[2,3-d]pyrimidine-7-yl)pyrrolidine-1-formate was replaced with tert-butyl 3-(4-amino-5-iodo-7H-pyrrole[2,3-d]pyrimidine-7-yl)piperidine-1-formate, and the remaining required raw materials, reagents and preparation methods were the same as those of Step 10 in Example 1, thereby obtaining tert-butyl 3-(4-amino-5-(7-methoxy-5-methylbenzothiophene-2-yl)-7H-pyrrole[2,3-d]pyrimidine-7-yl)piperi dine-1-formate.

Step 4: Preparation of 5-(7-methoxy-5-methylbenzothiophene-2-yl)-7-(piperidine-3-yl)-7H-pyrrole [2,3-d]pyrimidine-4-amino hydrochloride

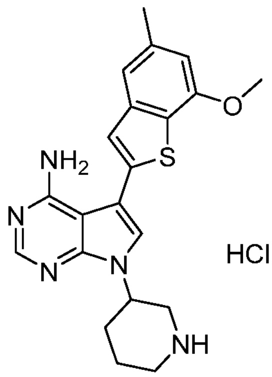

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-5-(7-methoxy-5-methylbenzothiophene-2-yl)-7H-pyrrole[2,3-d]pyrimidine -7-yl)piperidine-1-formate, and the remaining required raw materials, reagents and preparation methods were the same as those of Step 11 in Example 1, thereby obtaining 5-(7-methoxy-5-methylbenzothiophene-2-yl)-7-(piperidine-3-yl)-7H-pyrrole[2,3-d]pyrimidin e-4-amino hydrochloride.

Step 5: Preparation of 1-(3-(4-amino-5-(7-methoxy-5-methylbenzothiophene-2-yl)-7H-pyrrole[2,3-d]pyrimidine -7-yl)piperidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxy-5-methylbenzothiophene-2-yl)-7-(piperidine-3-yl)-7H-pyrrole [2,3-d]pyrimidine-4-amino hydrochloride, and the remaining required raw materials, reagents and preparation methods were the same as those of Step 12 in Example 1, thereby obtaining 1-(3-(4-amino-5-(7-methoxy-5-methylbenzothiophene-2-yl)-7H-pyrrole[2,3-d]pyrimidine-7-yl)piperidine-1-yl)prop-2-en-1-one, yield 50%.
$^1$H NMR (300 MHz, $CDCl_3$) δ 8.33 (s, 1H), 7.22 (d, J = 3.2 Hz, 2H), 7.17 (s, 1H), 6.63 (q, J = 10.3 Hz, 2H), 6.32 (d, J = 16.9 Hz, 1H), 5.72 (d, J = 10.1 Hz, 1H), 5.53 (s, 2H), 4.74 (s, 2H), 4.32 (s, 1H), 3.25 (s, 1H), 2.84 (s, 1H), 2.28 (s, 1H), 2.17 (s, 1H), 1.95 (s, 1H), 1.77 (s, 4H).

Example 16:

1-(3-(4-amino-5-(7-methoxy-5-methylbenzothiophene-2-yl)-7H-pyrrole[2,3-d]pyrimidine -7-yl)pyrrolidine-1-yl)propan-1-one

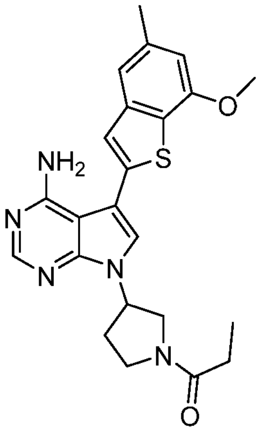

acryloyl chloride was replaced with propionyl chloride, and the remaining required raw materials, reagents and preparation methods were the same as those of Step 12 in Example 1, thereby obtaining 1-(3-(4-amino-5-(7-methoxy-5-methylbenzothiophene-2-yl)-7H-pyrrole[2,3-d]pyrimidine-7-yl)pyrrolidine-1-yl)propan-1-one, yield 46%.
$^1$H NMR (300 MHz, $CDCl_3$) δ 8.33 (s, 1H), 7.22 (s, 2H), 7.10 (d, J = 14.8 Hz, 1H), 6.64 (s, 1H), 5.55 (s, 2H), 5.52 - 5.39 (m, 1H), 4.10 - 4.02 (m, 1H), 4.00 (s, 3H), 3.86 (dd, J = 16.1, 7.0 Hz, 1H), 3.74 - 3.62 (m, 2H), 2.49 (s, 3H), 2.44 - 2.22 (m, 4H), 1.18 (m, 3H).

Example 17:

(E)-1-(3-(4-amino-5-(7-methoxy-5-methylbenzothiophene-2-yl)-7H-pyrrole[2,3-d]pyrimidin e-7-yl)azetidin-1-yl)-4-(pyrrolidine-1-yl)but-2-en-1-one

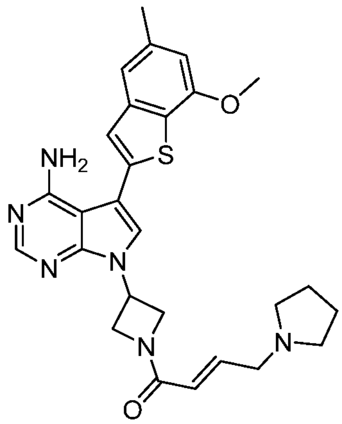

80 mg of 5-(7-methoxy-5-methylbenzothiophene-2-yl)-7-(azetidin-3-yl)-7H-pyrrole[2,3-d]pyrimidine-4-amino hydrochloride, 116 mg of trans-4-(pyrrolidine-1-yl) crotonate hydrochloride and 228 mg of 2-(7-azobenzotriazole)-N,N,N',N'-tetramethyluronium hexafluorophosphate were dissolved in 2 ml of N,N-dimethylformamide, 0.26 ml of N,N-diisopropylethylamine was added dropwise, and stirred at room temperature overnight. Water was added, and then the mixture was extracted with EtOAc. The organic phase was washed with a saturated aqueous solution of sodium chloride and dried over anhydrous sodium sulfate, filtered and concentrated, and the residue was isolated by column chromatography (dichloromethane: methanol (containing 1% ammonia) = 95:5) to obtain 30 mg of yellow solids, yield 23%.
$^1$H NMR (300 MHz, $CD_3OD$) $\delta$ 8.13 (s, 1H), 7.56 (s, 1H), 7.22 (s, 1H), 7.16 (s, 1H), 6.76 (dd, J = 14.8, 7.5 Hz, 1H), 6.69 (s, 1H), 6.46 (d, J = 15.1 Hz, 1H), 5.60 - 5.50 (m, 1H), 4.80 (d, J = 8.2 Hz, 2H), 4.55 (dd, J = 17.1, 8.5 Hz, 2H), 3.94 (s, 3H), 3.83 (d, J = 6.8 Hz, 2H), 3.18 (s, 4H), 2.42 (s, 3H), 2.01 (d, J = 3.7 Hz, 4H).

Example 18:

(E)-1-(3-(4-amino-5-(7-methoxy-5-methylbenzothiophene-2-yl)-7H-pyrrole[2,3-d]pyrimi dine-7-yl)pyrrolidine-1-yl)but-2-en-1-one

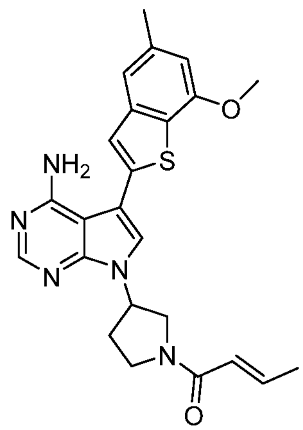

acryloyl chloride was replaced with crotonyl chloride, and the remaining required raw materials, reagents and preparation methods were the same as those of Step 12 in Example 1, thereby obtaining (E)-1-(3-(4-amino-5-(7-methoxy-5-methylbenzothiophene-2-yl)-7H-pyrrole[2,3-d]pyrimidine -7-yl)pyrrolidine-1-yl)but-2-en-1-one, yield 36%.
$^1$H NMR (300 MHz, $CDCl_3$) $\delta$ 8.31 (s, 1H), 7.20 (s, 2H), 7.09 (dd, J = 13.6, 4.4 Hz, 1H), 7.04 - 6.88 (m, 1H), 6.62 (s, 1H), 6.12 (dd, J = 22.6, 15.1 Hz, 1H), 5.73 (s, 2H), 5.55 - 5.38 (m, 1H), 4.20 - 4.04 (m, 1H), 3.98 (s, 4H), 3.81 (dd, J = 13.8, 7.8 Hz, 1H), 3.74 (dd, J = 13.8, 6.7 Hz, 2H), 2.49 (d, J = 10.5 Hz, 4H), 2.45 - 2.33 (m, 2H), 1.88 (dd, J = 10.8, 7.5 Hz, 3H).

Example 19:

(E)-1-(3-(4-amino-5-(7-methoxy-5-methylbenzothiophene-2-yl)-7H-pyrrole[2,3-d]pyrimi dine-7-yl)azetidin-1-yl)-4-(dimethylamino)but-2-en-1-one

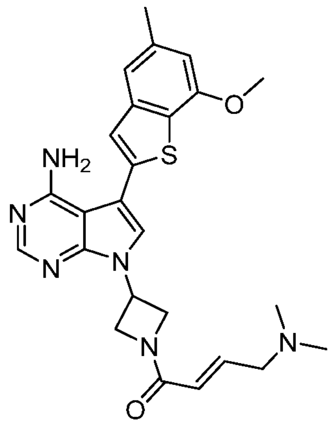

trans-4-(pyrrolidine-1-yl)crotonate hydrochloride was replaced with trans-4-(dimethylamino)crotonate hydrochloride, and the remaining required raw materials, reagents and preparation methods were the same as those in Example 17, thereby obtaining (E)-1-(3-(4-amino-5-(7-methoxy-5-methylbenzothiophene-2-yl)-7H-pyrrole[2,3-d]pyrimidine -7-yl)azetidin-1-yl)-4-(dimethylamino)but-2-en-1-one, yield 40%.
$^1$H NMR (300 MHz, $CDCl_3$) δ 8.29 (s, 1H), 7.35 (s, 1H), 7.23 (s, 2H), 7.00 - 6.86 (m, 1H), 6.64 (s, 1H), 6.32 (d, J = 15.8 Hz, 1H), 5.65 (s, 2H), 4.82 (s, 1H), 4.65 (s, 2H), 4.49 (s, 1H), 3.99 (s, 3H), 3.36 (d, J = 6.0 Hz, 2H), 2.47 (s, 9H), 1.25 (s, 4H).

Example 20:

1-(3-((4-amino-5-(7-methoxy-5-methylbenzothiophene-2-yl)-7H-pyrrole[2,3-d]pyrimidin e-7-yl)methyl)pyrrolidine-1-yl)prop-2-en-1-one

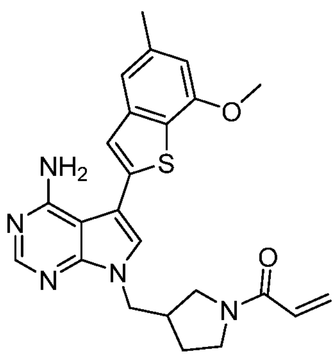

Step 1: Preparation of tert-butyl 3-((4-chloro-5-iodo-7H-pyrrole[2,3-d]pyrimidme-7-yl)methyl)pyrrolidme-1-formate

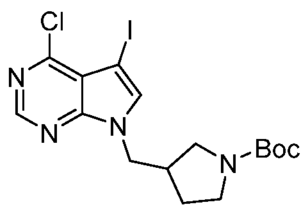

tert-butyl 3-hydroxypyrrolidine-1-formate was replaced with tert-butyl 3-hydroxymethylpyrrolidine-1-formate, and the remaining required raw materials, reagents and preparation methods were the same as those of Step 8 in Example 1, thereby obtaining tert-butyl 3-((4-chloro-5-iodo-7H-pyrrole[2,3-d]pyrimidine-7-yl)methyl)pyrrolidine-1-formate.

Step 2: Preparation of tert-butyl 3-((4-amino-5-iodo-7H-pyrrole[2,3-d]pyrimidine-7-yl)methyl)pyrrolidine-1-formate

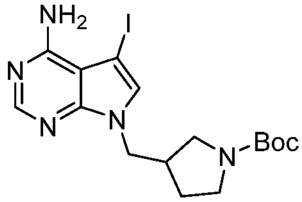

tert-butyl 3-(4-chloro-5-iodo-7H-pyrrole[2,3-d]pyrimidine-7-yl)pyrrolidine-1-formate was replaced with tert-butyl 3-((4-chloro-5-iodine-7H-pyrrole[2,3-d]pyrimidine-7-yl)methyl)pyrrolidine-1-formate, and the remaining required raw materials, reagents and preparation methods were the same as those of Step 9 in Example 1, thereby obtaining tert-butyl 3-((4-amino-5-iodo-7H-pyrrole[2,3-d]pyrimidine-7-yl)methyl)pyrrolidine-1-formate.

Step 3: Preparation of tert-butyl 3-((4-amino-5-(7-methoxy-5-methylbenzothiophene-2-yl)-7H-pyrrole[2,3-d]pyrimidine-7-yl) methyl)pyrrolidine-1-formate

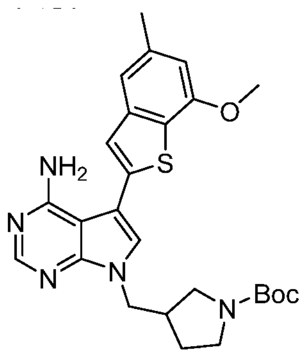

tert-butyl 3-(4-amino-5-iodo-7H-pyrrole[2,3-d]pyrimidine-7-yl)pyrrolidine-1-formate was replaced with tert-butyl 3-((4-amino-5-iodo-7H-pyrrole[2,3-d]pyrimidine-7-yl)methyl)pyrrolidine-1-formate, and the remaining required raw materials, reagents and preparation methods were the same as those of Step 10 in Example 1, thereby obtaining tert-butyl 3-((4-amino-5-(7-methoxy-5-methylbenzothiophene-2-yl)-7H-pyrrole[2,3-d]pyrimidine-7-yl)meth yl)pyrrolidine-1-formate.

Step 4: Preparation of 5-(7-methoxy-5-methylbenzothiophene-2-yl)-7-(pyrrolidine-3-methylene)-7H-pyrrole[2,3-d]pyrim idine-4-amine hydrochloride

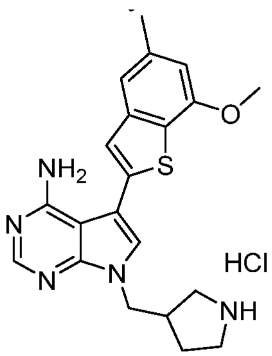

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-((4-amino-5-(7-methoxy-5-methylbenzothiophene-2-yl)-7H-pyrrole[2,3-d] pyrimidine-7-yl) methyl)pyrrolidine-1-formate, and the remaining required raw materials, reagents and preparation method were the same as those of Step 11 in Example 1, thereby obtaining 5-(7-methoxy-5-methylbenzothiothiophene-2-yl)-7-(pyrrolidine-3-methylene)-7H-pyrrole[2,3 -d]pyrimidine-4-amine hydrochloride.

Step 5: Preparation of 1-(3-((4-amino-5-(7-methoxy-5-methylbenzothiophene-2-yl)-7H-pyrrole [2,3-d]pyrimidine-7-yl)methyl)pyrrolidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxy-5-methylbenzothiophene-2-yl)-7-(pyrrolidine-3-methylene)-7H-pyrrole[2,3-d] pyrimidine-4-amine hydrochloride, and the remaining required raw materials, reagents and preparation methods were the same as those of Step 12 in Example 1, thereby obtaining 1-(3-((4-amino-5-(7-methoxy)-5-methylbenzothiophene-2-yl)-7H-pyrrole[2,3-d]pyrimidine-7 -yl)methyl)pyrrolidine-1-yl)prop-2-en-1-one, yield 37%.
$^1$H NMR (300 MHz, $CDCl_3$) $\delta$ 8.32 (s, 1H), 7.22 (s, 2H), 7.11 (d, J = 7.2 Hz, 1H), 6.64 (s, 1H), 6.39 (dd, J = 14.4, 7.6 Hz, 2H), 5.67 (dd, J = 9.9, 5.4 Hz, 1H), 5.55 (s, 2H), 4.38 - 4.15 (m, 2H), 4.00 (s, 3H), 3.75 (s, 2H), 3.65 - 3.47 (m, 1H), 3.38 (m, 1H), 2.89 (m, 1H), 2.49 (s, 3H), 2.17-1.96 (m, 1H).

**Example 21:**

**1-(3-(4-amino-5-(6-methoxybenzothiophene-2-yl)-7H-pyrrole[2,3-d]pyrimidine-7-yl)azet idin-1-yl)prop-2-en-1-one**

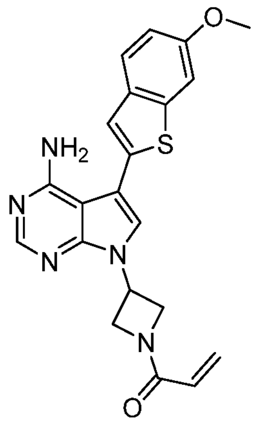

Step 1: Preparation of (2,2-diethoxyethyl)(3-methoxyphenyl)thioether

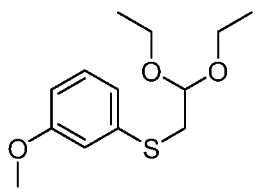

2-methoxy-4-methylthiophenol was replaced with 3-methoxythiophenol, and the remaining required raw materials, reagents and preparation methods were the same as those of Step 4 in Example 1, to obtain (2,2-diethoxyethyl)(3-methoxyphenyl)thioether.
$^1$H NMR (300 MHz, $CDCl_3$) 7.19 (t, J = 7.9 Hz, 1H), 6.94 (m, 2H), 6.75 - 6.68 (m, 1H), 4.65 (t, J = 5.5 Hz, 1H), 3.79 (s, 3H), 3.68 (m, 2H), 3.55 (m, 2H), 3.14 (d, J = 5.6 Hz, 2H), 1.20 (t, J = 7.0 Hz, 6H).

Step 2: Preparation of 6-methoxybenzothiophene

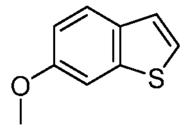

(2,2-diethoxyethyl)(2-methoxy-4-methylphenyl)thioether was replaced with (2,2-diethoxyethyl)(3-methoxyphenyl)thioether, the remaining required raw materials, reagents and preparation methods were the same as those of Step 5 in Example 1, to obtain 6-methoxybenzothiophene.
$^1$H NMR (300 MHz, $CDCl_3$) δ 7.70 (d, J = 8.7 Hz, 1H), 7.36 (d, J = 2.0 Hz, 1H), 7.00 (dd, J = 8.7, 2.0 Hz, 1H), 3.88 (s, 3H).

Step 3: Preparation of (6-methoxybenzothiophene-2-yl)boronic acid

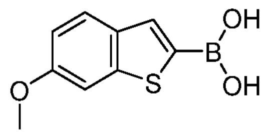

7-methoxy-5-methylbenzothiophene was replaced with 6-methoxybenzothiophene, and the remaining required raw materials, reagents and preparation methods were the same as those of Step 6 in Example 1, thereby obtaining (6-methoxybenzothiophene-2-yl)boronic acid.

Step 4: Preparation of tert-butyl 3-(4-amino-5-(6-methoxybenzothiophene-2-yl)-7H-pyrrole[2,3-d]pyrimidine-7-yl)azetidin-1-form ate

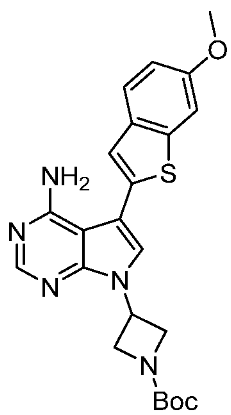

tert-butyl 3-(4-amino-5-iodo-7H-pyrrole[2,3-d]pyrimidine-7-yl)pyrrolidine-1-formate was replaced with tert-butyl 3-(4-amino-5-iodo-7H-pyrrole[2,3-d]pyrimidine-7-yl)azetidin-1-formate, and (7-methoxy-5-methylbenzothiophene-2-yl)boric acid was replaced with (6-methoxybenzothiophene-2-yl)boronic acid, and the remaining required raw materials, reagents and preparation methods were the same as those of Step 10 in Example 1, thereby obtaining tert-butyl 3-(4-amino-5-(6-methoxybenzothiophene-2-yl)-7H-pyrrole[2,3-d]pyrimidine-7-yl)azetidin-1-form ate.

Step 5: Preparation of 5-(6-methoxybenzothiophene-2-yl)-7-(azetidin-3-yl)-7H-pyrrole[2,3-d]pyrimidine-4-amine hydrochloride

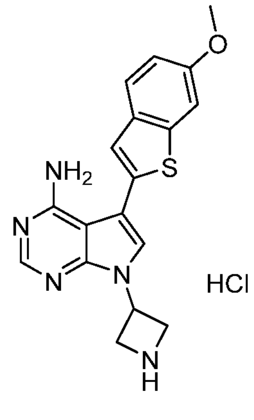

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-5-(6-methoxybenzothiophene-2-yl)-7H-pyrrole[2,3-d]pyrimidine -7-yl)azetidin-1-formate, and the remaining required raw materials, reagents and preparation methods were the same as those of Step 11 in Example 1, thereby obtaining 5-(6-methoxybenzothiophene-2-yl)-7-(azetidin-3-yl)-7H-pyrrole[2,3-d]pyrimidine-4-amine hydrochloride.

Step 6: Preparation of 1-(3-(4-amino-5-(6-methoxybenzothiophene-2-yl)-7H-pyrrole[2,3-d]pyrimidine-7-yl)azetidin -1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(6-methoxybenzothiophene-2-yl)-7-(azetidin-3-yl)-7H-pyrrole[2,3-d]pyrimidine-4-amine hydrochloride, and the remaining required raw materials, reagents and preparation methods were the same as those of Step 12 in Example 1, thereby obtaining 1-(3-(4-amino-5-(6-methoxybenzothiophene-2-) -7H-pyrrole[2,3-d]pyrimidine-7-yl)azetidin-1-yl)prop-2-en-1-one, yield 65%.
$^1$H NMR (300 MHz, $CDCl_3$) δ 8.32 (s, 1H), 7.68 (d, J = 8.7 Hz, 1H), 7.34 (s, 2H), 7.24 (s, 1H), 7.07 - 7.00 (m, 1H), 6.42 (d, J = 17.0 Hz, 1H), 6.25 (dd, J = 16.9, 10.3 Hz, 1H), 5.73 (dd, J = 16.6, 8.0 Hz, 2H), 5.52 (s, 2H), 4.77 (d, J = 7.6 Hz, 1H), 4.72 - 4.62 (m, 1H), 4.53 (s, 2H), 3.90 (s, 3H).

Example 22:

1-(3-(4-amino-5-(7-methoxybenzothiophene-2-yl)-7H-pyrrole[2,3-d]pyrimidine-7-yl)azet idin-1-yl)prop-2-en-1-one

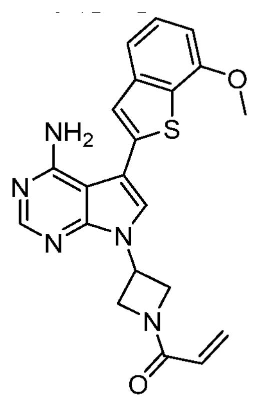

Step 1: Preparation of (2,2-diethoxyethyl)(2-methoxyphenyl)thioether

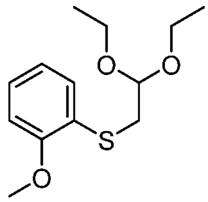

2-methoxy-4-methylthiophenol was replaced with 2-methoxythiophenol, and the remaining required raw materials, reagents and preparation methods were the same as those of Step 4 in Example 1, to obtain (2,2-diethoxyethyl)(2-methoxyphenyl)thioether.

Step 2: Preparation of 7-methoxybenzothiophene

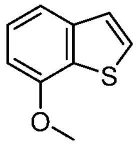

(2,2-diethoxyethyl)(2-methoxy-4-methylphenyl)thioether was replaced with (2,2-diethoxyethyl)(2-methoxyphenyl)thioether, and the remaining required raw materials, reagents and preparation methods were the same as those of Step 5 in Example 1, to obtain 7-methoxybenzothiophene.
$^1$H NMR (300 MHz, $CDCl_3$) δ 7.49 - 7.41 (m, 2H), 7.33 (m, 2H), 6.78 (d, J = 7.9 Hz, 1H), 4.01 (s, 3H).

Step 3: Preparation of (7-methoxybenzothiophene-2-yl)boronic acid

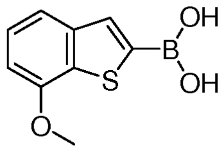

7-methoxy-5-methylbenzothiophene was replaced with 7-methoxybenzothiophene, and the remaining required raw materials, reagents and preparation methods were the same as those of Step 6 in Example 1, thereby obtaining (6-methoxybenzothiophene-2-yl)boronic acid.

Step 4: Preparation of tert-butyl 3-(4-amino-5-(6-methoxybenzothiophene-2-yl)-7H-pyrrole[2,3-d]pyrimidine-7-yl)azetidin-1-form ate

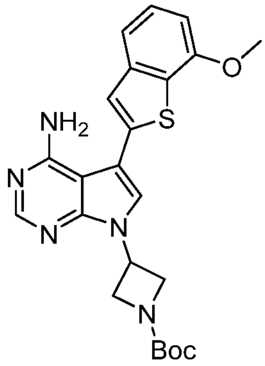

tert-butyl 3-(4-amino-5-iodo-7H-pyrrole[2,3-d]pyrimidine-7-yl)pyrrolidine-1-formate was replaced with 3-(4-amino-5-iodo-7H-pyrrole[2,3-d]pyrimidine-7-yl)azetidin-1-tert-butyl formate, (7-methoxy-5-methylbenzothiophene-2-yl)boric acid was replaced with (7-methoxybenzothiophene-2-yl)boronic acid, and the remaining required raw materials, reagents and preparation methods were the same as those of Step 10 in Example 1, thereby obtaining tert-butyl 3-(4-amino-5-(7-methoxybenzothiophene-2-yl)-7H-pyrrole[2,3-d]pyrimidine-7-yl)azetidin-1-form ate.

Step 5: Preparation of 5-(7-methoxybenzothiophene-2-yl)-7-(azetidin-3-yl)-7H-pyrrole[2,3-d]pyrimidine-4-amine hydrochloride

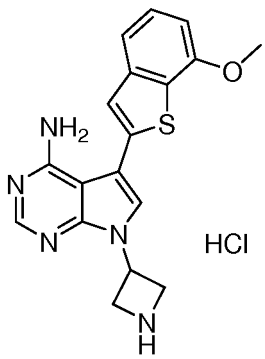

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-5-(7-methoxybenzothiophene-2-yl)-7H-pyrrole[2,3-d]pyrimidine -7-yl)azetidin-1-formate, and the remaining required raw materials, reagents and preparation methods were the same as those of Step 11 in Example 1, thereby obtaining 5-(7-methoxybenzothiophene-2-yl)-7-(azetidin-3-yl)-7H-pyrrole[2,3-d]pyrimidine-4-amine hydrochloride.

Step 6: Preparation of 1-(3-(4-amino-5-(7-methoxybenzothiophene-2-yl)-7H-pyrrole[2,3-d]pyrimidine-7-yl)azetidin -1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxybenzothiophene-2-yl)-7-(azetidin-3-yl)-7H-pyrrole[2,3-d]pyrimidine-4-amine hydrochloride, and the remaining required raw materials, reagents and preparation methods were the same as those of Step 12 in Example 1, thereby obtaining 1-(3-(4-amino-5-(7-methoxybenzothiophene-2-)-7H-pyrrole[2,3-d]pyrimidine-7-yl)azetidin-1 -yl)prop-2-en-1-one, yield 55%.
$^1H$ NMR (300 MHz, $CDCl_3$) δ 8.31 (s, 1H), 7.42 (d, J = 7.6 Hz, 1H), 7.37 (t, J = 3.8 Hz, 2H), 7.33 (d, J = 3.3 Hz, 1H), 6.81 (d, J = 7.5 Hz, 1H), 6.42 (dd, J = 16.9, 1.6 Hz, 1H), 6.24 (dd, J = 17.0, 10.2 Hz, 1H), 5.80 - 5.57 (m, 4H), 4.78- 4.62 (m, 2H), 4.55 (m, 2H), 4.02 (s, 3H).

**Example 23:**

**1-(3-(4-amino-5-(7-methoxy-5-methylbenzofuran-2-yl)-7H-pyrrole[2,3-d]pyrimidine-7-y l)azetidine-1-yl)prop-2-en-1-one**

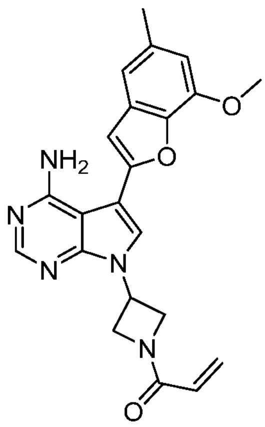

Step 1: Preparation of 1-(2,2-diethoxyethoxy)-2-methoxy-4-methylbenzene

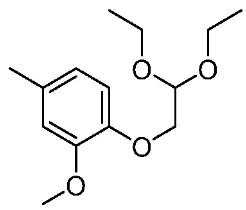

2-methoxy-4-methylthiophenol was replaced with 2-methoxy-4-methylphenol, and the remaining required raw materials, reagents and preparation methods were the same as those of Step 4 in Example 1, to obtain 1-(2, 2-diethoxyethoxy)-2-methoxy-4-methylbenzene.
$^1$H NMR (300 MHz, $CDCl_3$) δ 6.83 (d, *J* = 7.9 Hz, 1H), 6.68 (d, *J* = 10.8 Hz, 2H), 4.86 (t, *J* = 5.1 Hz, 1H), 4.03 (d, *J* = 5.2 Hz, 2H), 3.83 (s, 3H), 3.74- 3.57 (m, 4H), 2.29 (s, 3H), 1.23 (t, *J* = 7.0 Hz, 6H).

Step 2: Preparation of 7-methoxy-5-methylbenzofuran

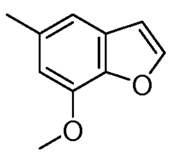

(2,2-diethoxyethyl)(2-methoxy-4-methylphenyl)thioether was replaced with 1-(2,2-diethoxyethoxy)-2-methoxy-4-methylbenzene, and the remaining required raw materials, reagents and preparation methods were the same as those of Step 5 in Example 1, to obtain 7-methoxy-5-methylbenzofuran.

Step 3: Preparation of (7-methoxy-5-methylbenzofuran-2-yl)boronic acid

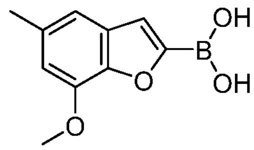

7-methoxy-5-methylbenzothiophene was replaced with 7-methoxybenzofuran, and the remaining required raw materials, reagents and preparation methods were the same as those of Step 6 in Example 1, to obtain (7-methoxy-5-methylbenzofuran-2-yl)boronic acid.

Step 4: Preparation of tert-butyl 3-(4-amino-5-(7-methoxy-5-methylbenzofuran-2-yl)-7H-pyrrole[2,3-d]pyrimidine-7-yl)azetidine-1 -formate

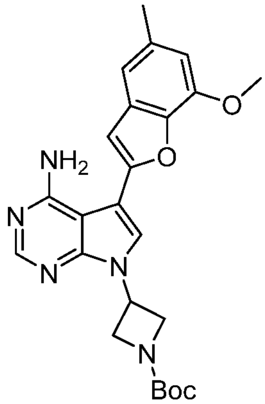

tert-butyl 3-(4-amino-5-iodo-7H-pyrrole[2,3-d]pyrimidine-7-yl)pyrrolidine-1-formate was replaced with tert-butyl 3-(4-amino-5-iodo-7H-pyrrole[2,3-d]pyrimidine-7-yl)azetidine-1-formate, (7-methoxy-5-methylbenzothiophene-2-yl)boric acid was replaced with (7-methoxy-5-methylbenzofuran-2-yl)boronic acid, and the remaining required raw materials, reagents and preparation methods were the same as those of Step 10 in Example 1, to obtain tert-butyl 3-(4-amino-5-(7-methoxy-5-methylbenzofuran-2-yl)-7H-pyrrole[2,3-d]pyrimidine-7-yl)azetidine-1 -formate.

Step 5: Preparation of 5-(7-methoxy-5-methylbenzofuran-2-yl)-7-(azetidine-3-yl)-7H-pyrrole[2,3-d]pyrimidine-4-a mine hydrochloride

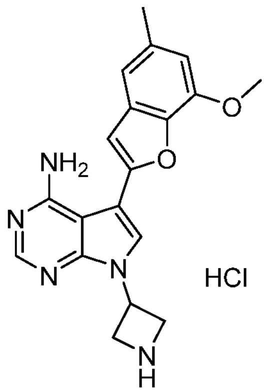

tert-butyl 3-(4-amino-5-(7-methoxy-5-methylbenzothiophene-2-yl)-7H-pyrrole[2,3-d]pyrimidine-7-yl)p yrrolidine-1- formate was replaced with tert-butyl 3-(4-amino-5-(7-methoxy-5-methylbenzofuran-2-yl)-7H-pyrrole[2,3-d]pyrimidine-7-yl)azeti dine-1-formate, and the remaining required raw materials, reagents and preparation methods were the same as those of Step 11 in Example 1, to obtain 5-(7-methoxy-5-methylbenzofuran-2-yl)-7-(azetidine-3-yl)-7H-pyrrole[2,3-d]pyrimidine-4-a mine hydrochloride.

Step 6: Preparation of 1-(3-(4-amino-5-(7-methoxy-5-methylbenzofuran-2-yl)-7H-pyrrole[2,3-d]pyrimidine-7-yl)az etidine-1-yl)prop-2-en-1-one

5-(7-methoxy-5-methylbenzothiophene-2-yl)-7-(pyrrolidine-3-yl)-7H-pyrrole[2,3-d]pyri midine-4 -amino hydrochloride was replaced with 5-(7-methoxy-5-methylbenzofuran-2-yl)-7-(azetidine-3-yl)-7H-pyrrole[2,3 -d]pyrimidine-4-amine hydrochloride, and the remaining required raw materials, reagents and preparation methods were the same as those of Step 12 in Example 1, to obtain 1-(3-(4-amino-5-(7-methoxy)-5-methylbenzofuran-2-yl)-7H-pyrrole[2,3-d]pyrimidine-7-yl)azetidine-1-yl)prop-2-en-1-one, yield 56%.
$^1$H NMR (300 MHz, $CD_3OD$) δ 8.14 (s, 1H), 8.00 (s, 1H), 6.94 (s, 2H), 6.71 (s, 1H), 6.38 (m, 2H), 5.79 (d, J = 10.0 Hz, 1H), 5.63 (s, 1H), 4.85-4.54 (m, 4H), 3.98 (s, 3H), 2.42 (s, 3H).

**Example 24:**

**1-(2-((4-amino-5-(7-methoxy-5-methylbenzothiophene-2-yl)-7H-pyrrole[2,3-d]pyrimidin e-7-yl)methyl)pyrrolidine-1-yl)prop-2-en-1-one**

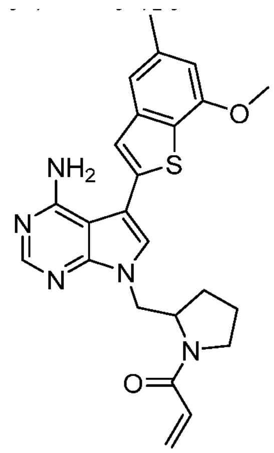

Step 1: Preparation of tert-butyl 2-((4-chloro-5-iodo-7H-pyrrole[2,3-d]pyrimidine-7-yl)methyl)pyrrolidine-1-formate

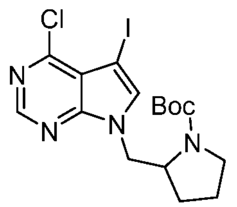

tert-butyl 3-hydroxypyrrolidine-1-formate was replaced with tert-butyl 2-hydroxymethylpyrrolidine-1-formate, and the remaining required raw materials, reagents and preparation methods were the same as those of Step 8 in Example 1, thereby obtaining tert-butyl 2-((4-chloro-5-iodo-7H-pyrrole[2,3-d]pyrimidine-7-yl)methyl)pyrrolidine-1-formate.

Step 2: Preparation of 2-((4-amino-5-iodo-7H-pyrrole[2,3-d]pyrimidine-7-yl)methyl)pyrrolidine-1-tert-butyl formate

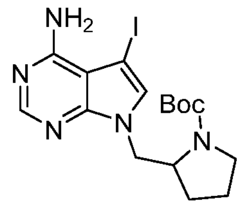

tert-butyl 3-(4-chloro-5-iodo-7H-pyrrole[2,3-d]pyrimidine-7-yl)pyrrolidine-1-formate was replaced with tert-butyl 2-((4-chloro-5-iodine-7H-pyrrole[2,3-d]pyrimidine-7-yl)methyl)pyrrolidine-1-formate, and the remaining required raw materials, reagents and preparation methods were the same as those of Step 9 in Example 1, thereby obtaining tert-butyl 2-((4-amino-5-iodo-7H-pyrrole[2,3-d]pyrimidine-7-yl)methyl)pyrrolidine-1-formate.

Step 3: Preparation of tert-butyl 2-((4-amino-5-(7-methoxy-5-methylbenzothiophene-2-yl)-7H-pyrrole[2,3-d]pyrimidine-7-yl) methyl)pyrrolidine-1-formate

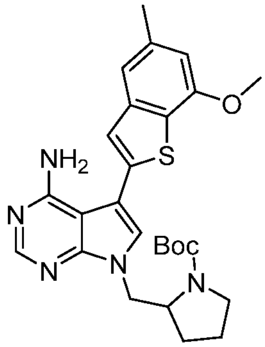

tert-butyl 3-(4-amino-5-iodo-7H-pyrrole[2,3-d]pyrimidine-7-yl)pyrrolidine-1-formate was replaced with tert-butyl 2-((4-amino-5-iodo-7H-pyrrole[2,3-d]pyrimidine-7-yl)methyl)pyrrolidine-1-formate, and the remaining required raw materials, reagents and preparation methods were the same as those of Step 10 in Example 1, thereby obtaining tert-butyl 2-((4-amino-5-(7-methoxy-5-methylbenzothiophene-2-yl)-7H-pyrrole[2,3-d]pyrimidine-7-yl)meth yl)pyrrolidine-1-formate.

Step 4: Preparation of 5-(7-methoxy-5-methylbenzothiophene-2-yl)-7-(pyrrolidine-2-methylene)-7H-pyrrole[2,3-d]pyrim idine-4-amine hydrochloride

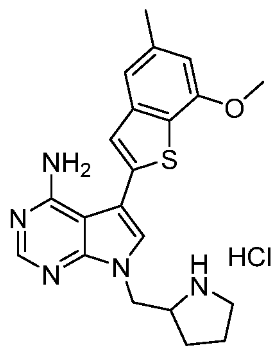

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 2-((4-amino-5-(7-methoxy-5-methylbenzothiophene-2-yl)-7H-pyrrole[2,3-d] pyrimidine-7-yl) methyl)pyrrolidine-1-formate, and the remaining required raw materials, reagents and preparation method were the same as those of Step 11 in Example 1, thereby obtaining 5-(7-methoxy-5-methylbenzothiothiophene-2-yl)-7-(pyrrolidine-2-methylene)-7H-pyrrole[2,3 -d]pyrimidine-4-amine hydrochloride.

Step 5: Preparation of 1-(2-((4-amino-5-(7-methoxy-5-methylbenzothiophene-2-yl)-7H-pyrrole [2,3-d]pyrimidine-7-yl)methyl)pyrrolidine-1-yl)prop-2-en-1-one

5-(7-methoxy-5-methylbenzothiophene-2-yl)-7-(pyrrolidine-3-yl)-7H-pyrrole[2,3-d]pyri midine-4-amino hydrochloride was replaced with 5-(7-methoxy-5-methylbenzothiophene-2-yl)-7-(pyrrolidine-2-methylene)-7H-pyrrole[2,3-d] pyrimidine-4-amine hydrochloride, and the remaining required raw materials, reagents and preparation methods were the same as those of Step 12 in Example 1, thereby obtaining 1-(2-((4-amino-5-(7-methoxy)-5-methylbenzothiophene-2-yl)-7H-pyrrole[2,3-d]pyrimidine-7-yl)methyl)pyrrolidine-1-yl)pr op-2-en-1-one, yield 61%.
$^1H$ NMR (300 MHz, $CDCl_3$) δ 8.34 (d, J = 9.2 Hz, 1H), 7.23 - 7.16 (m, 3H), 6.63 (s, 1H), 6.47 (d, J = 8.7 Hz, 2H), 5.75 (dd, J = 8.2, 3.6 Hz, 1H), 5.55 (m, 3H), 4.53 (s, 3H), 3.99 (s, 3H), 3.55 - 3.43 (m, 2H), 2.48 (s, 3H), 1.93 (m, 4H).

**Example 25:**

**2-(3-(4-amino-5-(7-methoxy-5-methylbenzothiophene-2-yl)-7H-pyrrole[2,3-d]pyrimidine -7-yl)pyrrolidine-1-carbonyl)-3-cyclopropyl acrylonitrile**

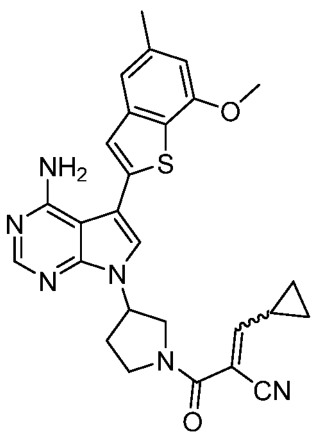

Step 1: Preparation of 3-(3-(4-amino-5-(7-methoxy-5-methylbenzothiophene-2-yl)-7H-pyrrole[2,3-d]pyrimidine -7-yl)pyrrolidme-1-yl)-3-oxopropiomtrile

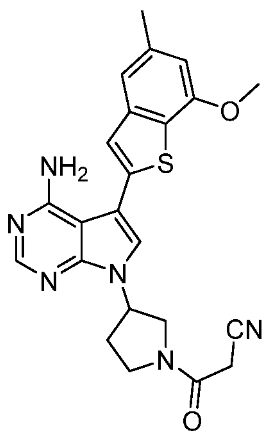

trans-4-(pyrrolidine-1-yl) crotonate hydrochloride was replaced with cyanoacetic acid, and the remaining required raw materials, reagents and preparation methods were the same as those of Example 17, to give 3-(3-(4-amino-5-(7-methoxy-5-methylbenzothiophene-2-yl)-7H-pyrrole[2,3-d]pyrimidine-7-yl)pyrrolidine-1-yl)-3-oxopropionitrile.
$^1$H NMR (300 MHz, DMSO-$d_6$) δ 8.29 (s, 1H), 7.69 m, 1H), 7.35 (s, 1H), 7.28 (s, 1H), 6.96 (s, 2H), 6.81 (s, 1H), 5.39 (m, 1H), 4.05-3.30 (m, 11H), 2.44 (s, 3H).

Step 2: Preparation of 2-(3-(4-amino-5-(7-methoxy-5-methylbenzothiophene-2-yl)-7H-pyrrole[2,3-d]pyrimidine -7-yl)azetidine-1-carbonyl)-3-cyclopropyl acrylonitrile

50 mg of 3-(3-(4-amino-5-(7-methoxy-5-methylbenzothiophene-2-yl)-7H-pyrrole[2,3-d]pyrimidine-7-yl)pyrrolidine-1-yl)-3-oxopropanenitrile, 17 μl of cyclopropylformaldehyde and 10 μl of piperidine were dissolved in 1 ml of methanol, stirred at room temperature overnight. Solids precipitated and were filtered, and the filter cake was dried to obtain 13 mg of beige solids, yield 80%.
$^1$H NMR (300 MHz, $CDCl_3$) δ 8.32 (s, 1H), 7.22 (s, 2H), 7.11 (s, 1H), 6.91 (m, 1H), 6.64 (s, 1H), 5.62 (s, 2H), 5.48 (s, 1H), 4.38 - 4.34 (m, 0.5H), 4.00 (s, 3H), 3.85-4.1(m, 2.5H), 3.80 - 3.68 (m, 1H), , 2.48 (s, 3H), 2.6 - 2.43 (m, 2H), 1.3-1.25(m, 1H), 1.0-0.8(m, 4H).

**Example 26: 1-(3-(4-amino-3-(7-methoxy-5-methylbenzothiophene-2-yl)-1H-pyrazole[3,4-d]pyrimidi ne-1-yl)azetidine-1-yl)prop-2-en-1-one**

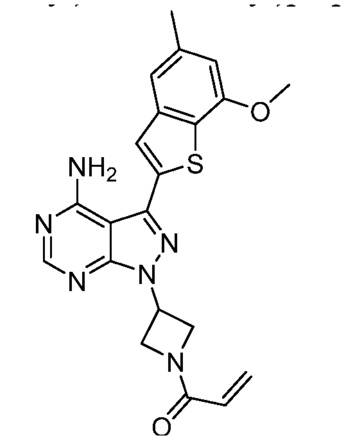

Step 1: Preparation of tert-butyl 3-((methylsulfonyl)oxy)azetidine-1-formate

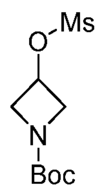

tert-butyl 3-hydroxypyrrolidine-1-formate was replaced with tert-butyl 3-hydroxyazetidine-1-formate, and the remaining required raw materials, reagents and preparation methods were the same as those of Step 3 in Example 2, to obtain tert-butyl 3-((methylsulfonyl)oxy)azetidine-1-formate.
$^1$H NMR (300 MHz, $CDCl_3$) δ 5.23-5.04 (m, 1H), 4.23 (m, 2H), 4.05 (m, 2H), 3.03 (s, 3H), 1.40 (s, 9H).

Step 2: Preparation of tert-butyl 3-(4-amino-3-iodo-1H-pyrazol[3,4-d]pyrimidine-1-yl)azetidine-1-formate

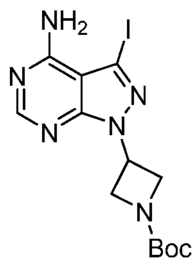

tert-butyl 3-((methylsulfonyl)oxy)pyrrolidine-1-formate was replaced with tert-butyl 3-((methylsulfonyl)oxy)azetidine-1-formate, and the remaining required raw materials, reagents and preparation methods were the same as those of Step 4 in Example 2, to obtain tert-butyl 3-(4-amino-3-iodo-1H-pyrazolo[3,4-d]pyrimidine-1-yl)azetidine-1-formate.

Step 3: Preparation of tert-butyl 3-(4-amino-3-(7-methoxy-5-methylbenzothiophene-2-yl)-1H-pyrazole [3,4-d]pyrimidine-1-yl)azetidine-1-formate

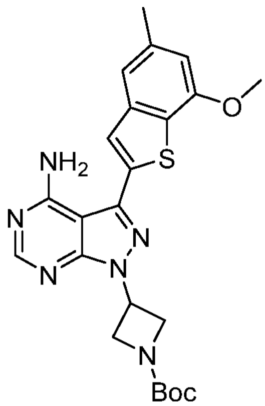

tert-butyl 3-(4-amino-5-iodo-7H-pyrrole[2,3-d]pyrimidine-7-yl)pyrrolidine-1-formate was replaced with tert-butyl 3-(4-amino-3-iodo-1H-pyrazol[3,4-d]pyrimidine-1-yl)azetidine-1-formate, and the remaining required raw materials, reagents and preparation methods were the same as those of Step 10 in Example 1, to give tert-butyl 3-(4-amino-3-(7-methoxy-5-methylbenzothiophene-2-yl)-1H-pyrazole[3,4-d]pyrimidine-1-yl)azeti dine-1-formate.

Step 4: Preparation of 3-(7-methoxy-5-methylbenzothiophene-2-yl)-1-(azetidine-3-yl)-1H-pyrazole[3,4-d]pyrimidine-4-a mine hydrochloride

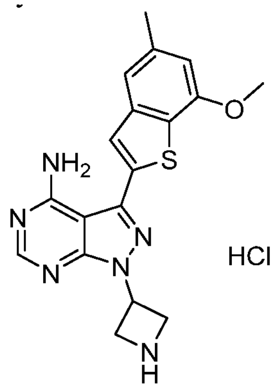

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 3-(4-amino-3-(7-methoxy-5-methylbenzothiophene-2-yl)-1H-pyrazole [3,4-d] pyrimidine-1-yl)azetidine-1-formate, and the remaining required raw materials, reagents and preparation methods were the same as those of Step 11 in Example 1, to give 3-(7-methoxy-5-methylbenzothiophene-2-yl)-1-(azetidine-3-yl)-1H-pyrazolo[3,4-d]pyrimidin e-4-amine hydrochloride.

Step 5: Preparation of 1-(3-(4-amino-3-(7-methoxy-5-methylbenzothiophene-2-yl)-1H-pyrazole [3,4-d]pyrimidine-1-yl)azetidine-1-yl)propyl-2-en-1-one

5-(7-methoxy-5-methylbenzothiophene-2-yl)-7-(pyrrolidine-3-yl)-7H-pyrrole[2,3-d]pyri midine-4 -amino hydrochloride was replaced with 3-(7-methoxy-5-methylbenzothiophene-2-yl)-1-(azetidine-3-yl)-1H-pyrazole[3,4-d]pyrimidin e-4-amine hydrochloride, and the remaining required raw materials, reagents and preparation methods were the same as those of Step 12 in Example 1, to give 1-(3-(4-amino-3-(7-methoxy-5-methylbenzothiophene-2-yl)-1H-pyrazolo[3,4-d]pyrimidine-1 -yl)azetidine-1-yl)propyl-2-en-1-one, yield 54%.
$^1$H NMR (300 MHz, DMSO-$d_6$) δ 8.29 (s, 1H), 7.71 (s, 1H), 7.36 (s, 1H), 6.86 (s, 1H), 6.40 (dd, J = 17.0, 10.5 Hz, 1H), 6.17 (d, J = 17.2 Hz, 1H), 5.73 (d, J = 11.7 Hz, 2H), 4.76-4.67 (m, 2H), 4.53-4.35 (m, 2H), 3.96 (s, 3H), 2.45 (s, 3H).

**Example 27: 1-(3-(4-amino-3-(7-methoxy-5-methylbenzothiophene-2-yl)-1H-pyrazole[3,4-d]pyrimidi ne-1-yl)azetidine-1-yl)prop-2-yn-1-one**

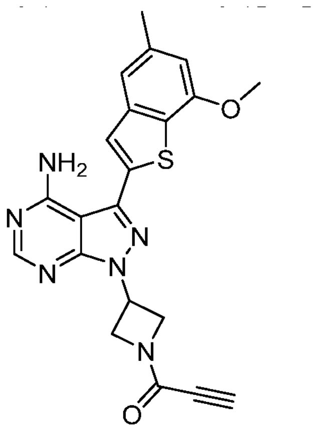

100 mg of 3-(7-methoxy-5-methylbenzothiophene-2-yl)-1-(azetidine-3-yl)-1H-pyrazole[3,4-d]pyrimidin e-4-amine hydrochloride, 20 μl of propiolic acid and 145 mg of benzotriazol-1-tris(trimethylamino)-trifluorophosphate were dissolved in 2 ml of N,N-dimethylformamide, 0.2 ml of N,N-diisopropylethylamine was added dropwise, and the mixture was stirred at room temperature for 8 hours to quench the reaction. Water was added, and the mixture was extracted with ethyl acetate. The organic phase was washed with a saturated aqueous solution of sodium chloride and dried over anhydrous sodium sulfate, filtered and concentrated, and the residue was isolated by column chromatography (dichloromethane: methanol = 97:3) to obtain 48 mg of light yellow solids, yield 44%.
$^1$H NMR (300 MHz, DMSO-$d_6$) δ 8.30 (s, 1H), 7.72 (s, 1H), 7.36 (s, 1H), 6.87 (s, 1H), 5.76 (s, 1H), 4.73-4.58 (m, 2H), 4.55 (s, 1H), 4.53-4.37 (m, 1H), 3.97 (s, 3H), 2.45 (s, 3H).

**Example 28:**

**5-(7-methoxy-5-methylbenzothiophene-2-yl)-7-(1-(vinylsulfonyl)azetidine-3-yl)-7H-pyrr** ole**[2,3-d]pyrimidine-4-amine**

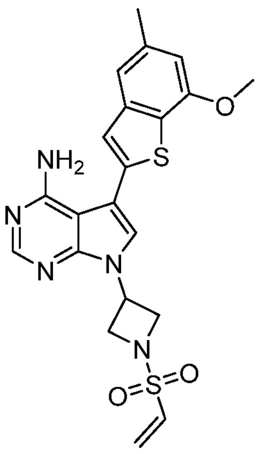

100 mg of 5-(7-methoxy-5-methylbenzothiophene-2-yl)-7-(azetidine-3-yl)-7H-pyrrole[2,3-d]pyrimidine -4-amine hydrochloride was suspended in 2 ml of dry dichloromethane, 0.18 ml of triethylamine was added, and 31 μl of 2-chloroethylsulfonyl chloride was added dropwise under ice cooling. Upon addition, the mixture was stirred at room temperature overnight, and the reaction mixture was evaporated to dryness on the next day. The residue was isolated by column chromatography (dichloromethane: methanol = 97:3) to obtain 46 mg of white solids, yield 52%.
$^1$H NMR (300 MHz, $CDCl_3$) δ 8.30 (s, 1H), 7.39 (s, 1H), 7.24 (s, 2H), 6.73 (dd, J = 16.6, 9.9 Hz, 1H), 6.65 (s, 1H), 6.43 (d, J = 16.6 Hz, 1H), 6.24 (d, J = 9.9 Hz, 1H), 5.62-5.54 (m, 3H), 4.45-4.35(m, 4H), 4.01 (s, 3H), 2.49 (s, 3H).

**Example 29: 1-(3-(4-amino-5-(7-methoxy-5-methylbenzothiophene-2-yl)-7H-pyrrole[2,3-d]pyrimidine -7-yl)azetidine-1-yl)-2-fluoro-prop-2-yn-1-one**

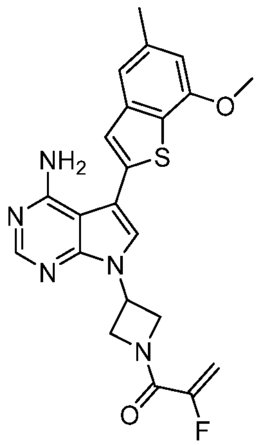

60 mg of 5-(7-methoxy-5-methylbenzothiophene-2-yl)-7-(azetidine-3-yl)-7H-pyrrole[2,3-d]pyrimidine -4-amine hydrochloride, 17 mg of 2-fluoro-propynoic acid and 80 mg of benzotriazol-1-tris(trimethylamino)-trifluorophosphate were dissolved in 2 ml of N,N-dimethylformamide, 0.12 ml of N,N-diisopropylethylamine was added dropwise, and the mixture was stirred at room temperature for 8 hours to quench the reaction. Water was added, and then the mixture was extracted with ethyl acetate. The organic phase was washed with a saturated aqueous solution of sodium chloride and dried over anhydrous sodium sulfate, filtered and concentrated, and the residue was isolated by column chromatography (dichloromethane: methanol = 97:3) to obtain 46 mg of light yellow solids, yield 61%.
$^1$H NMR (300 MHz, DMSO-$d_6$) δ 8.19 (s, 1H), 7.97 (s, 1H), 7.35 (s, 1H), 7.28 (s, 1H), 6.80 (s, 1H), 6.53 (s, 2H), 5.70-60 (m, 2H), 5.39-5.26 (m, 1H), 4.85 (s, 2H), 4.56-4.33 (m, 2H), 3.95 (s, 3H), 2.43 (s, 3H).

**Example 30: 1-(3-(4-amino-5-(1H-indol-2-yl)-7H-pyrrole[2,3-d]pyrimidine-7-yl)azetidine-1-yl)prop-2 -en-1-one**

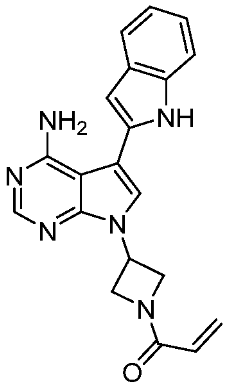

Step 1: Preparation of tert-butyl 1H-mdole-1-formate

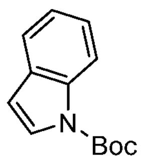

200 mg of indole, 0.7 ml of triethylamine and 48 mg of 4-dimethylaminopyridine were dissolved in 2 ml of dichloromethane, and then 375 mg of di-tert-butyl dicarbonate was added, placed at room temperature and stirred for 1 hour. The reaction mixture was concentrated and isolated by column chromatography (petroleum ether 100%) to obtain 370 mg of oily matter.
$^1$H NMR (300 MHz, $CDCl_3$) δ 8.16 (d, J = 8.0 Hz, 1H), 7.59 (dd, J = 10.5, 5.6 Hz, 2H), 7.32 (td, J = 8.0, 1.3 Hz, 1H), 7.27-7.19 (m, 1H), 6.58 (d, J = 3.7 Hz, 1H), 1.68 (s, 9H).

Step 2: Preparation of (1-tert-butyl formate-1H-indol-2-yl)boronic acid

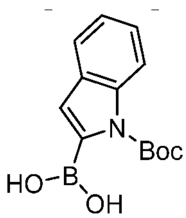

Under argon atmosphere, 370 mg of tert-butyl 1H-indole-1-formate and 0.6 ml of triisopropyl borate were dissolved in 3 ml of dry tetrahydrofuran, and 1.2 equivalents of lithium diisopropylamide were added dropwise under ice cooling, stirred for 2 hours in an ice-bath, and the mixture was quenched with 3 ml of 1N aqueous hydrochloric acid. Water was added, and then the mixture was extracted with ethyl acetate. The organic phase was washed with a saturated aqueous solution of sodium chloride and dried over anhydrous sodium sulfate, filtered and concentrated, and the obtained was directly used in the next step.

Step 3: Preparation of tert-butyl 2-(4-amino-7-(1-(tert-butyl formate)azetidine-3-yl)-7H-pyrrole[2,3-d]pyrimidine-5-yl)-1H-indole-1-formate

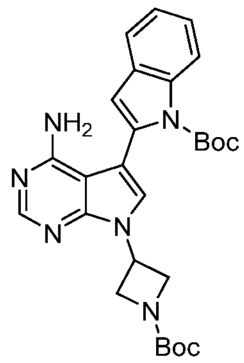

tert-butyl 3-(4-amino-5-iodo-7H-pyrrole[2,3-d]pyrimidin-7-yl)pyrrolidine-1-formate was replaced with tert-butyl 3-(4-amino-5-iodo-7H-pyrrole[2,3-d]pyrimidine-7-yl)azetidine-1-formate, (7-methoxy-5-methylbenzothiophene-2-yl)boric acid was replaced with (1-tert-butyl formate-1H-indol-2-yl)boronic acid, and the remaining required raw materials, reagents and preparation methods were the same as those of Step 10 in Example 1, to obtain tert-butyl 2-(4-amino-7-(1-(tert-butyl formate))azetidine-3-yl)-7H-pyrrole[2,3-d]pyrimidine-5-yl)-1H-indole-1-formate.
$^1$H NMR (300 MHz, $CDCl_3$) δ 8.32 (s, 1H), 8.29 (d, J = 8.2 Hz, 1H), 7.59 (d, J = 7.3 Hz, 1H), 7.42 - 7.29 (m, 3H), 6.68 (s, 1H), 5.65-5.56 (m, 1H), 4.97 (s, 2H), 4.55-4.49 (m, 2H), 4.27-4.22 (m, 2H), 1.48 (s, 9H), 1.19 (s, 9H).

Step4: Preparation of 7-(azetidine-3-yl)-5-(1H-indol-2-yl)-7H-pyrrole[2,3-d]pyrimidine-4-amine hydrochloride

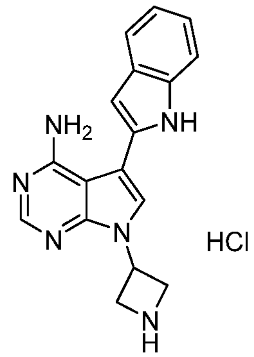

tert-butyl 3-(4-amino-5-(7-methoxy-5-methylbenzothiophene-2-yl)-7H-pyrrole[2,3-d]pyrimidine-7-yl)p yrrolidine-1-formate was replaced with tert-butyl 2-(4-amino-7-(1-tert-butyl formate)azetidine-3-yl)-7H-pyrrole[2,3-d]pyrimidine-5-yl)-1H-indole-1-formate, and the remaining required raw materials, reagents and preparation methods were the same as those of Step 11 in Example 1, to give 7-(azetidine-3-yl)-5-(1H-indol-2-yl)-7H-pyrrole[2,3-d]pyrimidine-4-amine hydrochloride.

Step 5: Preparation of 1-(3-(4-amino-5-(1H-indol-2-yl)-7H-pyrrole[2,3-d]pyrimidine-7-yl)azetidine-1-yl)prop-2-en-1-one

5-(7-methoxy-5-methylbenzothiophene-2-yl)-7-(pyrrolidine-3-yl)-7H-pyrrole[2,3-d]pyri midine-4 -amino hydrochloride was replaced with 7-(azetidine-3-yl)-5-(1H-indol-2-yl)-7H-pyrrole[2,3-d]pyrimidine-4-amine hydrochloride, and the remaining required raw materials, reagents and preparation methods were the same as those of Step 12 in Example 1, to obtain 1-(3-(4-amino-5-(1H-indol-2-yl)-7H-pyrrole[2,3-d]pyrimidine-7-yl)azetidine-1-yl)prop-2-en-1-one, yield 33%.
$^1$H NMR (300 MHz, DMSO-d6) δ 11.38 (s, 1H), 8.20 (s, 1H), 7.88 (s, 1H), 7.56 (d, J = 7.9 Hz, 1H), 7.39 (d, J = 7.8 Hz, 1H), 7.10 (t, J = 7.5 Hz, 1H), 7.02 (t, J = 7.3 Hz, 1H), 6.61 (s, 1H), 6.55 (s, 1H), 6.40 (dd, J = 17.0, 10.3 Hz, 1H), 6.17 (d, J = 16.8 Hz, 1H), 5.78 - 5.59 (m, 3H), 4.79 (t, J = 8.7 Hz, 1H), 4.65 (d, J = 6.1 Hz, 1H), 4.55 - 4.44 (m, 1H), 4.37 (d, J = 5.9 Hz, 1H).

**Example 31:**

**1-(3-(4-amino-5-(7-methoxy-5-methylbenzothiophene-2-yl)-7H-pyrrole[2,3-d]pyrimidine -7-yl)azetidine-1-yl)-2-(morpholinemethylene)prop-2-en-1-one**

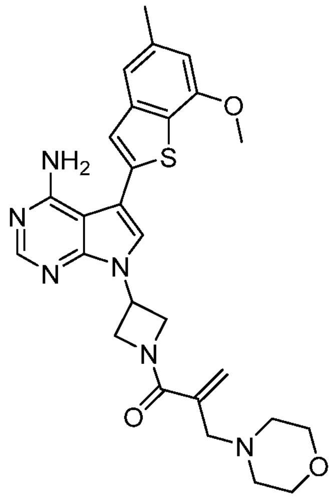

Step 1: Preparation of 2-(morpholinemethylene)acrylic acid

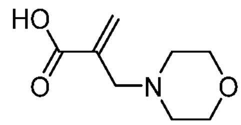

0.66 g of paraformaldehyde and 1 g of malonic acid were dissolved in a solution of 1,4 dioxane, then morpholine was added, heated to 70 °C for 2 hours, and the mixture was concentrated and isolated by column chromatography (dichloromethane :methanol = 98:2) to obtain 850 mg of white solids, yield 52%.
$^1H$ NMR (300 MHz, $CDCl_3$) $\delta$ 11.15 (s, 1H), 6.38 (s, 1H), 5.62 (s, 1H), 3.78 (s, 4H), 3.37 (s, 2H), 2.66 (s, 4H).

Step 2: Preparation of 1-(3-(4-amino-5-(7-methoxy-5-methylbenzothiophene-2-yl)-7H-pyrrole[2,3-d]pyrimidine-7-yl)azetidine-1-yl)-2-(morpholinemethylene)prop-2-en-1-one

2-fluoro-propynoic acid was replaced with 2-(morpholinemethylene)acrylic acid, and the remaining required raw materials, reagents and preparation methods were the same as those of Example 28, to obtain 1-(3-(4-amino-5)-(7-methoxy-5-methylbenzothiophene-2-yl)-7H-pyrrole[2,3-d]pyrimidine-7-yl)azetidine-1-yl)-2-(morpholinemethylene)prop-2-en-1-one, yield 45%.
$^1H$ NMR (300 MHz, DMSO-d6) $\delta$ 8.19 (s, 1H), 7.83 (s, 1H), 7.35 (s, 1H), 7.27 (s, 1H), 6.80 (s, 1H), 6.54 (s, 2H), 5.59 (d, J = 20.1 Hz, 3H), 4.66 (s, 2H), 4.50 - 4.32 (m, 2H), 3.95 (s, 3H), 3.55 (s, 4H), 3.11 (s, 2H), 2.43 (s, 3H), 2.37 (s, 4H).

**Example 32: (E)-1-(3-(4-amino-5-(7-methoxy-5-methylbenzothiophene-2-yl)-7H-pyrrole[2,3-d]pyrimidin e-7-yl)pyrroHdme-1-yl)-4-(4-ethylpiperazin-1-yl)but-2-en-1-one**

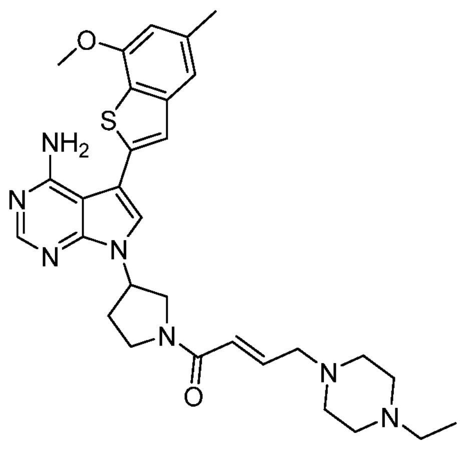

propiolic acid was replaced with trans -4-(4-ethylpiperazin-1-yl)crotonate hydrochloride, and the remaining required raw materials, reagents and preparation methods were the same as those of Example 3, to obtain (E)-1-(3-(4-amino-5-(7-methoxy-5-methylbenzothiophene-2-yl)-7H-pyrrole[2,3-d]pyrimidine -7-yl)pyrrolidine-1-yl)-4-(4-ethylpiperazin-1-yl)but-2-en-1-one, yield 44%.
$^1$H NMR (300 MHz, $CDCl_3$) δ 8.33 (s, 1H), 7.21 (d, *J* = 2.5 Hz, 2H), 7.10 (d, *J* = 13.3 Hz, 1H), 6.94 (dd, *J* = 15.0, 6.2 Hz, 1H), 6.63 (s, 1H), 6.30 (dd, *J* = 23.5, 15.2 Hz, 1H), 5.50 (d, *J* = 5.5 Hz, 3H), 3.99 (s, 3H), 4.32 - 3.58 (m, 4H), 3.19 (dd, *J* = 11.9, 6.1 Hz, 2H), 2.72 (s, 3H), 2.83 - 2.59 (m, 10H), 2.58 - 2.29 (m, 2H), 2.48 (s, 3H), 1.30-1.07 (m, 3H).

**Example 33: (E)-1-(3-(4-amino-5-(7-methoxy-5-methylbenzothiophene-2-yl)-7H-pyrrole[2,3-d]pyrimidin e-7-yl)pyrrolidine-1-yl)-4-(azetidine-1-yl)but-2-en-1-one**

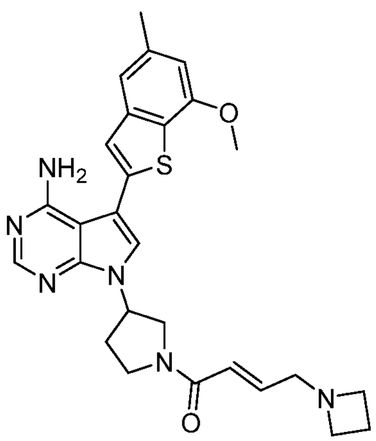

propynoic acid was replaced with trans -4-(azetidine-1-yl)crotonate hydrochloride, and the remaining required raw materials, reagents and preparation methods were the same as those of Example 3, to obtain (E)-1-(3-(4-amino-5-(7-methoxy-5-methylbenzothiophene-2-yl)-7H-pyrrole[2,3-d]pyrimidine -7-yl)pyrrolidine-1-yl)-4-(azetidine-1-yl)but-2-en-1-one, yield 47%.
$^1$H NMR (300 MHz, $CDCl_3$) δ 8.33 (s, 1H), 7.24 - 7.18 (m, 2H), 7.10 (d, *J* = 11.0 Hz, 1H), 6.94 - 6.79 (m, 1H), 6.64 (s, 1H), 6.37 (dd, *J* = 38.3, 15.0 Hz, 1H), 5.56 - 5.39 (m, 3H), 4.29 - 3.65 (m, 4H), 3.99 (s, 3H), 3.48 - 3.20 (m, 6H), 2.48 (s, 3H), 2.65 - 2.09 (m, 4H).

**Example 34: 1-(3-(4-amino-5-(7-methoxy-5-methylbenzothiophene-2-yl)-7H-pyrrole[2,3-d]pyrimidine -7-yl)pyrrolidine-1-yl)but-2-yn-1-one**

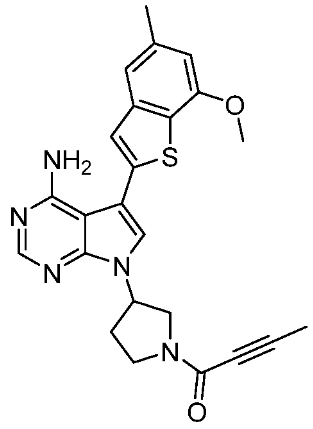

propynoic acid was replaced with 2-butynoic acid, and the remaining required raw materials, reagents and preparation methods were the same as those of Example 3 to obtain 1-(3-(4-amino-5-(7-methoxy-5-methylbenzothiophene-2-yl)-7H-pyrrole[2,3-d]pyrimidine-7-yl)pyrrolidine-1-yl)but-2-yn-1-one, yield 33%.
$^1$H NMR (300 MHz, $CDCl_3$) δ 8.32 (d, *J* = 3.8 Hz, 1H), 7.24 - 7.19 (m, 2H), 7.11 (d, *J* = 11.6 Hz, 1H), 6.64 (s, 1H), 5.62 (s, 2H), 5.55 - 5.40 (m, 1H), 4.32 - 3.61 (m, 4H), 4.00 (s, 3H), 2.49 (s, 3H), 2.68 - 2.22 (m, 2H), 2.00 (d, *J* = 15.2 Hz, 3H).

**Example 35: (S)-1-(3-(4-amino-5-(7-methoxy-5-methylbenzothiophene-2-yl)-7H-pyrrole[2,3-d]pyrimi dine-7-yl)pyrrolidine-1-yl)prop-2-yn-1-one**

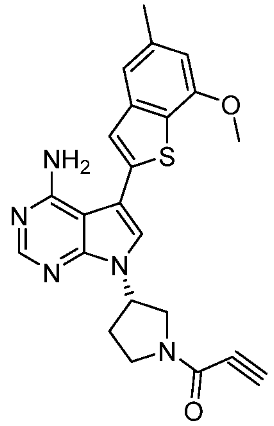

100 mg of (S)-5-(7-methoxy-5-methylbenzothiophene-2-yl)-7-(pyrrolidine-3-yl)-7H-pyrrole[2,3-d]pyri midine-4-amino hydrochloride, 20 μl of propiolic acid and 145 mg of 2-(7-azobenzotriazole)-N,N,N',N'-tetramethylurea hexafluorophosphate were dissolved in 2 ml of N,N-dimethylformamide, 0.2 ml of N,N-diisopropylethylamine was added dropwise, and the mixture was stirred at room temperature for 8 hours to quench the reaction. Water was added, and then the mixture was extracted with ethyl acetate. The organic phase was washed with a saturated aqueous solution of sodium chloride and dried over anhydrous sodium sulfate, filtered and concentrated, and the residue was isolated by column chromatography (dichloromethane: methanol = 97:3) to obtain 48 mg of yellow solids, yield 44%.
$^1$H NMR (300 MHz, $CDCl_3$) δ 8.33 (s, 1H), 7.22 (s, 2H), 7.11 (d, *J* = 9.3 Hz, 1H), 6.65 (s, 1H), 5.69 (s, 2H), 5.59 - 5.40 (m, 1H), 4.37 - 3.57 (m, 4H), 4.00 (s, 3H), 3.09 (d, *J* = 15.2 Hz, 1H), 2.64 - 2.24 (m, 2H), 2.49 (s, 3H).

**Example 36:**

**1-(3-(4-amino-5-(2-methyl-1H-benzimidazol-5-yl)-7H-pyrrole[2,3-d]pyrimidine-7-yl)pyrroli dine-1-yl)prop-2-en-1-one**

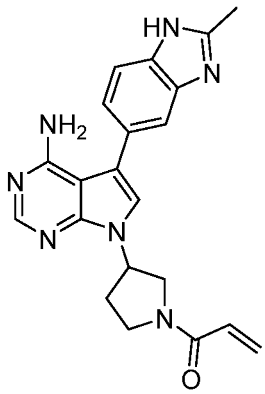

(7-methoxy-5-methylbenzothiophene-2-yl)boronic acid was replaced with tert-butyl 2-methyl-5-(4,4,5,5-tetramethyl-1,3,2-dioxyborane-2-yl)-1H-benzimidazole-1-formate (preparation method see patent WO2011055215), and the remaining required raw materials, reagents and preparation methods were the same as those of Example 1, yield 37%. $^1$H NMR (400 MHz, DMSO) δ 8.17 (s, 1H), 7.55 (d, *J* = 8.1 Hz, 1H), 7.50 (s, 1H), 7.39 (s, 0.5H), 7.34 (s, 0.5H), 7.23 (d, *J* = 8.1 Hz, 1H), 6.62 (m, 1H), 6.22 - 6.12 (m, 1H), 5.73 - 5.63 (m, 1H), 5.45 - 5.30 (m, 1H), 3.85 - 3.75 (m, 1H), 3.65 - 3.48 (m, 3H), 2.50 (s, 3H), 2.48 - 2.25 (m, 2H).

**Example 37 (S)-1-(3-(5-(7-methoxy-5-methylbenzothiophene-2-yl)-4-methylamino-7H-pyrrole[2,3-d] pyrimidine-7-yl)pyrrolidine-1-yl)prop-2-en-1-one**

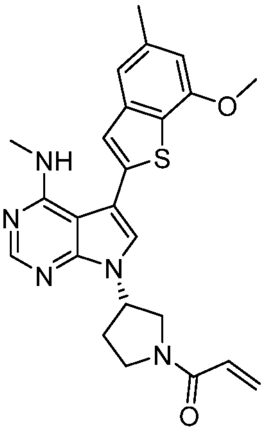

The preparation method was the same as that in Example 12, yield 55%.
$^1$H NMR (300 MHz, DMSO-d) δ 8.28 (s, 1H), 7.55 (d, J = 17.7 Hz, 1H), 7.28 (d, J = 6.9 Hz, 2H), 6.79 (s, 1H), 6.71 - 6.53 (m, 1H), 6.16 (d, J = 16.8 Hz, 2H), 5.71 (dd, J = 22.5, 9.6 Hz, 1H), 5.37 (d, J = 25.8 Hz, 1H), 4.21 - 3.99 (m, 1H), 3.94 (s, 3H), 3.73 (d, J = 6.9 Hz, 2H), 3.54 (dd, J = 15.4, 8.9 Hz, 1H), 2.95 (d, J = 3.7 Hz, 3H), 2.46 - 2.31 (m, 5H).

**Example 38**

**(S,E)-1-(3-(4-amino-5-(7-methoxy-5-methylbenzothiophene-2-yl)-7H-pyrrole[2,3-d]pyri midine-7-yl)pyrrolidine-1-yl)-4-dimethylamino-but-2-en-1-one**

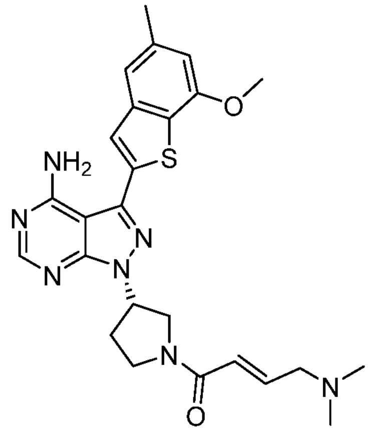

The preparation method was the same as that in Example 26, yield 47%.
$^1$H NMR (300 MHz, DMSO-d) δ 8.29 (s, 1H), 7.67 (s, 1H), 7.34 (s, 1H), 6.85 (s, 1H), 6.69 - 6.42 (m, 2H), 5.59 - 5.41 (m, 1H), 4.10 (s, 1H), 3.95 (s, 3H), 3.86 (d, J = 7.2 Hz, 3H), 3.23 (d, J = 12.7 Hz, 2H), 2.47 - 2.34 (m, 5H), 2.30 (s, 3H), 2.27 (s, 3H).

**Example 39 (S)-1-(3-(4-amino-3-(7-methoxy-5-methylbenzothiophene-2-yl)1H-pyrazolo[3,4-d]pyrimidi ne-1-yl)pyrrolidine-1-yl)prop-2-yn-1-one**

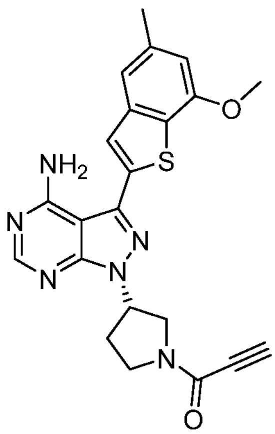

The preparation method was the same as that in Example 26, yield 39%.
$^1$H NMR (300 MHz, DMSO-d) δ 8.29 (s, 1H), 7.68 (s, 1H), 7.35 (s, 1H), 6.85 (s, 1H), 5.52 (s, 1H), 4.51 (d, J = 23.6 Hz, 1H), 4.10 (d, J = 16.8 Hz, 1H), 3.96 (s, 3H), 3.91 - 3.51 (m, 3H), 2.44 (s, 5H).

**Example 40 (S)-3-(7-methoxy-5-methylbenzothiophene-2-yl)-1-(1-vinylsulfonyl)pyrrolidine-3-yl)-1H-py razole[3,4-d]pyrimidine-4-amine)**

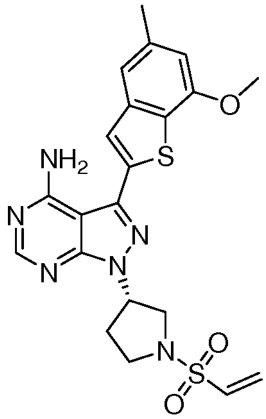

The preparation method was the same as that in Example 27, yield 46%.
$^1$H NMR (300 MHz, DMSO-d) δ 8.29 (s, 1H), 7.69 (s, 1H), 7.36 (s, 1H), 6.88 (dd, J = 17.9, 8.4 Hz, 2H), 6.10 (dd, J = 16.8, 13.4 Hz, 2H), 5.48 (dt, J = 10.7, 5.4 Hz, 1H), 3.97 (s, 3H), 3.79 (dd, J = 10.6, 7.1 Hz, 1H), 3.59 (dd, J = 15.8, 6.2 Hz, 2H), 3.46 (dd, J = 9.9, 6.8 Hz, 1H), 2.47 - 2.35 (m, 5H).

**Example 41 1-(3-(4-amino-5-(4,7-dimethoxybenzothiophene-2-yl)-7H-pyrrole[2,3-d]pyrimidine-7-yl)aze tidine-1-yl)prop-2-en-1-one**

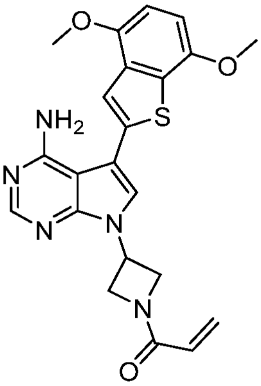

The preparation method was the same as that in Example 13, yield 60%.
$^1$H NMR (300 MHz, DMSO-d) δ 8.19 (s, 1H), 7.95 (s, 1H), 7.40 (s, 1H), 6.86 (s, 2H), 6.54 (s, 2H), 6.45 - 6.30 (m, 1H), 6.15 (d, J = 17.1 Hz, 1H), 5.71 (d, J = 10.2 Hz, 1H), 5.62 (s, 1H), 4.72 (d, J = 8.2 Hz, 2H), 4.42 (d, J = 9.2 Hz, 2H), 3.91 (s, 3H), 3.88 (s, 3H).

**Example 42**

**1-(3-(4-amino-5-(6-methoxybenzothiophene-2-yl)-7H-pyrrole[2,3-d-pyrimidine-7-yl)azetidi ne-1-yl)prop-2-en-1-one**

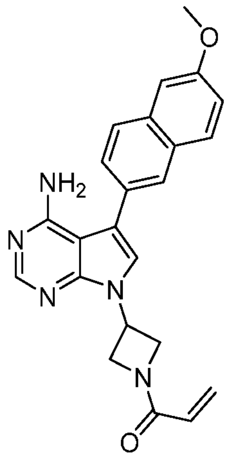

The preparation method was the same as that in Example 1, yield 45%.
$^1$H NMR (300 MHz, DMSO-d) δ 8.18 (s, 1H), 7.93 (d, J = 8.8 Hz, 2H), 7.87 (d, J = 9.1 Hz, 1H), 7.80 (s, 1H), 7.63 (d, J = 8.3 Hz, 1H), 7.38 (s, 1H), 7.21 (d, J = 8.9 Hz, 1H), 6.38 (dd, J = 17.1, 10.3 Hz, 1H), 6.16 (d, J = 17.1 Hz, 1H), 5.71 (d, J = 12.4 Hz, 1H), 5.62 (d, J = 8.7 Hz, 1H), 4.72 (m, J = 2H), 4.42 (m, 2H), 3.89 (s, 3H).

**Example 43 1-(3-(4-amino-5-(benzothiophene-2-yl)-7H-pyrrole[2,3-d]pyrimidine-7-yl)azetidine-1-yl)pro p-2-en-1-one**

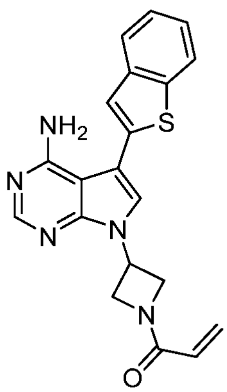

The preparation method was the same as that in Example 1, yield 43%.
$^1$H NMR (300 MHz, DMSO-d) δ 8.18 (s, 1H), 7.97 (d, J = 7.3 Hz, 1H), 7.90 (s, 1H), 7.85 (d, J = 7.0 Hz, 1H), 7.44 (s, 1H), 7.36 (td, J = 13.1, 6.5 Hz, 2H), 6.54 (s, 2H), 6.36 (dd, J = 17.0, 10.2 Hz, 1H), 6.19 - 6.08 (m, 1H), 5.74 (s, 1H), 5.72 - 5.67 (m, 1H), 5.61 (dt, J = 13.8, 6.8 Hz, 1H), 4.77 - 4.64 (m, 2H), 4.48 - 4.32 (m, 2H).

**Example 44 1-(3-(4-amino-5-(7-methoxy-5-methylbenzothiophene-2-yl)-7H-pyrrole[2,3-d]pyrimidine -7-yl)azetidine-1-yl)-2-(pyrrolidine-1-methylene)prop-2-en-1-one**

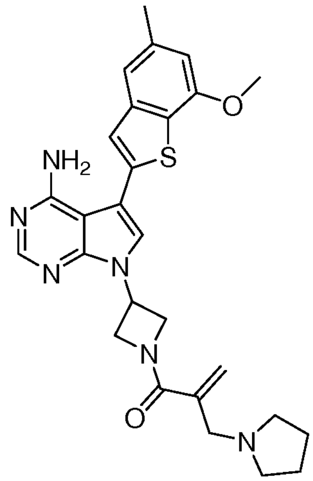

The preparation method was the same as that in Example 31, yield 45%.
$^1$H NMR (300 MHz, DMSO-d6) δ 8.19 (s, 1H), 7.93 (s, 1H), 7.35 (s, 1H), 7.28 (s, 1H), 6.81 (s, 1H), 6.52 (s, 2H), 5.59 (d, J = 20.1 Hz, 3H), 4.77 (s, 2H), 4.50 - 4.35 (m, 2H), 3.95 (s, 3H), 3.4-3.2 (6H), 2.44 (s, 3H), 1.89 (s, 4H).

**Example 45 (S) 1-(3-(4-amino-5-(7-methoxy-5-methylbenzothiophene-2-yl)-7H-pyrrole[2,3-d]pyrimidine-7-yl)pyrrolidine-1-yl)-2-(morpholinemethylene)prop-2-en-1-one**

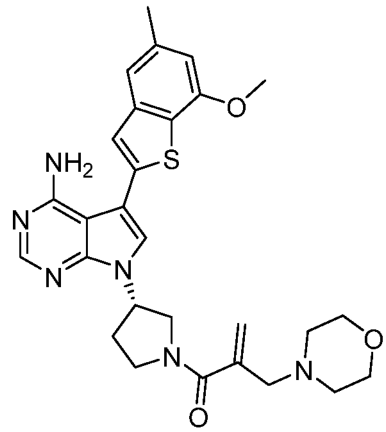

The preparation method was the same as that in Example 31, yield 47%.
$^1$H NMR (300 MHz, DMSO-d6) δ 8.19 (s, 1H), 7.50 (d, J = 9.4 Hz, 1H), 7.31 (d, J = 6.6 Hz, 1H), 7.27 (s, 1H), 6.80 (s, 1H), 6.48 (s, 2H), 5.46-5.35 (m, 2H), 5.29-5.26 (m, 1H), 4.05 - 3.62 (m, 4H), 3.94 (s, 3H), 3.40 - 3.20 (m, 6H), 2.43 (s, 3H), 2.37 (s, 4H), 2.24(s, 2H).

**Example 46 (S) 1-(3-(4-amino-5-(7-methoxy-5-methylbenzothiophene-2-yl)-7H-pyrrole[2,3-d]pyrimidine-7-yl)pyrrolidine-1-yl)-2-((diethylamino)methylene)prop-2-en-1-one**

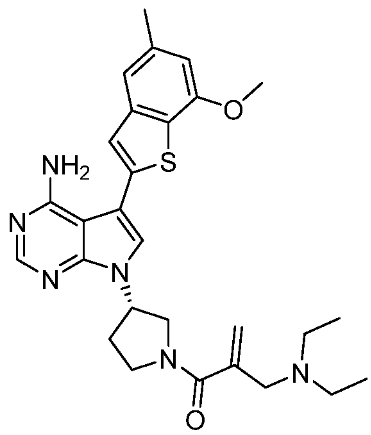

The preparation method was the same as that in Example 31, yield 49%.
$^1$H NMR (300 MHz, DMSO-d6) δ 8.19 (s, 1H), 7.50 (d, J = 9.4 Hz, 1H), 7.31 (d, J = 6.6 Hz, 1H), 7.27 (s, 1H), 6.80 (s, 1H), 6.48 (s, 2H), 5.46-5.35 (m, 2H), 5.29-5.26 (m, 1H), 4.05 - 3.62 (m, 4H), 3.94 (s, 3H), 3.40 - 3.20 (m, 6H), 2.43 (s, 3H), 2.40 (s, 2H), 1.02-0.68(m, 6H).

**Example 47**

**(S) 1-(3-(4-amino-5-(7-methoxy-5-methylbenzothiophene-2-yl)-7H-pyrrole[2,3-d]pyrimidine-7-yl)pyrrolidine-1-yl)-2-((dimethylamino)methylene)prop-2-en-1-one**

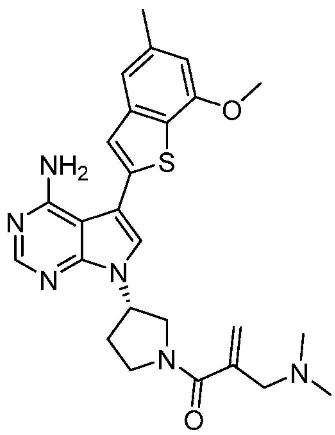

The preparation method was the same as that in Example 31, yield 43%.
$^1$H NMR (300 MHz, DMSO-d6) δ 8.19 (s, 1H), 7.53 (d, J = 9.4 Hz, 1H), 7.31 (d, J = 6.6 Hz, 1H), 7.27 (s, 1H), 6.80 (s, 1H), 6.48 (s, 2H), 5.52-5.41 (m, 2H), 5.38-5.29 (m, 1H), 4.05 - 3.62 (m, 4H), 3.94 (s, 3H), 3.40 - 3.20 (m, 6H), 2.43 (s, 3H), 2.40 (s, 2H), 2.22(s, 3H), 2.11 (s, 3H).

**Example 48**

**(S) 1-(3-(4-amino-5-(7-methoxy-5-methylbenzothiophene-2-yl)-7H-pyrrole[2,3-d]pyrimidine-7-yl)pyrrolidine-1-yl)-2-(piperidine-1-methylene)prop-2-en-1-one**

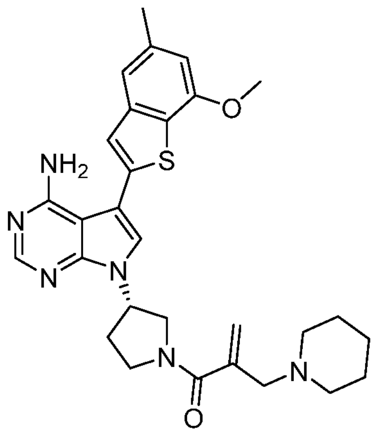

The preparation method was the same as that in Example 31, yield 44%.

$^1$H NMR (300 MHz, DMSO-d6) δ 8.19 (s, 1H), 7.48 (s, 1H), 7.31 (d, J = 6.6 Hz, 1H), 7.27 (s, 1H), 6.80 (s, 1H), 6.48 (s, 2H), 5.46-5.35 (m, 2H), 5.29-5.26 (m, 1H), 4.05 - 3.62 (m, 4H), 3.94 (s, 3H), 3.40 - 3.20 (m, 6H), 2.43 (s, 3H), 2.42(s, 2H), 1.42 - 1.18(m, 6H).

**Example 49**

**(S) 1-(3-(4-amino-5-(7-methoxy-5-methylbenzothiophene-2-yl)-7H-pyrrole[2,3-d]pyrimidine-7-yl)pyrrolidine-1-yl)-2-((4-methylpiperazin-1-yl)-methylene)prop-2-en-1-one**

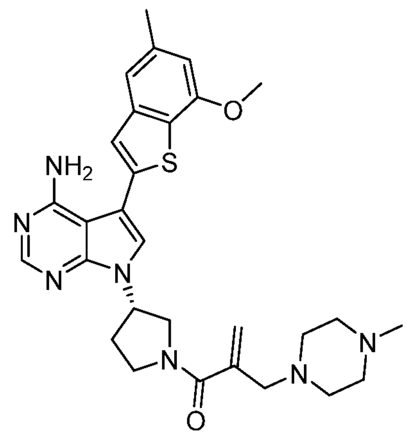

The preparation method was the same as that in Example 31, yield 49%.

$^1$H NMR (300 MHz, DMSO-d6) δ 8.19 (s, 1H), 7.48 (d, J = 9.4 Hz, 1H), 7.31 (d, J = 6.6 Hz, 1H), 7.27 (s, 1H), 6.80 (s, 1H), 6.48 (s, 2H), 5.46-5.35 (m, 2H), 5.29-5.26 (m, 1H), 4.05 - 3.62 (m, 4H), 3.94 (s, 3H), 3.40 - 3.20 (m, 2H), 2.43 (s, 3H), 2.50 (m, 8H), 2.24(s, 3H).

**Example 50**

**N-(2-(4-amino-3-(7-methoxy-5-methylbenzothiophene-2-yl)-1H-pyrazole[3,4-d]pyrimidine-1-yl)ethyl)acrylamide**

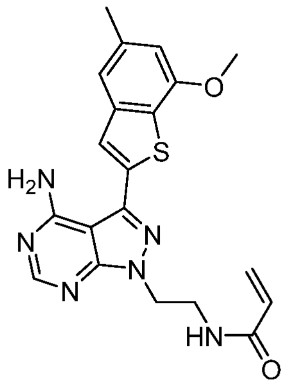

The preparation method was the same as that in Example 31, yield 46%.
$^1$H NMR (300 MHz, DMSO-d6) δ 8.37 (s, 1H), 7.53 (s, 1H), 7.27 (s, 1H), 6.91 (s, 1H), 6.69 (s, 1H), 6.26 (d, J = 17.2 Hz, 1H), 6.15 - 6.01 (m, 3H), 5.63 (d, J = 10.2 Hz, 1H), 4.69 - 4.59 (m, 2H), 4.01 (s, 3H), 3.88 (dd, J = 10.2, 5.3 Hz, 2H), 2.50 (s, 3H).

**Example 51**

**1-(3-(4-amino-5-(7-methoxy-5-methylbenzothiophene-2-yl)-7H-pyrrole[2,3-d]pyrimidine -7-yl)azetidine-1-yl)-2-((diethylamino)methylene)prop-2-en-1-one**

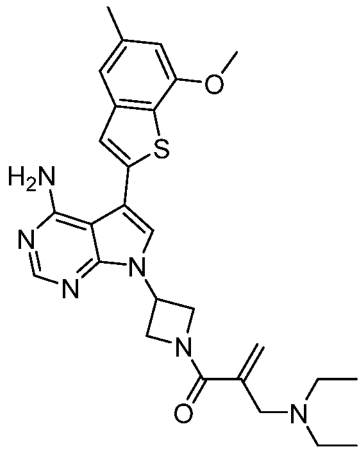

The preparation method was the same as that in Example 31.
$^1$H NMR (300 MHz, DMSO-d6) δ 8.18 (s, 1H), 7.83 (s, 1H), 7.34 (s, 1H), 7.28 (s, 1H), 6.80 (s, 1H), 6.51 (s, 2H), 5.65-5.45 (m, 3H), 4.67 (s, 2H), 4.45- 4.37 (m, 2H), 3.94 (s, 3H), 3.40 - 3.20 (m, 6H), 2.43 (s, 3H), 1.02-0.68(m, 6H).

**Example 52**

**1-(3-(4-amino-5-(7-methoxy-5-methylbenzothiophene-2-yl)-7H-pyrrole[2,3-d]pyrimidine -7-yl)azetidine-1-yl)-2-((4-methylpiperazin-1-yl)methylene)prop-2-en-1-one**

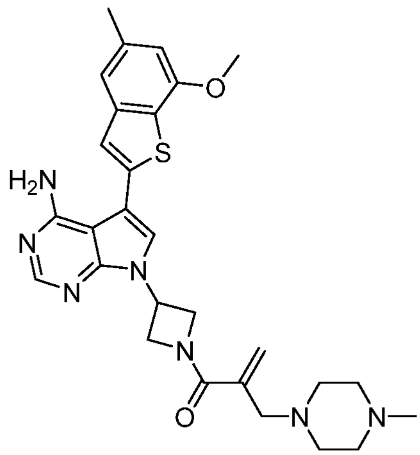

The preparation method was the same as that in Example 31.
$^1$H NMR (300 MHz, DMSO-d6) δ 8.19 (s, 1H), 7.78 (s, 1H), 7.34 (s, 1H), 7.28 (s, 1H), 6.80 (s, 1H), 6.51 (s, 2H), 5.60-5.53 (m, 3H), 4.70-4.62 (m, 2H), 4.48- 4.31 (m, 2H), 3.94 (s, 3H), 3.40 - 3.20 (m, 4H), 3.12 (s, 2H), 2.43 (s, 7H), 2.18 (s, 3H).

**Example 53**

**1-(3-(4-amino-5-(7-methoxy-5-methylbenzothiophene-2-yl)-7H-pyrrole[2,3-d]pyrimidine -7-yl)azetidine-1-yl)-2-((dimethylamino)methylene)prop-2-en-1-one**

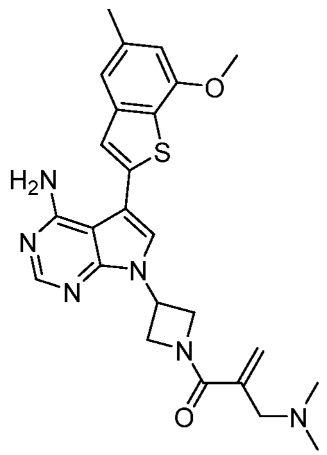

The preparation method was the same as that in Example 31, yield 47%.
$^1$H NMR (300 MHz, DMSO-d6) δ 8.18 (s, 1H), 7.85 (s, 1H), 7.35 (s, 1H), 7.28 (s, 1H), 6.81 (s, 1H), 6.51 (s, 2H), 5.75-5.68 (m, 2H), 5.60-5.52 (m, 1H), 4.72-4.71 (m, 2H), 4.50-4.42 (m, 1H), 4.38- 4.32 (m, 1H), 3.95 (s, 3H), 3.40 - 3.20 (m, 2H), 2.44 (s, 3H), 2.31(s, 6H).

**Example 54**

**(S) 1-(3-(4-amino-3-(7-methoxy-5-methylbenzothiophene-2-yl)-1H-pyrazole[3,4-d]pyrimidine-1 -yl)pyrrolidine-1-yl)-2-(morpholinemethylene)prop-2-en-1-one**

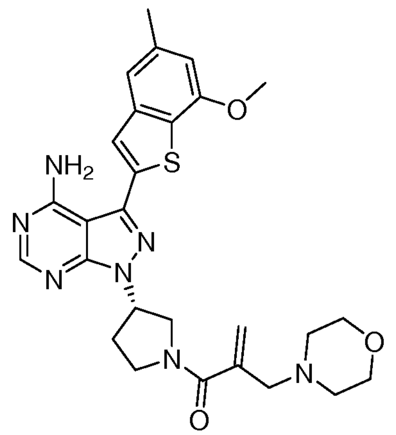

The preparation method was the same as that in Example 31, yield 46%.
$^1$H NMR (300 MHz, DMSO-d6) δ 8.29 (s, 1H), 7.67 (s, 1H), 7.34 (s, 1H), 7.25 (s, 2H), 6.85 (s, 1H), 5.57 - 5.30 (m, 3H), 4.11 (m, 1H), 3.96 (s, 3H), 3.81 (m, 3H), 3.40 - 3.20 (m, 6H), 2.44 (s, 3H), 2.43 - 2.34 (m, 2H), 2.37 (m, 4H).

**Example 55**

**1-(3-(4-amino-3-(7-methoxy-5-methylbenzothiophene-2-yl)-1H-pyrazole[3,4-d]pyrimidi ne-1-yl)azetidine-1-yl)-2-(morpholinemethylene)prop-2-en-1-one**

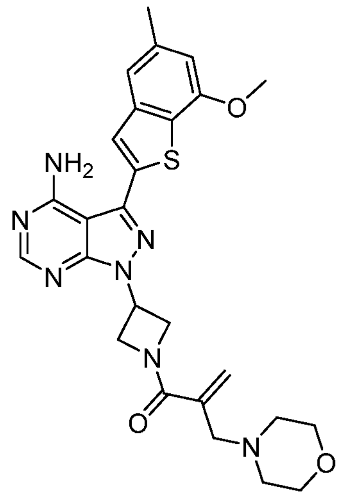

The preparation method was the same as that in Example 31, yield 45%.
$^1$H NMR (300 MHz, DMSO-d6) δ 8.29 (s, 1H), 7.71 (s, 1H), 7.36 (s, 1H), 7.25 (s, 2H), 6.86 (s, 1H), 5.75 - 5.65 (m, 1H), 5.75 (s, 2H), 4.75-4.67 (m, 2H), 4.48-4.38 (m, 2H), 3.96 (s, 3H), 3.59 (s, 3H), 3.40 - 3.20 (m, 2H), 2.45 (s, 3H), 2.37 (m, 4H).

**Example 56**

**(S) 1-(3-(4-amino-3-(7-methoxy-5-methylbenzothiophene-2-yl)-1H-pyrazole[3,4-d]pyrimidine-1 -yl)pyrrolidine-1-yl)prop-2-en-1-one**

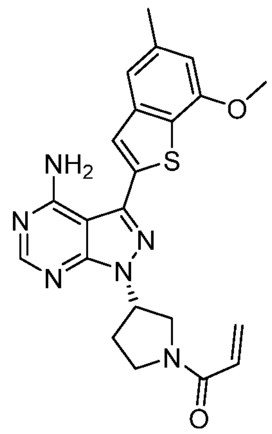

The preparation method was the same as that in Example 2, yield 55%.
$^1$H NMR (300 MHz, DMSO-d6) δ 8.29 (s, 1H), 7.67 (s, 1H), 7.34 (s, 1H), 7.25 (s, 2H), 6.85 (s, 1H), 6.75 - 6.48 (m, 2H), 6.16 (d, J = 16.9 Hz, 1H), 5.75 - 5.61 (m, 2H), 5.51 (d, J = 20.1 Hz, 1H), 4.11 (m, 1H), 3.94 (d, J = 9.9 Hz, 3H), 3.81 (m, 3H), 2.44 (s, 3H), 2.43 - 2.34 (m, 2H).

**Example 57**

**1-(3-(4-amino-5-(5,7-dimethoxybenzothiophene-2-yl)-7H-pyrrole[2,3-d]pyrimidine-7-yl )pyrrolidine-1-yl)prop-2-en-1-one**

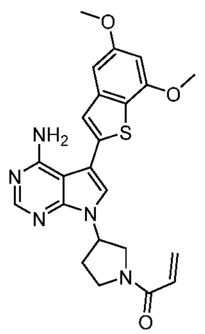

Step 1: the raw material 2-methoxy-4-methylthiophenol in the Step 4 of Example 1 was replaced with 2,4-dimethoxythiophenol, the remaining steps were the same as those in Example 1, and the title compound 1-(3-(4-amino-5-(5,7-dimethoxybenzothiophene-2-yl)-7H-pyrrole[2,3-d]pyrimidine-7-yl) pyrrolidine-1-yl)prop-2-en-1-one can be prepared.
$^1$H NMR (300 MHz, $CDCl_3$) δ8.33 (s, 1H), 7.21 (s, 1H), 7.11 (d, J = 10.2 Hz, 1H), 6.87 (s, 1H), 6.48 (s, 2H), 6.42 (d, J = 5.6 Hz, 1H), 5.74 (s, 1H), 5.60 (s, 2H), 5.49 (s, 1H), 4.05 (s, 2H), 3.97 (s, 3H), 3.88 (s, 3H), 3.81 (s, 2H), 2.45 (s, 3H).

**Example 58**

**1-(3-(4-amino-5-(7-methoxy-5-(trifluoromethyl)benzothiophene-2-yl)-7H-pyrrole[2,3-d ]pyrimidine-7-yl)pyrrolidine-1-yl)prop-2-en-1-one**

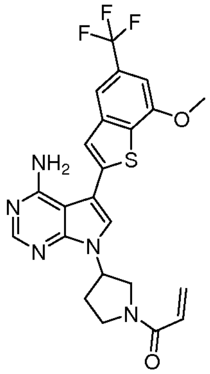

Step 1: Preparation of 2-iodo-5-(trifluoromethyl)phenol

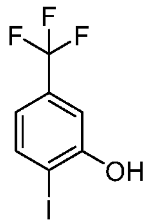

495 mg of sodium hydride (60%) was suspended in 40 ml of dried toluene and cooled in an ice bath. 3-trifluoromethylphenol was slowly added dropwise, and stirring for 10 minutes. 2.08 g of elemental iodine was further added, and stirred at room temperature for 16 hours. The mixture was diluted with 3N aqueous hydrochloric acid, extracted with ethyl acetate, washed with sodium thiosulfate solution, and the organic phases were combined and concentrated by column chromatography (petroleum ether: ethyl acetate = 98:2) to give 2.35 g of oily matter.
$^1$H NMR (300 MHz, $CDCl_3$) δ 5.68 (s, 1H), 6.93 (dd, J = 8.4, 2.1 Hz, 1H), 7.22 (d, J = 2.1 Hz, 1H),7.79 (d, J = 8.4 Hz, 1H).

Step 2: Preparation of 1-iodo-2-methoxy-4-(trifluoromethyl)benzene

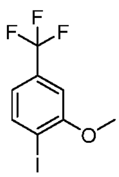

2.35 g of 2-iodo-5-(trifluoromethyl)phenol obtained in the Step 1 was dissolved in 25 ml of ethanol, and 3.9 g of potassium carbonate and 1.4 g of iodomethane were added, and refluxed under argon for 13 hours. The obtained was filtered through diatomite, and the filtrate was concentrated to dryness and isolated by column chromatography (petrole ether 100%) to obtain 2 g of oily matter.
$^1$H NMR (300 MHz, CDCl3) δ 3.92 (s, 3H), 6.94 (d, J = 8.1 Hz, 1H), 6.99 (s, 1H), 7.86 (d, J = 8.1Hz, 1H).

Step 3: Preparation of (2,2-diethoxy)(2-methoxy-4-(trifluoromethyl)benzene)sulfane

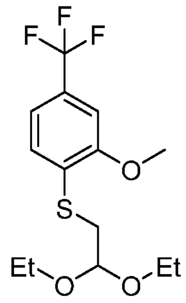

1-iodo-2-methoxy-4-(trifluoromethyl)benzene obtained in the Step 2 was used as a raw material, and (2,2-diethoxy)(2-methoxy-4-(trifluoromethyl)benzene)sulfane can be obtained by the method reported in Adv. Synth. Catal. 2015, 357, 2205-2212.

$^1$H NMR (300 MHz, $CDCl_3$) $\delta$ 7.37 (d, J = 8.1 Hz, 1H), 7.17 (d, J = 8.1 Hz, 1H), 7.00 (s, 1H), 4.68 (t, J = 5.5 Hz, 1H), 3.93 (s, 3H), 3.69 (tt, J = 14.1, 7.1 Hz, 2H), 3.62 - 3.46 (m, 2H), 3.14 (d, J = 5.5 Hz, 2H), 1.20 (t, J = 7.1 Hz, 6H).

Step 4: the raw material (2,2-diethoxyethyl)(2-methoxy-4-methylphenyl)thioether of Step 5 of Example 1 was replaced with (2,2-diethoxy)(2-methoxy-4-(trifluoromethyl)benzene)sulfane, and the title compound was obtained according to the procedure of Example 1.

$^1$H NMR (300 MHz, $CDCl_3$) $\delta$ 8.35 (s, 1H), 7.70 (s, 1H), 7.35 (s, 1H), 7.15 (d, J = 10.1 Hz, 1H), 6.96 (s, 1H), 6.49 (d, J = 8.6 Hz, 1H), 6.43 (d, J = 7.1 Hz, 1H), 5.80 - 5.68 (m, 1H), 5.53 (m, 3H), 4.24 - 4.09 (m, 1H), 4.07 (s, 3H), 3.85 (m, 3H), 2.59 (m, 1H), 2.44 (m, 1H).

**Example 59**

**(S)-1-(3-(4-amino-5-(5-chloro-7-methoxybenzothiophene-2-yl)-7H-pyrrole[2,3-d]pyrim idine-7-yl)pyrrolidine-1-yl)prop-2-en-1-one**

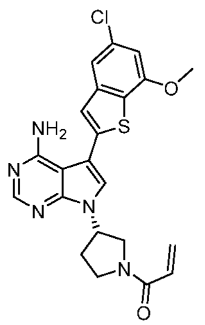

Step 1: Preparation of 4-chloro-2-methoxyaniline

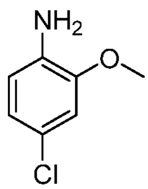

3 g of iron powder and 375 mg of ammonium chloride solids were heated to reflux in 50 ml of water for 15 minutes, then 2 g of 4-chloro-2-methoxynitrobenzene was added, refluxed for 1.5 hours, and cooled to room temperature. pH was adjusted to neutral with a saturated aqueous solution of sodium bicarbonate. The mixture was filtered through diatomite, and the filtrate was extracted with ethyl acetate and dried over anhydrous sodium sulfate to obtain 1.45 g of product, yield 86%.

Step 2: Preparation of 4-chloro-1-iodo-2-methoxybenzene

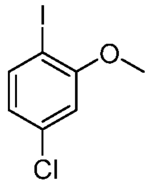

1.45g of 4-chloro-2-methoxyaniline was dissolved in a solution of 60 ml of water and 10 ml of concentrated sulfuric acid and cooled to below 5 °C in an ice bath. Further, 634 mg of sodium nitrite was dissolved in 1 ml of water and slowly added dropwise to the reaction solution, and the mixture was stirred under ice cooling for 45 minutes, and then 1.98 g of potassium iodide aqueous solution (5 ml) was added dropwise. After stirred at room temperature for 1 hour, the mixture was extracted with EtOAc, washed with a saturated aqueous solution of sodium thiosulfate, dried over anhydrous sodium sulfate and then isolated by column chromatography (PE 100%) to obtain 2.17 g of liquid, yield 88%.

Step 3: Preparation of (4-chloro-2-methoxybenzene) (2,2-diethoxyethyl)thioether

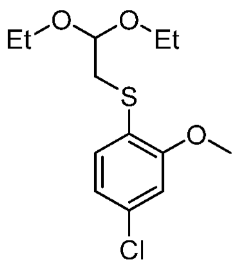

4-chloro-1-iodo-2-methoxybenzene obtained in the Step 2 was used as a raw material, and (4-chloro-2-methoxybenzene)(2,2-diethoxyethyl)thioether was obtained by the method reported in Adv. Synth. Catal. 2015, 357, 2205-2212.
$^1$H NMR (300 MHz, $CDCl_3$) δ 7.27 (d, J = 8.3 Hz, 1H), 6.89 (dd, J = 8.3, 2.1 Hz, 1H), 6.83 (d, J = 2.0 Hz, 1H), 4.62 (t, J = 5.6 Hz, 1H), 3.88 (s, 3H), 3.72 - 3.47 (m, 4H), 3.06 (d, J = 5.6 Hz, 2H), 1.20 (m, 6H).
Step 4: the raw material (2,2-diethoxyethyl)(2-methoxy-4-methylphenyl)thioether of Step 5 of Example 1 was replaced with (4-chloro-2-methoxybenzene)(2,2-diethoxyethyl)thioether, and the title compound was obtained according to the procedure of Example 1.
$^1$H NMR (300 MHz, $CDCl_3$) δ 8.34 (s, 1H), 7.40 (s, 1H), 7.22 (s, 1H), 7.12 (d, J = 10.4 Hz, 1H), 6.78 (s, 1H), 6.48 (d, J = 8.5 Hz, 1H), 6.42 (d, J = 5.0 Hz, 1H), 5.70-5.80 (m, 1H), 5.57 (s, 2H), 5.45-5.55 (m, 1H), 4.05-4.14 (m, 2H), 4.00 (s, 3H), 3.75-3.90 (m, 3H), 2.40-2.60 (m, 2H).
The preparation method for the following compounds was similar to that for compound 59.

| | | | |
|---|---|---|---|
| 60 | (S)-1-(3-(4-amino-5-(5-fluoro-7-methoxybenzothiophene-2-yl)-7H-pyrrole[2,3-d]pyrimidine-7-yl) pyrrolidine-1-yl)prop-2-en-1-one | 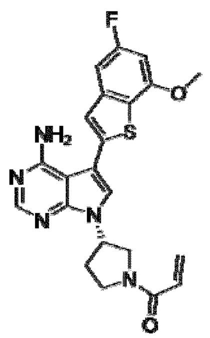 | $^1$H NMR (300 MHz, $CDCl_3$) δ 8.32 (s, 1H), 7.23 (s, 1H), 7.11 (t, J = 10.1 Hz, 2H), 6.60 (d, J = 10.9 Hz, 1H), 6.48 (d, J = 9.7 Hz, 1H), 6.45 - 6.38 (m, 1H), 5.81 - 5.69 (m, 3H), 5.55-5.45 (m, 1H), 4.23 -4.04 (m, 2H), 4.00 (s, 3H), 3.93 - 3.77 (m, 2H), 2.60-2.40 (m, 2H). |

(continued)

| | | | |
|---|---|---|---|
| 62 | (S)-1-(3-(4-amino-5-(5-bromo-7-methoxybenzothiophene-2-yl)-7H-pyrrole[2,3-d]pyrimidine-7-yl) pyrrolidine-1-yl)prop-2-en-1-one | 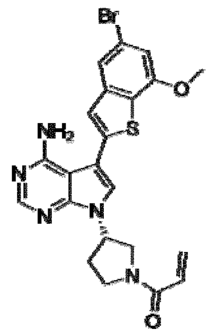 | $^1$H NMR (300 MHz, $CDCl_3$) δ 8.34 (s, 1H), 7.56 (s, 1H), 7.21 (s, 1H), 7.12 (d, J = 10.5 Hz, 1H), 6.90 (s, 1H), 6.48 (d, J = 8.6 Hz, 1H), 6.42 (d, J = 4.9 Hz, 1H), 5.70-5.80 (m, 1H), 5.57 (s, 2H), 5.52 - 5.44 (m, 1H), 4.25 - 4.04 (m, 2H), 4.00 (s, 3H), 3.75-3.90 (m, 2H), 2.40-2.60 (m, 2H). |
| 67 | (S)-1-(3-(4-amino-5-(4-chloro-7-methoxybenzothiophene-2-yl)-7H-pyrrole[2,3-d]pyrimidine-7-yl) pyrrolidine-1-yl)prop-2-en-1-one | 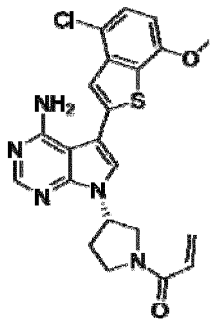 | $^1$H NMR (300 MHz, $CDCl_3$) δ 8.35 (s, 1H), 7.42 (d, J = 1.9 Hz, 1H), 7.33 (dd, J = 8.5, 1.6 Hz, 1H), 7.15 (d, J = 11.3 Hz, 1H), 6.73 (dd, J = 8.4, 2.0 Hz, 1H), 6.48 (dd, J = 6.1, 4.3 Hz, 1H), 6.45 - 6.39 (m, 1H), 5.75 (ddd, J = 12.1, 7.8, 4.4 Hz, 1H), 5.55 (s, 2H), 5.50 (s, 1H), 4.30 - 4.06 (m, 2H), 4.00 (s, 3H), 3.91 - 3.73 (m, 2H), 2.40-2.65 (m, 2H). |
| 74 | (S)-1-(3-(4-amino-5-(4-bromo-7-methoxybenzothiophene-2-yl)-7H-pyrrole[2,3-d]pyrimidine-7-yl) pyrrolidine-1-yl)prop-2-en-1-one | 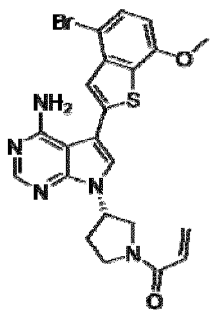 | $^1$H NMR (300 MHz, $CDCl_3$) δ 8.35 (s, 1H), 7.49 (dd, J = 8.1, 1.2 Hz, 1H), 7.40 (d, J = 1.7 Hz, 1H), 7.15 (d, J = 11.1 Hz, 1H), 6.69 (dd, J = 8.1, 1.8 Hz, 1H), 6.49 (d, J = 9.4 Hz, 1H), 6.43 (d, J = 6.8 Hz, 1H), 5.81 - 5.70 (m, 1H), 5.56 (s, 2H), 5.51 (s, 1H), 4.28 - 4.04 (m, 2H), 4.00 (s, 3H), 3.90 - 3.68 (m, 2H), 2.70 - 2.29 (m, 2H). |

**Example 61**

**1-(3-(4-amino-2-chloro-5-(7-methoxy-5-methylbenzothiophene-2-yl)-7H-pyrrole[2,3-d] pyrimidine-7-yl)azetidine-1-yl)prop-2-en-1-one**

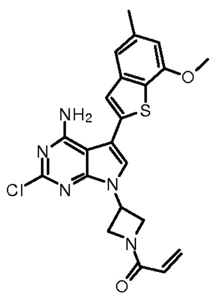

The raw material 4-chloro-7H-pyrrole[2,3-d]pyrimidine of Step 1 in Example 13 was replaced with 2,4-dichloro-7H-pyrrole[2,3-d]pyrimidine, and operation steps were the same as those in Example 13. 1-(3-(4-amino-2-chloro-5-(7-methoxy-5-methylbenzothiophene-2-yl)-7H-pyrrole[2,3-d]pyri midine-7-yl)azetidine-1-yl)prop-2-en-1-one can be prepared , yield 45%.

1H NMR (300 MHz, CDC13) δ 7.36 (s, 1H), 7.23 (s, 2H), 6.66 (s, 1H), 6.42 (d, J = 16.9 Hz, 1H), 6.27 (d, J = 10.3 Hz, 1H), 5.80 - 5.65 (m, 4H), 4.73 (m, 2H), 4.45 (s, 2H), 4.00 (s, 3H), 2.49 (s, 3H).

The preparation method for the following compounds was similar to that for compound 61.

| | | | |
|---|---|---|---|
| 64 | 1-(3-(4-amino-5-(7-methoxy-5-methylbenzothiophene-2-yl)-2-methyl-7H-pyrrole[2,3-d]pyrimidine-7-yl)pyrrolidine-1-yl)prop-2-en-1-one | 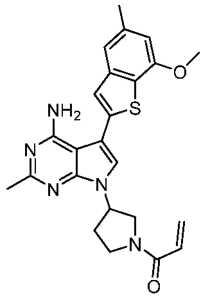 | $^1$H NMR (300 MHz, $CDCl_3$) δ 7.20 (d, J = 4.6 Hz, 2H), 7.05 (d, J = 9.7 Hz, 1H), 6.63 (s, 1H), 6.45 (m, 2H), 5.73 (m, 1H), 5.61 - 5.41 (m, 3H), 4.21 - 4.07 (m, 1H), 3.98 (s, 3H), 3.96 - 3.70 (m, 3H), 2.55 (s, 3H), 2.48 (s, 3H), 2.47 - 2.31 (m, 2H). |
| 65 | 1-(3-(2,4-diamino-5-(7-methoxy-5-methylbenzothiophene)-7H-pyrrole[2,3-d] pyrimidine-7-yl)pyrrolidine-1-yl)prop-2-en-1-one | 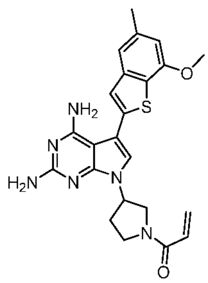 | $^1$H NMR (300 MHz, $CDCl_3$) δ 7.18 (d, J = 5.3 Hz, 2H), 6.82 (d, J = 9.5 Hz, 1H), 6.62 (s, 1H), 6.44 (dd, J = 17.4, 7.4 Hz, 2H), 5.73 (s, 1H), 5.39 (s, 2H), 5.29 (d, J = 5.5 Hz, 1H), 4.71 (s, 2H), 4.16 - 4.03 (m, 1H), 3.99 (s, 3H), 3.96 - 3.85 (m, 1H), 3.77 (s, 2H), 2.47 (s, 3H), 2.39 (s, 2H). |

### Example 63

**1-(3-(4-(dimethylamino)-5-(7-methoxy-5-methylbenzothiophene-2-yl)-7H-pyrrole[2,3-d ]pyrimidine-7-yl)pyrrolidine-1-yl)prop-2-en-1-one**

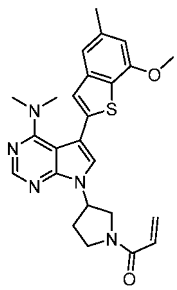

The raw material ammonia of Step 2 in Example 12 was replaced with an aqueous solution of dimethylamine, and the remaining steps were the same as those in Example 12. 1-(3-(4-(dimethylamino)-5-(7-methoxy-5-methylbenzothiophene-2-yl)-7H-pyrrole[2,3-d]pyri midine-7-yl)pyrrolidine-1-yl)prop-2-en-1-one was prepared. The yield was 59%. $^1$H NMR (300 MHz, $CDCl_3$) δ 8.42 (s, 1H), 7.22 - 7.08 (m, 3H), 6.62 (s, 1H), 6.45 (m, 2H), 5.73 (m, 1H), 5.53 (m, 1H), 4.25 - 4.03 (m, 2H), 4.00 (s, 3H), 3.79 (s, 2H), 2.94 (s, 6H), 2.54 (s, 1H), 2.48 (s, 3H), 2.44 - 2.36 (m, 1H).

### Example 66

**1-(3-(4-amino-5-(7-methoxy-5-methylbenzothiophene-2-yl)-2-(2-morpholine ethoxy)-7H-pyrrole[2,3-d]pyrimidine-7-yl)pyrrolidine-1-yl)prop-2-en-1-one**

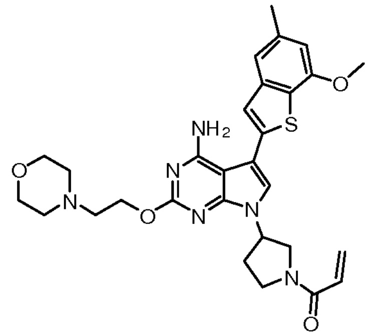

Step 1: Preparation of 6-amino-5-(2,2-diethoxy)-2-mercaptopyrimidine-4-ol

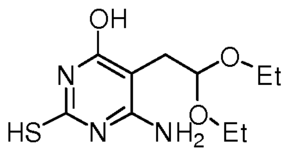

2.15 g of ethyl 2-cyano-4,4-diethoxybutyrate was dissolved in 5.3 ml of 20% sodium ethoxide solution in ethanol, and then 1.2 g of thiourea was added and heated to reflux overnight. The reaction solution was concentrated to dryness on the next day, diluted with 38 ml of water and washed with ethyl ether. The pH of the aqueous phase was adjusted to be neutrality with acetic acid, and a large amount of solidswere precipitated, filtered, and the solids were collected and dried to give 1.5 g of pale yellow solids, yield 62%.
$^1$H NMR (300 MHz, DMSO-d6) δ 11.75 (s, 1H), 11.44 (s, 1H), 6.07 (s, 2H), 4.50 (t, J = 5.6, 1H), 3.59 (dq, J = 7.0, 9.5, 2H), 3.40 (dq, J = 7.0, 9.6, 2H), 2.44 (d, J = 5.6, 2H), 1.07 (t, J = 7.0, 6H);

Step 2: Preparation of 2-thio-1,2,3,7-tetrahydro-4H-pyrrole[2,3-d]pyrimidine-4-one.

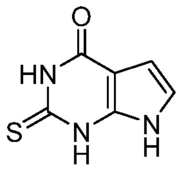

1 g of 6-amino-5-(2,2-diethoxy)-2-mercaptopyrimidine-4-ol obtained in the Step 2 was stirred in 12 ml of 0.2 N aqueous solution of HCl for 24 hours at room temperature, and a large amount of solids were precipitated, filtered and dried to obtain 2-thio-1,2,3,7-tetrahydro-4H-pyrrole[2,3-d]pyrimidine-4-one.
$^1$H NMR (300 MHz, DMSO-d6) δ 13.20 (s, 1H), 11.86 (s, 1H), 11.26 (s, 1H), 6.72 (s, 1H), 6.33 (d, J = 2.8 Hz, 1H).

Step 3: Preparation of 2-(methylthio)-3,7-dihydro-4H-pyrrole[2,3-d]pyrimidine-4-one.

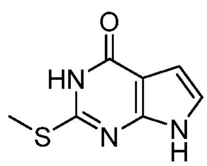

320 mg of sodium hydroxide was dissolved in 15 ml of ethanol, and 660 mg of 2-thio-1,2,3,7-tetrahydro-4H-pyrrole[2,3-d]pyrimidine-4-one obtained in Step 3 was dissolved in the solution. 0.25 ml of iodomethane was added dropwise under ice cooling. Upon addition, the reaction mixture was warmed to room temperature and stirred for 3 hours. The reaction solution was concentrated and dissolved in a small amount of water, and the pH was adjusted to be weakly acidic with 5N HCl, and a large amount of solids were precipitated. The solids were collected by filtration and dried to give 700 mg of white solids. The yield was 97%.
$^{1}$H NMR (300 MHz, DMSO-d6) δ 12.02 (s, 1H), 11.74 (s, 1H), 6.89 - 6.91 (m, 1H), 6.34 - 6.36 (m, 1H), 2.09 (s, 3H).

Step 4: Preparation of 4-chloro-2-(methylthio)-7H-pyrrole [2,3-d]pyrimidine.

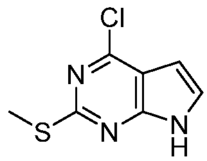

700 mg of 2-(methylthio)-3,7-dihydro-4H-pyrrole[2,3-d]pyrimidine-4-one obtained in Step 3 was heated to reflux overnight in 10 ml of phosphorus oxychloride. The reaction solution was concentrated on the next day, diluted with water, extracted with ethyl acetate, and the organic phase was washed with saturated sodium chloride and dried over anhydrous sodium sulfate, and then concentrated to dryness to give 4-chloro-2-(methylthio)-7H-pyrrole[2, 3-d]pyrimidine.
$^{1}$H NMR (300 MHz, DMSO-d6) δ 12.40 (s, 1H), 7.52 (d, J = 3.4 Hz, 1H), 6.51 (s, 1H), 2.55 (s, 3H).

Step 5: Preparation of 4-chloro-5-iodo-2-(methylthio)-7H-pyrrole [2,3-d]pyrimidine.

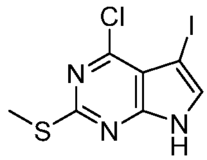

100 mg of above prepared 4-chloro-2-(methylthio)-7H-pyrrole[2,3-d]pyrimidine and 146 mg of N-iodosuccinimide were dissolved in 2 ml of N,N-dimethylformamide, and stirred at room temperature for 18 hours. The reaction mixture was diluted with water, and a large amount of solids were precipitated, dried and filtered to give 140 mg of purple solids, yield 91%.
$^{1}$H NMR (300 MHz, DMSO-d6) δ 12.72 (s, 1H), 7.75 (s, 1H), 2.55 (s, 3H).

Step 6: Preparation of tert-butyl 3-(4-chloro-5-iodo-2-(methylthio)-7H-pyrrole[2,3-d]pyrimidine-7-yl)pyrrolidine-1-formate.

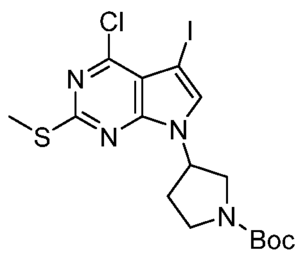

4-chloro-5-iodo-7H-pyrrole[2,3-d]pyrimidine inStep 8 of Example 1 was replaced with4-chloro-5-iodo-2-(methylthio) -7H-pyrrole [2,3-d]pyrimidine, and the remaining required raw materials and the procedures were the same as those of Step 8 of Example 1, to obtain tert-butyl 3-(4-chloro-5-iodo-2-(methylthio)-7H-pyrrole[2,3-d]pyrimidine-7-yl)pyrrolidine-1-formate.

$^1H$ NMR (300 MHz, $CDCl_3$) δ 7.20 (s, 1H), 5.37 (s, 1H), 3.88 (dd, J = 11.7, 6.9 Hz, 1H), 3.55 (s, 3H), 2.60 (s, 3H), 2.42 (td, J = 13.9, 7.2 Hz, 1H), 2.22 (td, J = 13.2, 6.7 Hz, 1H), 1.48 (s, 9H).

Step 7: Preparation of tert-butyl 3-(4-amino-5-iodo-2-(methylthio)-7H-pyrrole[2,3-d]pyrimidine-7-yl)pyrrolidine-1-formate.

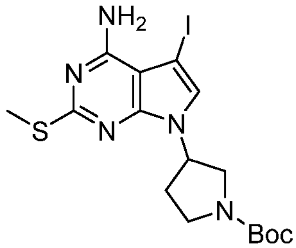

Preparation method was the same as that of Step 9 in Example 1.
$^1H$ NMR (300 MHz, $CDCl_3$) δ 6.91 (s, 1H), 5.55 (s, 2H), 5.31 (s, 1H), 3.91 - 3.77 (m, 1H), 3.53 (s, 3H), 2.55 (s, 3H), 2.37 (dd, J = 13.5, 6.5 Hz, 1H), 2.28 - 2.13 (m, 1H), 1.48 (s, 9H).

Step 8: Preparation of tert-butyl 3-(4-amino-5-iodo-2-(methylsulfonyl)-7H-pyrrole[2,3-d]pyrimidine-7-yl)pyrrolidine-1-forma te.

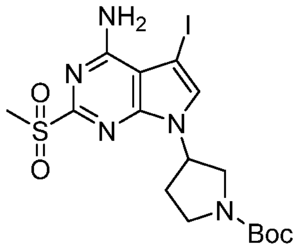

1.2 g of above prepared tert-butyl 3-(4-amino-5-iodo-2-(methylthio)-7H-pyrrole[2,3-d]pyrimidine-7-yl)pyrrolidine-1-formate was dissolved in ethanol, and 2.5 equivalents of m-chloroperoxybenzoic acid was added in batches, and stirred at room temperature overnight. The next day, the obtained was concentrated to dryness by column chromatography (dichloromethane: methanol = 98:2) to afford 1.28 g of tert-butyl 3-(4-amino-5-iodo-2-(methylsulfonyl)-7H-pyrrole[2,3-d]pyrimidine-7-yl)pyrrolidine-1-forma te.
$^1H$ NMR (300 MHz, $CDCl_3$) δ 7.21 (s, 1H), 6.20 (s, 2H), 5.46 (s, 1H), 3.87 (s, 1H), 3.70-3.50 (m, 3H), 3.30 (s, 3H), 2.42 (s, 1H), 2.19 (s, 1H), 1.49 (s, 9H).

Step 9: Preparation of tert-butyl 3-(4-amino-5-iodo-2-(2-morpholineethoxy)-7H-pyrrole[2,3-d]pyrimidine-7-yl)pyrrolidine-1 -formate.

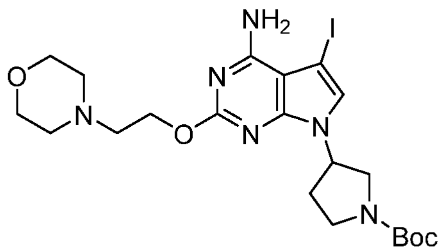

413 mg of 2-morpholineethanol and 384 mg of potassium t-butoxide were dissolved in tetrahydrofuran, stirred at room temperature for 10 minutes, then 800 mg of tert-butyl 3-(4-amino-5-iodo-2-(methylsulfonyl)-7H-pyrrole[2,3-d]

pyrimidine-7-yl)pyrrolidine-1-forma te was added, sealed at 80 ° C overnight. The reaction mixture was concentrated to dryness on the next day and isolated by column chromatography (dichloromethane: methanol = 98: 2+ 0.1% ammonia) to afford 780 mg of solids, yield 89%.

$^1$H NMR (300 MHz, $CDCl_3$) δ 6.87 (s, 1H), 5.56 (s, 2H), 5.24 (s, 1H), 4.45 (t, J = 6.0 Hz, 2H), 3.80 (s, 1H), 3.75 - 3.69 (m, 4H), 3.65-3.45 (m, 3H), 2.80 (t, J = 6.0 Hz, 2H), 2.64 - 2.50 (m, 4H), 2.41 - 2.25 (m, 1H), 2.18 (dd, J = 13.5, 6.6 Hz, 1H), 1.48 (s, 9H).

Step 10: tert-butyl 3-(4-amino-5-iodo-7H-pyrrole[2,3-d]pyrimidine-7-yl)pyrrolidine-1-formate of Step 10 in Example 1 was replaced with tert-butyl 3-(4-amino-5-iodo-2-(2-morpholineethoxy)-7H-pyrrole[2,3-d]pyrimidine-7-yl)pyrrolidine-1-formate, the remaining steps were similar to those in Example 1, and the title compound was prepared.

$^1$H NMR (300 MHz, $CDCl_3$) δ 7.19 (d, J = 4.4 Hz, 2H), 6.93 (d, J = 9.2 Hz, 1H), 6.62 (s, 1H), 6.51 - 6.37 (m, 2H), 5.78 - 5.65 (m, 1H), 5.44 (s, 2H), 5.41 - 5.26 (m, 1H), 4.48 (t, J = 5.8 Hz, 2H), 4.17 - 4.03 (m, 1H), 3.98 (s, 3H), 3.79 (d, J = 8.6 Hz, 3H), 3.72 (s, 4H), 2.82 (t, J = 5.8 Hz, 2H), 2.59 (s, 4H), 2.47 (s, 3H), 2.44 - 2.31 (m, 2H).

**Example 68**

**1-(3-(4-amino-5-(7-methylbenzothiophene-2-yl)-7H-pyrrole[2,3-d]pyrimidine-7-yl)pyrr olidine-1-yl)prop-2-en-1-one**

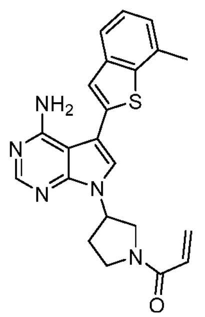

The raw material 2-methoxythiophenol of step 1 in Example 22 was replaced with 2-methylthiophenol, and the remaining steps were similar to those in Example 22. 1-(3-(4-amino-5-(7-methylbenzothiophene-2-yl)-7H-pyrrole[2,3-d] pyrimidine-7-yl)pyrrolidi ne-1-yl) prop-2-en-1-one was prepared, yield 56%.

$^1$H NMR (300 MHz, $CDCl_3$) δ 8.34 (s, 1H), 7.65 (d, J = 8.0 Hz, 1H), 7.33 (m, 2H), 7.20 - 7.09 (m, 2H), 6.53 - 6.45 (m, 1H), 6.45 - 6.39 (m, 1H), 5.78 - 5.68 (m, 1H), 5.58 (s, 2H), 5.51 (dd, J = 12.1, 5.9 Hz, 1H), 4.26 - 4.04 (m, 2H), 3.88 - 3.76 (m, 2H), 2.58 (s, 3H), 2.55 - 2.35 (m, 2H).

**Example 69**

**(E)-1-(3-(4-amino-5-(7-methoxy-5-methylbenzothiophene-2-yl)-7H-pyrrole[2,3-d]pyri midine-7-yl)pyrrolidine-1-yl)-4-(3-fluoropyrrolidine-1-yl)but-2-en-1-one**

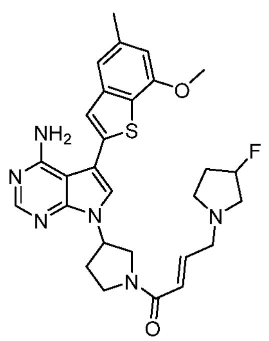

trans-4-(pyrrolidine-1-yl) crotonate hydrochloride in Example 7 was replaced with trans-4-(3-fluoropyrrolidine-1-yl)but-2-enoate hydrochloride, and the remaining steps were similar to those in Example 7. (E)-1-(3-(4-amino-5-(7-methoxy-5-methylbenzothiophene-2-yl)-7H-pyrrole[2,3-d]pyrimidine -7-yl)pyrrolidine-1-yl)-4-(3-fluoropyrrolidine-1-yl)but-2-en-1-one was prepared.

The preparation method for the following compounds was similar to that for compound 69.

| | | | |
|---|---|---|---|
| 77 | (E)-1-(3-(4-amino-5-(7-methoxy-5-methylbenzothiophene-2-yl)-7H-pyrrole[2,3-d]pyrimidine-7-yl) pyrrolidine-1-yl)-4-(4-hydroxypiperidine-1-yl)but-2-en-1-one | 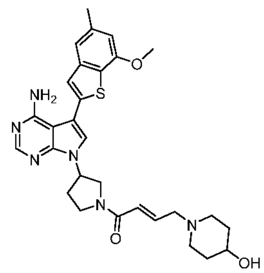 | $[M+H]^+$: 547 |
| 81 | (E)-1-(3-(4-amino-5-(7-methoxy-5-methylbenzothiophene-2-yl)-7H-pyrrole[2,3-d]pyrimidine-7-yl) pyrrolidine-1-yl)-4-((2-methoxyethyl) (methyl)amino)but-2-en-1-one | 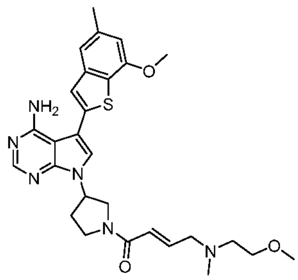 | $^1$H NMR (300 MHz, DMSO-d6) δ 8.19 (s, 1H), 7.58 (d, J = 19.6 Hz, 1H), 7.32 (s, 1H), 7.27 (s, 1H), 6.79 (s, 1H), 6.65 (dd, J = 14.9, 6.5 Hz, 1H), 6.57 - 6.35 (m, 3H), 5.35 (d, J = 26.1 Hz, 1H), 4.08 (d, J = 7.4 Hz, 1H), 3.92 (d, J = 10.9 Hz, 3H), 3.87 (s, 1H), 3.83 - 3.64 (m, 2H), 3.18 (dd, J = 18.5, 9.5 Hz, 6H), 2.43 (s, 5H), 2.19 (d, J = 9.5 Hz, 3H). |

**Example 70**

**1-(4-(4-amino-5-(7-methoxy-5-methylbenzothiophene-2-yl)-7H-pyrrole[2,3-d]pyrimidin e-7-yl)-2-(hydroxymethyl)pyrrolidine-1-yl)prop-2-en-1-one**

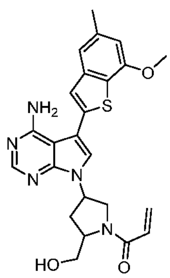

Step 1: Preparation of tert-butyl 2-(((tert-butyldiphenylsilyl)oxy)methyl)-4-hydroxypyrrolidine-1-formate

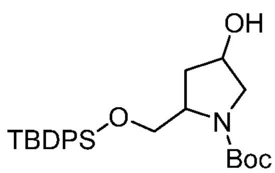

500 mg of tert-butyl 4-hydroxy-2-(hydroxymethyl)pyrrolidine-1-formate and 164 mg of imidazole were dissolved in N,N-dimethylformamide. 0.62 ml of t-butyldiphenylchlorosilane was added dropwise under ice cooling. After stirred at room temperature for 1 hour, the reaction was quenched with water, extracted with ethyl acetate and isolated by

column chromatography (petroleum ether: ethyl acetate = 85: 15) to afford 900 mg of oily matter, yield 86%. $[M+H]^+$: 456

Step 2: Preparation of tert-butyl 4-(4-amino-5-(7-methoxy-5-methylbenzothiophene-2-yl)-7H-pyrrole[2,3-d]pyrimidin-7-yl)-2 -(((tert-butyldiphenylsilyl)oxy)methylene)pyrrolidine-1-formate.

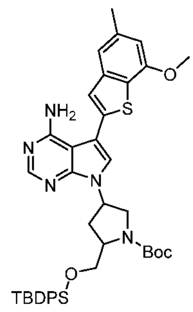

tert-butyl 3-hydroxypyrrolidine-1-formate of Step 8 in Example 1 was replaced with tert-butyl 2-((((tert-butyldiphenylsilyl)oxy)methyl)-4-hydroxypyrrolidine-1-formate, and the remaining steps were similar tothose of Steps 8 to 10 of Example 1. $[M+H]^+$: 748

Step 3: Preparation of tert-butyl 4-(4-amino-5-(7-methoxy-5-methylbenzothiophene-2-yl)-7H-pyrrole[2,3-d]pyrimidin-7-yl)-2 -(hydroxymethyl)pyrrolidine-1-formate.

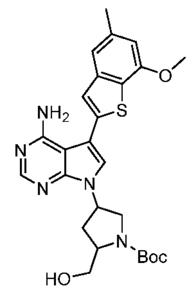

300 mg of tert-butyl 4-(4-amino-5-(7-methoxy-5-methylbenzothiophene-2-yl)-7H-pyrrole[2,3-d]pyrimidin-7-yl)-2 -(((tert-butyldiphenylsilyl)oxy)methylene)pyrrolidine-1-formate was dissolved in 3 ml of tetrahydrofuran, and then 2.5 equivalents of tetrabutylammonium fluoride was added thereto, stirred at room temperature overnight. The next day, the obtained was concentrated to dryness by column chromatography (dichloromethane: methanol = 98:2) to give 170 mg of solids, yield 83%. $[M+H]^+$: 510

Step 4: tert-butyl 4-(4-amino-5-(7-methoxy-5-methylbenzothiophen-2-yl)-7H-pyrrole[2,3-d]pyrimidine-7-yl)-2 -(hydroxymethyl)pyrrolidine-1-formate obtained in Step 3 was used as a raw material, and the title compound can be obtained in the same manner as those in Step 11 to Step 12 of Example 1.

$^1$H NMR (300 MHz, $CDCl_3$) $\delta$ 8.30 (s, 1H), 7.20 (m, 3H), 6.64 (s, 1H), 6.43 (m, 2H), 5.80 - 5.73 (m, 1H), 5.62 (s, 2H), 5.26 (m, 1H), 4.50 - 4.29 (m, 2H), 3.99 (s, 3H), 3.94 (m, 2H), 3.88 - 3.76 (m, 2H), 2.72-2.65 (m, 1H), 2.48 (s, 3H), 2.40-2.25 (m, 1H).

Example 71

1-(3-(4-amino-6-chloro-5-(7-methoxy-5-methylbenzothiophene-2-yl)-7H-pyrrole[2,3-d] pyrimidine-7-yl)pyrimidine-1-yl)prop-2-en-1-one

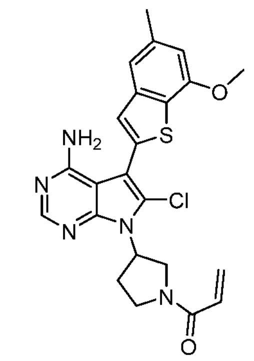

Step 1: Preparation of tert-butyl 3-(4-chloro-5-(7-methoxy-5-methylbenzothiophene-2-yl)-7H-pyrrole[2,3-d]pyrimidin-7-yl) pyrrolidine-1-formate

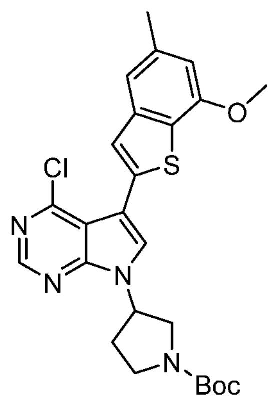

tert-butyl 3-(4-amino-5-iodo-7H-pyrrole[2,3-d]pyrimidine-7-yl)pyrrolidine-1-formate of Step 10 in Example 1 was replaced with tert-butyl 3-(4-chloro-5-iodo-7H-pyrrole[2,3-d]pyrimidine-7-yl)pyrrolidine-1-formate, other raw materials were the same, and tert-butyl 3-(4-chloro-5-(7-methoxy-5-methylbenzothiophene-2-yl)-7H-pyrrole[2,3-d]pyrimidine-7-yl) pyrrolidine-1-formate can be prepared. [M+H]=499

Step 2: Preparation of tert-butyl 3-(4,6-dichloro-5-(7-methoxy-5-methylbenzothiophene-2-yl)-7H-pyrrole[2,3-d]pyrimidine-7-yl)pyrrolidine-1-formate

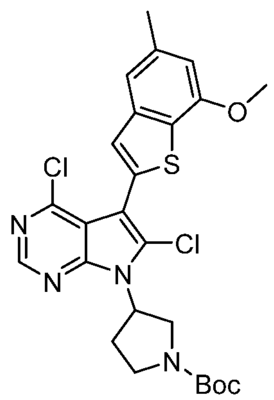

50 mg of tert-butyl 3-(4-chloro-5-(7-methoxy-5-methylbenzothiophene-2-yl)-7H-pyrrole[2,3-d]pyrimidine-7-yl) pyrrolidine-1-formate obtained in Step 1 and 3 mg of triphenylphosphine sulfide were dissolved in 1 ml of dichloromethane,

and stirred at room temperature for 5 minutes, then 16 mg of N-chlorosuccinimide was added, and the mixture was stirred at room temperature for 1 hour. The reaction mixture was concentrated to dryness, and isolated by column chromatography (PE: EA) = 55:45 to obtain tert-butyl 3-(4,6-dichloro-5-(7-methoxy-5-methylbenzothiophene-2-yl)-7H-pyrrole[2,3-d]pyrimidin-7-yl)pyrrolidine-1-formate. [M+H]=533

Step 3: Preparation of tert-butyl 3-(4-amino-6-chloro-5-(7-methoxy-5-methylbenzothiophene-2-yl)-7H-pyrrole[2,3-d]pyrimidi ne-7-yl)pyrrolidine-1-formate

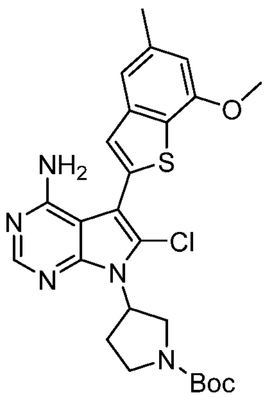

40 mg of tert-butyl 3-(4,6-dichloro-5-(7-methoxy-5-methylbenzothiophene-2-yl)-7H-pyrrole[2,3-d]pyrimidine-7-yl)pyrrolidine-1-formate obtained in the Step 2 was heated in a mixed solution of 2 ml of ammonia and 2 ml of dioxane at 110 °C for 48 hours. The reaction mixture was concentrated to dryness, and isolated by column chromatography (dichloromethane:methanol = 98:2) to afford tert-butyl 3-(4-amino-6-chloro-5-(7-methoxy-5-methylbenzothiophene-2-yl)-7H-pyrrole[2,3-d]pyrimidi ne-7-yl)pyrrolidine-1-formate in 78% yield. [M+H]=514

Step 4: According to Example 1, the raw material tert-butyl 3-(4-amino-5-(7-methoxy-5-methylbenzothiophene-2-yl)-7H-pyrrole[2,3-d]pyrimidine-7-yl)p yrrolidine-1-formate of Step 11 in Example 1 was replaced with tert-butyl 3-(4-amino-6-chloro-5-(7-methoxy-5-methylbenzothiophene-2-yl)-7H-pyrrole[2,3-d]pyrimidi ne-7-yl)pyrrolidine-1-formate to prepare the title compound.

$^1$H NMR (300 MHz, $CDCl_3$) δ 8.34 (s, 1H), 7.41 (d, J = 2.0 Hz, 1H), 7.14 (d, J = 11.4 Hz, 1H), 6.68 (s, 1H), 6.51 - 6.39 (m, 2H), 5.80 - 5.69 (m, 1H), 5.57 (s, 2H), 5.54 - 5.43 (m, 1H), 4.24 - 4.03 (m, 2H), 3.98 (s, 3H), 3.91 - 3.75 (m, 2H), 2.60 - 2.52 (m, 1H), 2.51 (s, 3H), 2.45 (s, 1H).

**Example 72**

**N-(2-(4-amino-5-(7-methoxy-5-methylbenzothiophene-2-yl)-7H-pyrrole[2,3-d]pyrimidi ne-7-yl)ethyl)acrylamide**

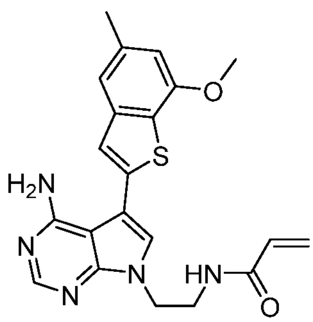

Step 1: Preparation of tert-butyl (2-(4-chloro-5-iodo-7H-pyrrole[2,3-d]pyrimidine-7-yl)ethyl)amino formate

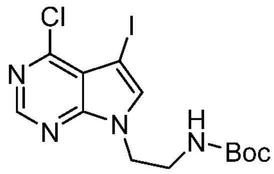

100 mg of 4-chloro-5-iodo-7H-pyrrole[2,3-d]pyrimidine obtained in Step 7 of Example 1, 108 mg of N-Boc-bromoethylamine and 200 mg of cesium carbonate were heated in 2 ml of N,N-dimethylformamide at 80 °C for 6 hours. The mixture was diluted with water and extracted with EtOAc. The organic phase was dried over anhydrous sodium sulfate. The reaction mixture was concentrated to dryness and isolated by column chromatography (PE: EA = 85: 15) to give 145 mg of tert-butyl (2-(4-chloro-5-iodo-7H-pyrrole[2,3-d]pyrimidine-7-yl)ethyl)amino formate, yield 85%.
$^1$H NMR (300 MHz, $CDCl_3$) δ 8.60 (s, 1H), 7.38 (s, 1H), 4.75 (s, 1H), 4.40 (s, 2H), 3.52 (dd, J = 12.9, 7.0 Hz, 2H), 1.40 (s, 9H).

Step 2: Preparation of title compound

According to Example 1, the intermediate obtained in Step 1 was used as the raw material for replacing tert-butyl 3-(4-chloro-5-iodo-7H-pyrrole[2,3-d]pyrimidine-7-yl)pyrrolidine-1-formate in Step 9 of Example 1, so as to prepare the title compound.
$^1$H NMR (300 MHz, $CDCl_3$) δ 8.31 (s, 1H), 7.20 (d, J = 3.8 Hz, 2H), 7.11 (s, 2H), 6.64 (s, 1H), 6.25 (d, J = 17.3 Hz, 1H), 6.14 - 6.01 (m, 1H), 5.63 (d, J = 16.7 Hz, 3H), 4.47 - 4.36 (m, 2H), 4.00 (s, 3H), 3.77 (dd, J = 10.4, 4.9 Hz, 2H), 2.48 (s, 3H).

**Example 73**

**N-(4-(4-amino-5-(7-methoxy-5-methylbenzothiophene-2-yl)-7H-pyrrole[2,3-d]pyrimidi ne-7-yl)cyclohexyl)acrylamide**

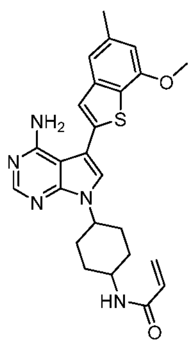

tert-butyl 3-hydroxypyrrolidine-1-formate of Step 8 in Example 1 was replaced with tert-butyl (4-hydroxycyclohexyl)amino formate, and the title compound can be obtained in the same manner as that in Example 1.
$^1$H NMR (300 MHz, $CDCl_3$) δ 8.31 (s, 1H), 7.20 (m, 3H), 6.64 (s, 1H), 6.35 (d, J = 16.6 Hz, 1H), 6.24 (d, J = 10.1 Hz, 1H), 6.18 (d, J = 9.9 Hz, 1H), 5.69 (d, J = 10.1 Hz, 1H), 5.55 (s, 2H), 4.62 (s, 1H), 4.34 (s, 1H), 3.99 (s, 3H), 2.48 (s, 3H), 2.06 (s, 4H), 1.85 (s, 4H).

**Example 75**

**(4S)-1-acrylamide-4-(4-amino-3-(7-methoxy-5-methylbenzothiophene-2-yl)-1H-pyrazol e[3,4-d]pyrimidine-1-yl)-N-(2-(dimethylamine)ethyl-N-methylpyrrolidine-2-formamide**

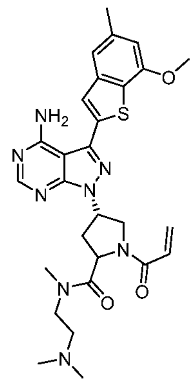

Step 1: Preparation of 1-(tert-butyl 2-methyl(4S)-4-(4-amino-3-(7-methoxy-5-methylbenzothiophene-2-yl)-)-1H-pyrazole[3,4-d] pyrimidine-1-yl)pyrrolidine-1,2-diformate

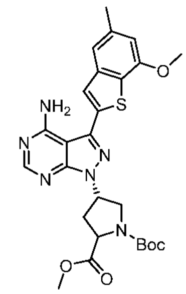

tert-butyl 3-hydroxypyrrolidine-1-formate in Step 3 of Example 2 was replaced with methyl N-Boc-trans-4-hydroxy-L-proline ester, and the remaining steps were the same as those of Example 2, to obtain 1-(tert-butyl 2-methyl(4S)-4-(4-amino-3-(7-methoxy-5-methylbenzothiophene-2-yl)-1H-pyrazole[3,4-d]py rimidine-1-yl)pyrrolidine-1,2-diformate. $[M+H]^+$: 539

Step 2: Preparation of (4S)-4-(4-amino-3-(7-methoxy-5-methylbenzothiophene-2-yl)-1H-pyrazole[3,4-d]pyrimidine -1-yl)-1-(tert-butyl formate)pyrrolidine-2-formic acid.

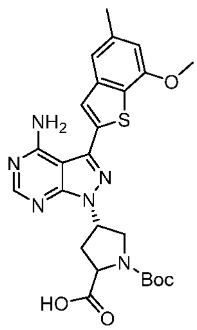

670 mg of 1-(tert-butyl 2-methyl(4S)-4-(4-amino-3-(7-methoxy-5-methylbenzothiophene-2-yl)-1H-pyrazole[3,4-d]py rimidine-1-yl)pyrrolidine-1,2-diformate obtained in Step 1 was dissolved in 6 ml of methanol and cooled in an ice bath. 3.8 ml of 4N sodium hydroxide aqueous solution was added dropwise, and the mixture was stirred at room temperature for 5 hours. The pH was adjusted to weak acidity with diluted hydrochloric acid, and the mixture was extracted with ethyl acetate and dried over anhydrous sodium sulfate. The reaction mixture was concentrated to dryness to give 530 mg of crude product.

Step 3: Preparation of tert-butyl (4S)-4-(4-amino-3-(7-methoxy-5-methylbenzothiophene-2-yl)-1H-pyrazole[3,4-d]pyrimidine -1-yl)-2-((2-(dimethylamino)ethyl)(methyl)formamide)pyrrolidine-1-formate

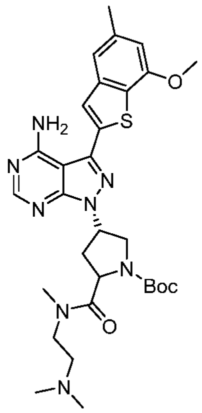

200 mg of (4S)-4-(4-amino-3-(7-methoxy-5-methylbenzothiophene-2-yl)-1H-pyrazole[3,4-d]pyrimidine -1-yl)-1-(tert-butyl formate)pyrrolidine-2-formic acid obtained in Step 2 , 50 μl of N,N,N'-trimethylethane-1,2-diamine, 252 mg of BOP and 0.12 ml of diisopropylethylamine were dissolved in 5 ml of acetonitrile and stirred at room temperature overnight. The next day, it was concentrated to dryness and then isolated by column chromatography (dichloromethane: methanol = 95:5 + 0.1% ammonia) to give 100 mg of yellow solid. $[M+H]^+$: 609

Step 4: tert-butyl (4S)-4-(4-amino-3-(7-methoxy-5-methylbenzothiophene-2-yl)-1H-pyrazole[3,4-d]pyrimidine -1-yl)-2-((2-(dimethylamino)ethyl)(methyl)formamide)pyrrolidine-1-formate obtained in Step 3 was used for replacing tert-butyl 3-(4-amino-3-(7-methoxy-5-methylbenzothiophene-2-yl)-1H-pyrazole[3,4-d]pyrimidine-1-yl )pyrrolidine-1-formate of Step 6 of Example 2, so as to obtain the title compound. $[M+H]^+$: 563

**Example 76**

**5-(7-methoxy-5-methylbenzothiophene-2-yl)-7-(2-morpholineethyl)-7H-pyrrole[2,3-d]p yrimidine-4-amine**

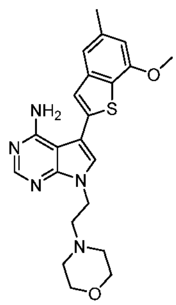

Step 1: Preparation of 2-morpholinoethyl mesylate

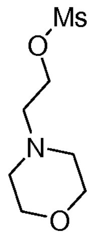

tert-butyl 3-hydroxypyrrolidine-1-formate of Step 3 in Example 2 was replaced with 2-morpholine ethanol to obtain 2-morpholinoethyl mesylate which was directly used in the next step.

Step 2: Preparation of 4-(2-(4-chloro-5-iodo-7H-pyrrole[2,3-d]pyrimidine-7-yl)ethyl)morpholine

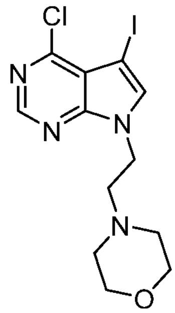

200 mg of 4-chloro-5-iodo-7H-pyrrole[2,3-d]pyrimidine and 480 mg of 2-morpholinolethyl mesylate obtained in Step 1 were dissolved in 2 ml of N,N-dimethylformamide, 456 mg of cesium carbonate was then added, and heated to 90 °C overnight under argon. The next day, water was added and the mixture was extracted with ethyl acetate. The organic phase was washed with saturated sodium chloride aqueous solution and dried over anhydrous sodium sulfate, filtered and concentrated, and the residue was isolated by column chromatography (dichloromethane: methanol = 97:3) to give 38 mg of yellow solids.
$^1$H NMR (300 MHz, $CDCl_3$) δ 8.60 (s, 1H), 7.50 (s, 1H), 4.37 (t, J = 6.1 Hz, 2H), 3.67 (s, 4H), 2.74 (t, J = 6.3 Hz, 2H), 2.50 (s, 4H).
Step 3: tert-butyl 3-(4-chloro-5-iodo-7H-pyrrole[2,3-d]pyrimidine-7-yl)pyrrolidine-1-formate of Step 9 in Example 1 was replaced with 4-(2-(4-chloro-5-iodo-7H-pyrrole[2,3-d]pyrimidine-7-yl)ethyl)morpholine obtained in Step 2, and the title compound can be prepared according to Step 9 to Step 10 in Example 1.
$^1$H NMR (300 MHz, $CDCl_3$) δ 8.33 (s, 1H), 7.25 (s, 1H), 7.22 (d, J = 2.3 Hz, 2H), 6.64 (s, 1H), 5.56 (s, 2H), 4.34 (t, J = 6.3 Hz, 2H), 4.00 (s, 3H), 3.74 - 3.65 (m, 4H), 2.79 (t, J = 6.3 Hz, 2H), 2.53 (s, 4H), 2.49 (s, 3H).

**Example 78 N-(3-((4-amino-3-(7-methoxy-5-methylbenzothiophene-2-yl)-1H-pyrazole[3,4-d]pyrimi dine-1-yl)methyl)phenyl)acrylamide**

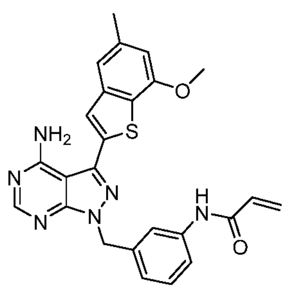

Step 1: Preparation of 1-(3-aminobenzyl)-3-iodo-1H-pyrazole[3,4-d]pyrimidine-4-amine

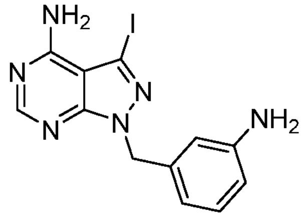

the raw material 4-chloro-5-iodo-7H-pyrrole[2,3-d]pyrimidine of Step 8 in Example 1 was replaced with 3-iodo-1H-pyrazole[3,4-d]pyrimidine-4-amine and 3-hydroxypyrrolidine-1-tert-butyl formate was replaced with (3-aminophenyl)methanol to give 1-(3-aminobenzyl)-3-iodo-1H-pyrazole[3,4-d]pyrimidine-4-amine. $[M+H]^+$: 367

Step 2: the raw material tert-butyl 3-(4-amino-5-iodo-7H-pyrrole[2,3-d]pyrimidine-7-yl)pyrrolidine-1-formate of Step 10 of Example 1 was replaced with 1-(3-aminobenzyl)-3-iodo-1H-pyrazole[3,4-d]pyrimidine-4-amine obtained in Step 1, and the remaining steps were the same as those in Example 1, and the title compound N-(3-((4-amino-3-(7-methoxy-5-methylbenzothiophene-2-yl)-1H-pyrazolo[3,4-d]pyrimidine-1-yl)methyl)phenyl)acrylamide were obtained. $^1H$ NMR (300 MHz, DMSO-d6) δ 10.14 (s, 1H), 8.31 (s, 1H), 7.67 (d, J = 11.4 Hz, 2H), 7.50 (s, 1H), 7.35 (s, 1H), 7.33 - 7.25 (m, 2H), 7.03 (d, J = 7.8 Hz, 1H), 6.85 (s, 1H), 6.39 (dd, J = 16.9, 9.9 Hz, 2H), 6.22 (d, J = 16.7 Hz, 1H), 5.72 (d, J = 9.9 Hz, 1H), 5.54 (s, 2H), 3.95 (s, 3H), 2.44 (s, 3H).

**Example 79**

**1-(3-(4-amino-5-(3,5-dimethoxyphenyl)-7H-pyrrole[2,3-d]pyrimidine-7-yl)pyrrolidine-1-yl)prop-2-en-1-one**

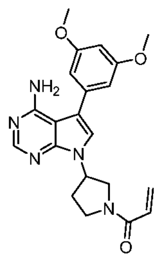

Step 1: the raw material (7-methoxy-5-methylbenzothiophene-2-yl)boronic acid of Step 10 of Example 1 was replaced with 3,5-dimethoxyphenylboronic acid pinacol ester, the remaining steps were the same as those in Example 1, and the title compound 1-(3-(4-amino-5-(3,5-dimethoxyphenyl)-7H-pyrrole[2,3-d]pyrimidine-7-yl)pyrrolidine-1-yl)p rop-2-en-1-one was obtained.

The preparation method of the following compounds was similar to that of compound 79.

| | | | |
|---|---|---|---|
| 82 | 1-(3-(4-amino-5-(pyridine-4-yl)-7H-pyrrole[2,3-d]pyrimidine-7-yl) pyrrolidine-1-yl)prop-2-en-1-one | 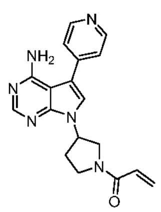 | $^1H$ NMR (300 MHz, $CDCl_3$) δ 8.68 (d, J = 4.0 Hz, 2H), 8.36 (s, 1H), 7.40 (d, J = 5.6 Hz, 2H), 7.07 (d, J = 9.0 Hz, 1H), 6.51 - 6.45 (m, 1H), 6.45 - 6.39 (m, 1H), 5.75 (m, 1H), 5.59 - 5.43 (m, 1H), 5.25 (s, 2H), 4.26 - 4.04 (m, 2H), 3.89 - 3.76 (m, 2H), 2.62 - 2.36 (m, 2H). |

(continued)

| | | | |
|---|---|---|---|
| 83 | 1-(3-(4-amino-5-(furan-3-yl)-7H-pyrrole[2,3-d]pyrimidine-7-yl) pyrrolidine-1-yl)prop-2-en-1-one | 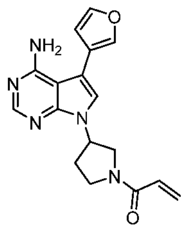 | $^1$H NMR (300 MHz, $CDCl_3$) δ 8.30 (s, 1H), 7.55 (d, J = 1.7 Hz, 2H), 6.91 (d, J = 9.1 Hz, 1H), 6.55 (s, 1H), 6.50 - 6.38 (m, 2H), 5.80 - 5.68 (m, 1H), 5.54 - 5.43 (m, 1H), 5.40 (s, 2H), 4.20 - 3.93 (m, 2H), 3.83 - 3.72 (m, 2H), 2.41 (m, 2H). |

**Example 80**

**1-(3-(4-amino-5-(3-amino-1H-indazol-6-yl)-7H-pyrrole[2,3-d]pyrimidine-7-yl)pyrrolidi ne-1-yl)prop-2-en-1-one**

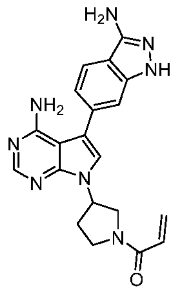

Step 1: Preparation of tert-butyl

3-(4-amino-5-(4-cyano-3-fluorophenyl)-7H-pyrrole[2,3-d]pyrimidine-7-yl)pyrrolidine-1-for mate

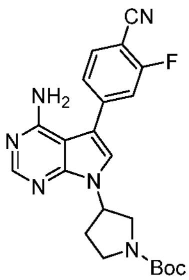

200 mg of tert-butyl 3-(4-amino-5-iodo-7H-pyrrole[2,3-d]pyrimidine-7-yl)pyrrolidine-1-formate, 91 mg of 4-cyano-3-fluorobenzeneboronic acid, 98 mg of sodium carbonate and 27 mg of tetrakistriphenylphosphine palladium were dissolved in 4 ml of a mixed solution of 1,4-dioxane and 1 ml of water. Air was replaced with argon, and the mixture was heated at 80 °C overnight. The reaction mixture was concentrated to dryness, and the residue was isolated by column chromatography (dichloromethane: methanol = 98:2) to give 150 mg of light yellow solids. $[M+H]^+$: 423

Step 2: Preparation of tert-butyl

3-(4-amino-5-(3-amino-1H-indazol-6-yl)-7H-pyrrole[2,3-d]pyrimidine-7-yl)pyrrolidine-1-for mate

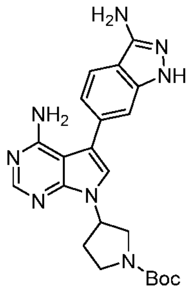

100 mg of above obtained tert-butyl 3-(4-amino-5-(4-cyano-3-fluorophenyl)-7H-pyrrole[2,3-d]pyrimidine-7-yl)pyrrolidine-1-for mate was dissolved in 4 ml of n-butanol, and 1 ml of hydrazine hydrate was added thereto, and refluxed under argon for 12 hours. The reaction mixture was concentrated to dryness, and isolated by column chromatography (dichloromethane :methanol = 98:2) to afford 62 mg of solids, tert-butyl 3-(4-amino-5-(3-amino-1H-indazol-6-yl)-7H-pyrrole[2,3-d]pyrimidine-7-yl)pyrrolidine-1-formate.

$^1$H NMR (300 MHz, $CDCl_3$) δ 8.33 (s, 1H), 7.65 (d, J = 8.2 Hz, 1H), 7.38 (s, 1H), 7.18 (d, J = 8.2 Hz, 1H), 7.03 (s, 1H), 5.49 (s, 1H), 5.30 (s, 2H), 4.20 (s, 2H), 3.97 - 3.87 (m, 1H), 3.70-3.50 (m, 3H), 2.50 - 2.38 (m, 1H), 2.35-2.23 (m,1H), 1.47 (s, 9H).

Step 3: tert-butyl 3-(4-amino-5-(7-methoxy-5-methylbenzothiophene-2-yl)-7H-pyrrole[2,3-d]pyrimidine-7-yl)p yrrolidine-1-formate of Step 11 in Example 1 was replaced with above obtained tert-butyl 3-(4-amino-5-(3-amino-1H-indazol-6-yl)-7H-pyrrole[2,3-d]pyrimidine-7-yl)pyrrolidine-1-for mate, and the title compound was obtained in the same procedure.

$^1$H NMR (300 MHz, $CDCl_3$) δ 8.34 (s, 1H), 7.65 (d, J = 8.2 Hz, 1H), 7.37 (s, 1H), 7.17 (d, J = 9.0 Hz, 1H), 7.00 (d, J = 8.2 Hz, 1H), 6.54 - 6.36 (m, 2H), 5.74 (s, 1H), 5.52 (s, 1H), 5.29 (s, 2H), 4.13 (m, 2H), 3.81 (s, 2H), 2.44 (s, 2H).

**Example 84**

**1-(3-(4-amino-3-(7-methoxy-5-methylbenzothiophene-2-yl)-1H-pyrazole[3,4-d]pyrimid ine-1-yl)pyrrolidine-1-yl)-2-chloroethane-1-one**

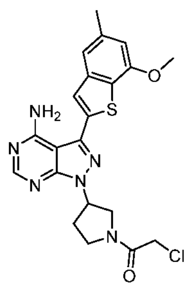

Step 1: The title compound was prepared by replacing acryloyl chloride in Step 7 of Example 2 with chloroacetyl chloride.

$^1$H NMR (300 MHz, $CDCl_3$) δ 8.38 (s, 1H), 7.52 (s, 1H), 7.27 (s, 1H), 6.68 (s, 1H), 5.89 (d, J = 7.8 Hz, 2H), 5.58 (s, 1H), 4.20 - 4.03 (m, 4H), 4.01 (s, 3H), 3.74 (s, 2H), 2.67 (s, 2H), 2.50 (s, 3H).

**Example 85**

**2-(7-(1-acryloylpyrrolidine-3-yl)-4-amino-7H-pyrrole[2,3-d]pyrimidine-5-yl)-7-methoxybenzothiophene-5-formic acid**

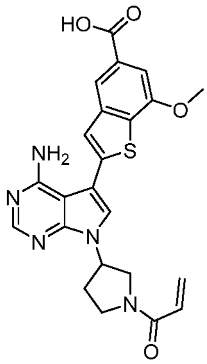

Step 1: Preparation of ethyl 7-hydroxybenzothiophene-5-formate

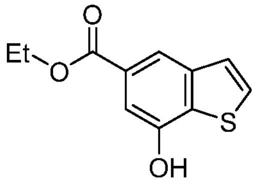

According to the literature J. AM. CHEM. SOC. 2007, 129, 14092-14099, ethyl 7-hydroxybenzothiophene-5-formate can be obtained in a similar manner.

Step 2: ethyl 7-methoxybenzothiophene-5-formate

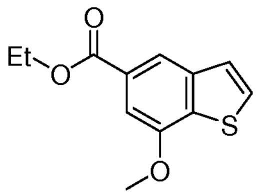

200 mg of above-obtained ethyl 7-hydroxybenzothiophene-5-formate, 150 mg of potassium carbonate and 155 mg of iodomethane were dissolved in acetonitrile, and the tube was sealed, heated and refluxed for 20 hours. The reaction mixture was filtered, and the filtrate was concentrated and isolated by column chromatography (petroleum ether 100%) to afford 230 mg of oily matter.
$^1$H NMR (300 MHz, $CDCl_3$) δ 8.19 (d, J = 1.0 Hz, 1H), 7.50 (d, J = 5.4 Hz, 1H), 7.43 (s, 1H), 7.40 (d, J = 5.4 Hz, 1H), 4.43 (q, J = 7.1 Hz, 2H), 4.06 (s, 3H), 1.44 (t, J = 7.1 Hz, 3H).

Step 3: Preparation of 7-methoxybenzothiophene-5-formic acid

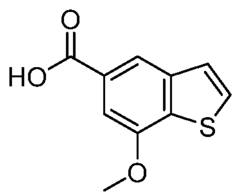

200 mg of ethyl 7-methoxybenzothiophene-5-formate obtained in the Step 2 was dissolved in a mixed solution of 4 ml of tetrahydrofuran, 1 ml of methanol and 1 ml of water, and 36 mg of lithium hydroxide was added thereto and stirred for 24 hours. The pH was adjusted to 2 with 2N diluted hydrochloric acid and the obtained mixture was extracted with ethyl acetate. The organic phase was concentrated and isolated by column chromatography (dichloromethane: methanol = 98:2) to give 165 mg of white solids, yield 93%.
$^1$H NMR (300 MHz, $CDCl_3$) δ 8.30 (s, 1H), 7.53 (d, J = 5.4 Hz, 1H), 7.49 (s, 1H), 7.44 (d, J = 5.3 Hz, 1H), 4.09 (s, 3H).
Step 4: 7-methoxy-5-methylbenzothiophene and n-butyllithium of Step 6 in Example 1 were replaced with 7-methoxybenzothiophene-5-formic acid and lithium diisopropylamide respectively, and the title compound can be prepared by the same procedure as in Example 1.
$^1$H NMR (300 MHz, DMSO-d6) δ 12.96 (s, 1H), 8.21 (s, 1H), 8.12 (s, 1H), 7.65 (d, J = 17.5 Hz, 1H), 7.55 (s, 1H), 7.40 (s, 1H), 6.70 - 6.47 (m, 3H), 6.17 (d, J = 16.4 Hz, 1H), 5.67 (d, J = 12.6 Hz, 1H), 5.37 (m, 1H), 4.12 (m, 1H), 4.03 (s, 3H), 3.90 (m, 1H), 3.79 - 3.68 (m,1H), 3.54 (m, 1H), 2.39 (m, 2H).

**Example 86**

**(S)-1-(3-(4-amino-5-(7-methoxybenzothiophene-2-yl)-7H-pyrrole[2,3-d]pyrimidine-7-yl )pyrrolidine-1-yl)prop-2-en-1-one**

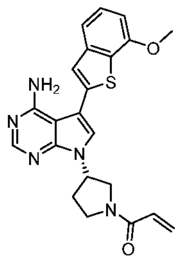

(7-methoxy-5-methylbenzothiophene-2-yl)boronic acid of Step 3 in Example 12 was replaced with (7-methoxybenzothiophene-2-yl)boric acid obtained in Example 22, and the title compound can be prepared.
$^1$H NMR (300 MHz, $CDCl_3$) δ 8.34 (s, 1H), 7.45 - 7.31 (m, 2H), 7.29 (d, J = 1.7 Hz, 1H), 7.12 (d, J = 10.7 Hz, 1H), 6.81 (d, J = 7.8 Hz, 1H), 6.51 - 6.45 (m, 1H), 6.45 - 6.39 (m, 1H), 5.81 - 5.68 (m, 1H), 5.58 (s, 2H), 5.51 (dd, J = 14.3, 6.9 Hz, 1H), 4.25 - 4.04 (m, 2H), 4.02 (s, 3H), 3.75-3.93 (m, 2H), 2.40-2.60 (m, 2H).

**Control compound**

(S)-1-(3-(4-amino-5-(quinolin-3-yl)-7H-pyrrole[2,3-d]pyrimidine-7-yl)pyrrolidine-1-yl)prop-2-en-1-one

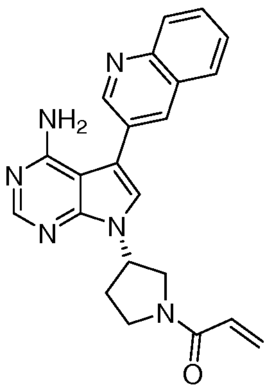

The preparation method was based on Example 1.
$^1$H NMR (300 MHz, DMSO-d) δ 9.05 (s, 1H), 8.35 (s, 1H), 8.21 (s, 1H), 8.11 - 7.96 (m, 2H), 7.76 (d, J = 7.2 Hz, 1H),

7.69 (d, J = 16.2 Hz, 1H), 7.66 - 7.59 (m, 1H), 6.72 - 6.53 (m, 1H), 6.39 (s, 2H), 6.17 (d, J = 16.5 Hz, 1H), 5.69 (t, J = 11.4 Hz, 1H), 5.51 - 5.31 (m, 1H), 4.22 - 4.09 (m, 0.5H), 4.05 - 3.86 (m,1.5H), 3.76 (dd, J = 12.4, 6.2 Hz, 1.5H), 3.55 (dd, J = 20.0, 7.9 Hz, 0.5H), 2.41 (dd, J = 16.2, 8.9 Hz, 2H).

**Effects of a compound on FGFR1, FGFR4 enzyme activity at molecular level**

1. Test method

The enzyme reaction substrate Poly(Glu, Tyr) 4:1 was diluted with potassium-free PBS (10 mM sodium phosphate buffer, 150 mM NaCl, pH 7.2-7.4) to 20μg/mL, an enzyme plate was coated at 125 μL/well. The plate was placed at 37 °C for 12-16 hours. The liquid in the well was discarded, the plate was washed, and the panel was washed three times with 200 μL/well of T-PBS (PBS containing 0.1% Tween-20) for 5 minutes each time. The enzyme plate was dried in an oven at 37 °C for 1-2 hours.

50 μL of ATP solution diluted with reaction buffer (50 mM HEPES pH 7.4, 50 mM $MgCl_2$, 0.5 mM $MnCl_2$, 0.2 mM $Na_3VO_4$, 1 mM DTT) was added to each well to a final concentration of 5 μM. The compound was diluted to a suitable concentration in DMSO, 1 μL/well or containing the corresponding concentration of DMSO (negative control well), and the FGFR1, FGFR4 kinase domain recombinant protein diluted in 49 μL of reaction buffer was added to initiate the reaction. Two control wells without ATP were required for each experiment. The reaction was carried out for 1 hour at 37 °C on a shaker (100 rpm). The plate was washed three times with T-PBS. Primary antibody, PY99 dilution was added at 100 μL/well and shaken at 37 °C for 0.5 hour. The plate was washed three times with T-PBS. Secondary antibody, horseradish peroxidase-labeled goat anti-mouse IgG dilution was added at 100 μL/well, and shaken at 37 °C for 0.5 hour. The plate was washed three times with T-PBS. 2 mg/ml OPD chromogenic solution was added at 100 μL/well (diluted with 0.1 M citric acid-sodium citrate buffer (pH=5.4) containing 0.03% $H_2O_2$) for 1-10 minutes at 25 °C in darkness. (Ultrasound was required for the dissolution of OPD, and the chromogenic solution needed to be prepared when used). 2 M $H_2SO_4$ was added at 50 μL/well to quench the reaction and a tunable wavelength microplate reader SPECTRA MAX 190 was used at a wavelength of 490 nm.

The inhibition rate of the sample was obtained by the following formula:

$$\text{inhibition}\ (\%) = \left(1-\frac{\text{OD of Compound} - \text{OD of Control well without ATP}}{\text{OD of Negative control} - \text{OD of Control well without ATP}}\right)\times 100\%$$

The $IC_{50}$ value was obtained by four-parameter regression using the software attached to the microplate reader.

2, Experimental results

Table 2. Inhibitory activities of compounds on FGFR1 and FGFR4 enzyme activities

| Compound in the examples | FGFR1 | FGFR4 |
|---|---|---|
| NO.1 | A | A |
| NO.2 | A | B |
| NO.3 | A | A |
| NO.4 | A | B |
| NO.5 | A | B |
| NO.6 | A | B |
| NO.7 | A | B |
| NO.8 | A | B |
| NO.9 | B | C |
| NO.10 | B | C |

(continued)

| Compound in the examples | FGFR1 | FGFR4 |
|---|---|---|
| NO.11 | A | B |
| NO.12 | A | A |
| NO.13 | A | A |
| NO.14 | A | A |
| NO.15 | A | B |
| NO.16 | A | B |
| NO.17 | A | B |
| NO.18 | A | B |
| NO.19 | A | B |
| NO.20 | B | B |
| NO.21 | A | B |
| NO.22 | A | A |
| NO.23 | B | C |
| NO.24 | A | B |
| NO.25 | A | A |
| NO.26 | A | A |
| NO.27 | A | B |
| NO.28 | A | A |
| NO.29 | A | B |
| NO.31 | A | A |
| NO.35 | A | A |
| NO.36 | B | B |
| NO.38 | A | C |
| NO.39 | A | A |
| NO.40 | A | A |
| NO.41 | A | B |
| NO.42 | A | A |
| NO.43 | A | B |
| NO.44 | A | A |
| NO.45 | B | A |
| NO.46 | B | A |
| NO.47 | B | A |
| NO.48 | B | A |
| NO.49 | B | A |
| NO.50 | C | C |
| NO.51 | A | A |
| NO.52 | A | A |
| NO.53 | A | A |

(continued)

| Compound in the examples | FGFR1 | FGFR4 |
|---|---|---|
| NO.54 | A | B |
| NO.55 | A | A |
| NO.56 | A | A |
| NO.57 | B | C |
| NO.58 | A | A |
| NO.59 | A | |
| NO.60 | A | |
| NO.61 | B | B |
| NO.62 | A | |
| NO.63 | C | C |
| NO.64 | C | B |
| NO.65 | B | B |
| NO.66 | C | |
| NO.68 | C | C |
| NO.69 | A | B |
| NO.70 | A | |
| NO.71 | A | |
| NO.72 | B | |
| NO.73 | A | B |
| NO.75 | A | |
| NO.76 | B | C |
| NO.77 | A | B |
| NO.78 | A | B |
| NO.79 | B | C |
| NO.80 | C | C |
| NO.81 | A | B |
| NO.82 | C | C |
| NO.83 | C | C |
| NO.84 | A | C |
| NO.85 | C | C |
| NO.86 | A | B |
| Control compound | C | B |

Table 3. Inhibitory activities of compounds on EGFR enzyme activities

| Example | EGFR |
|---|---|

(continued)

| Example | EGFR |
|---|---|
| NO.12 | >1000 nM |
| Wherein: A indicates that $IC_{50}$ is less than (≤) 10 nM<br>B indicates that $IC_{50}$ is less than (≤) 100 nM and greater than (>) 10nM<br>C indicates that $IC_{50}$ is greater than (>) 100 nM | |

It can be seen from Table 2 that the compounds of the present invention have a significant inhibitory effect on the FGFR1 and FGFR4 enzymes at the nM level. Since FGFR1 and FGFR2, 3 have high homology, it is expected that the compounds of the present invention have a significant inhibition effect on FGFR1-4.

In addition, the following conclusions can be drawn from Table 2:

i) By comparing compound No. 1-8 (containing substituted benzothiophene group and Y-E-Z structure is more complicated) and compounds NO. 9-10 and NO. 50 (containing substituted benzothiophene group, but Y-E-Z structure is simpler), it can be seen that when the Y-E-Z structure is more complicated, the compound has higher FGFR inhibitory activity; similar conclusions can also be obtained from the comparison results of the compound NO. 2 and the compound NO. 50;
ii) By comparing compound No. 13 (containing substituted benzothiophene group) and compound NO. 23 (containing substituted benzofuran group), it is known that when the compound contains a substituted benzothiophene group, it will have a higher FGFR inhibitory activity;
iii) By comparing compound NO.43 and compound NO.22, it can be seen that the substitution of the methoxy group on the benzothiophene group is advantageous for enhancing the FGFR inhibitory activity of the compound;
iv) By comparing compound NO.43 and compound NO.13, it is known that the substitution of methoxy and methyl groups on the benzothiophene group is advantageous for enhancing the FGFR inhibitory activity of the compound;
v) By comparing compound No. 12 (R1 is a substituted benzothiophene group) and a control compound (R1 is an unsubstituted quinolyl), it is known that, compared to a compound in which R1 is a substituted benzothiophene group, when R1 is an unsubstituted quinolyl, the inhibitory activity of the obtained compound on FGFR is remarkably lowered; in other words, when R1 is an unsubstituted quinolyl, the obtained compound has substantially no FGFR inhibitory activity.

As can be seen from Table 3, the compound NO. 12, which is a representative compound of the present invention, has substantially no EGFR inhibitory activity.

**Impact of compounds on SNU16 cell proliferation**

1. Test method

The inhibitory effect of the compound on SNU16 cells proliferation was examined by CCK-8 Cell Counting Kit (Dojindo). The specific steps are as follows: SNU16 cells in logarithmic growth phase were inoculated into a 96-well culture plate at a suitable density, 90 μL per well, and cultured overnight, different concentrations of compounds were added for 72 hr, and the solvent control group was set (negative control). After the cells were treated by the compound for 72 h, the effect of the compound on cell proliferation was detected by CCK-8 cell counting kit (Dojindo). 10 μL of CCK-8 reagent was added to each well and placed in a 37 °C incubator for 2-4 hours. The full-wavelength microplate reader SpectraMax 190 was used at a measure wavelength of 450 nm. The inhibition rate (%) of the compound on tumor cell growth was calculated by the following formula: inhibition rate (%) = (OD of negative control well - OD of administration well) / OD of negative control well × 100%. The IC50 value was obtained by four-parameter regression using the software attached to the microplate reader.

2, experimental results

Table 4. Effect of compounds on SNU16 cell proliferation

| Compound in the examples | SNU16 |
|---|---|
| NO.1 | A |
| NO.2 | A |
| NO.26 | C |
| NO.27 | C |
| NO.28 | C |
| NO.29 | A |
| NO.31 | A |
| NO.36 | C |
| NO.38 | A |
| NO.39 | C |
| NO.40 | C |
| NO.41 | B |
| Wherein: A indicates that $IC_{50}$ is less than (≤) 10 nM<br>B indicates that $IC_{50}$ is less than (≤) 100 nM and greater than (>) 10nM<br>C indicates that $IC_{50}$ is greater than (>) 100 nM | |

As can be seen from Table 4, the compounds of the present invention have a significant inhibitory effect on the reproductive activity of FGFR-dependent cell lines.

**The effect of compounds on the growth of a transplanted tumor in non-small cell lung cancer cells NCI-H1581 nude mouse experimental method**

The tumor tissue in the vigorous growth period was cut into about 1.5 mm3, and inoculated subcutaneously in the right axilla of nude mouse under aseptic conditions. The diameter of transplanted subcutaneously tumor in the nude mouse was measured with a vernier caliper. The mice were randomly divided into groups after the tumor was grown to an average volume of about 90 $mm^3$. The compound No. 12 obtained in Example 12 was orally administered once a day at a dose of 100 mg/kg and 20 mg/kg, respectively, for 14 days, and the growth inhibition effects of compound NO.12 on subcutaneously transplanted tumor in human lung cancer NCI-H1581 nude mouse were measured.

In the solvent control group, an equal amount of physiological saline was given. During the entire experiment, the diameter of the transplanted tumor was measured twice a week, and the body weight of the mice was weighed. The tumor volume (TV) is calculated as: $TV = 1/2 \times a \times b^2$, where a and b represent length and width, respectively. According to the measured results, the relative tumor volume (RTV) was calculated as: $RTV=V_t/V_0$, wherein $V_0$ was the tumor volume measured when the mouse was administered in cage (i.e. $d_0$), $V_t$ was the tumor volume at each measurement.

The evaluation index of anti-tumor activity was: 1) Relative tumor proliferation rate T/C (%), which was calculated as follows: $T/C(\%) = (T_{RTV}/C_{RTV}) \times 100\%$, $T_{RTV}$: RTV of treatment group; $C_{RTV}$: RTV of negative control group; 2) tumor volume growth inhibition rate GI%, which was calculated as follows: $GI\% = [1-(TVt-TV_0)/(CVt-CV_0)] \times 100\%$, TVt was the tumor volume measured every time in the treatment group; $TV_0$ was the tumor volume obtained when the treatment group was administered in cage; CVt was the tumor volume of the control group at each measurement; $CV_0$ was the tumor volume obtained when the control group was administered in cage; 3) tumor weight inhibition rate, which was calculated as follows: tumor weight inhibition rate% = $(Wc-W_T)/Wc \times 100\%$, Wc: tumor weight of the control group, $W_T$: tumor weight of the treatment group.

On the day of the end of the experiment, 2 hours after administration, blood samples and tumor tissues of each group were collected and preserved.

Experimental result

Table 5. Experimental therapeutic effect of compound NO.12 on transplanted tumor in human lung cancer NCI-H1581 nude mice

| Group | Dosage, administration mode | | Animal number | | Body weight (g) | | V ($mm^3$, mean±SD) | | RTV (mean±SD) | T/C (%) |
|---|---|---|---|---|---|---|---|---|---|---|
| | | | $d_0$ | $d_{14}$ | $d_0$ | $d_{14}$ | $d_0$ | $d_{14}$ | | |
| Solvent control | 0.2 ml/20 g qd/14 | po | 12 | 12 | 18.2 | 24.9 | 93±19 | 4951±1929 | 52.22±14-82 | |
| Compound N0.12 | 100 mg/kg qd/14 | po | 6 | 6 | 18.4 | 17.3 | 93±20 | 144±93 | 1.51±0.88* | 2.90 |
| | 20 mg/kg qd/14 | po | 6 | 6 | 16.9 | 21.0 | 92±22 | 1474±619 | 16.33±6.14* | 31.28 |

From table 5, the following conclusions can be obtained:

i) Compared with the negative control group, after being administrated with compound NO.12 for 14 days, the body weight change of the animals was -5.9% (100 mg/kg, po) and 24.2% (20 mg/kg, po), respectively, which were much smaller than 36.8% of the negative control group;
ii) Compared with the negative control group, after being administrated with compound NO.12 for 14 days, the tumor volume change of the animals was 54.8% (100 mg/kg, po) and 1500% (20 mg/kg, po), respectively, which were much smaller than 5223% of the negative control group;
iii) Compared with the negative control group, after being administrated with compound NO.12 for 14 days, the relative tumor volumes of the animals were 1.51 (100 mg/kg, po) and 16.33 (20 mg/kg, po), respectively, which were much smaller than 52.22 of the negative control group;
iv) Compared with the negative control group, after being administrated with compound NO.12 for 14 days, the relative tumor proliferation rate of the animals were 2.9% (100 mg/kg, po) and 31.28% (20 mg/kg, po), respectively.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I, or a stereoisomer, a geometric isomer, a tautomer thereof, a pharmaceutically acceptable salt thereof, a prodrug thereof and a hydrate or solvate thereof,

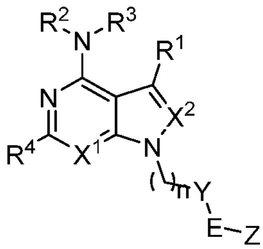

I

wherein $R^1$ is selected from the group consisting of substituted or unsubstituted 5-14 membered heteroaryl containing 1-3 heteroatoms selected from S, O, N and Se and substituted or unsubstituted 6-14 membered aryl, and the "substituted" refers to being substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halocycloalkyl), -$NR^6R^7$, halogen, -(C1-C6 alkylene)-L1, C(=O)$R^8$; and when $R^1$ is a N-containing 5-14 membered heteroaryl, $R^1$ is not a group selected from the group consisting of unsubstituted quinolyl and unsubstituted isoquinolyl;
$R^2$ and $R^3$ may be the same or different and are respectively independently selected from: H, substituted or unsubstituted C1-C6 alkyl, substituted or unsubstituted C3-C8 cycloalkyl, C(=O)$R^8$ and $S(=O)_2R^9$;
$R^4$ is selected from: H, substituted or unsubstituted C1-C6 alkyl, substituted or unsubstituted C3-C8 cycloalkyl, $NR^6R^7$, halogen, hydroxy, cyano, substituted or unsubstituted C1-C6 alkoxy and substituted or unsubstituted C1-C6 alkylthio;
$X^1$ and $X^2$ may be the same or different and are respectively independently selected from: N and $CR^{10}$; $R^{10}$ is selected from: H, substituted or unsubstituted C1-C6 alkyl, substituted or unsubstituted C3-C8 cycloalkyl, halogen, hydroxy, cyano, substituted or unsubstituted C1-C6 alkoxy and substituted or unsubstituted C1-C6 alkylthio;
n is 0, 1, 2, 3, 4 or 5;
Y is selected from: substituted or unsubstituted 3-10 membered heterocyclyl containing 1-3 heteroatoms selected from N, O or S, -$NR^6R^7$, substituted or unsubstituted C3-C8 cycloalkyl and -L2-(substituted or unsubstituted C6-C10 aryl)-, and the "substituted" refers to being independently substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halogenated cycloalkyl), $NR^{11}R^{12}$ , halogen, 4-10 membered heterocyclyl containing 1-3 heteroatoms selected from N, O or

S, -(C1-C6 alkylene)-L1, C(=O)$R^8$ and -Boc,
E is selected from the group consisting of none, C(=O), $S(=O)_2$, C(=S) and S(=O);
Z is selected from the group consisting of none, substituted or unsubstituted C2-C6 alkenyl, substituted or unsubstituted C2-C6 alkynyl, substituted or unsubstituted C1-C6 alkyl, substituted or unsubstituted C3-C8 cycloalkyl, -($R^{13}$)-N($R^{11}$)-($R^{14}$)-(substituted or unsubstituted C1-C6 alkoxy);
$R^6$ and $R^7$ may be the same or different and are respectively independently selected from H, substituted or unsubstituted C1-C6 alkyl, substituted or unsubstituted C3-C8 cycloalkyl, C(=O)$R^8$, -(C1-C6 alkylene)-L1, -(C1-C6 alkylene)-(substituted or unsubstituted 4-8 membered heterocyclyl containing 1-3 heteroatoms selected from N, O or S), -CN, halogen, -OH, -(C1-C6 alkylene)-(substituted or unsubstituted C4-C8 cycloalkyl), or -(C1-C6 alkylene)-L2-(C1-C6 alkylene)-(substituted or unsubstituted C1-C6 alkoxy);
$R^8$ and $R^9$ may be the same or different and are respectively independently selected from: H, hydroxy, substituted or unsubstituted C1-C6 alkyl, substituted or unsubstituted C3-C8 cycloalkyl, substituted or unsubstituted C1-C6 alkoxy, substituted or unsubstituted C2-C4 alkenyl, substituted or unsubstituted C2-C4 alkynyl, -L2-(C1-C6 alkylene)-LI and -N$R^{11}R^{12}$;
LI is selected from -OH, C1-C4 alkoxy, -N$R^{11}R^{12}$, or a 4-7 membered heterocyclyl having 1 or 2 N atoms;
L2 is selected from the group consisting of -N$R^{11}$- and -N (substituted or unsubstituted C3-C6 cycloalkyl);
for $R^2$ to $R^{10}$, $X^1$, $X^2$, E, Z and L2, the "substituted" refers to being independently substituted with one or more groups selected from the group consisting of C1-C6 alkyl, halogenated C1-C6 alkyl, C3-C8 cycloalkyl, halogenated C3-C8 cycloalkyl, hydroxy, C1-C6 alkoxy, halogenated C1-C6 alkoxy, -O-(C3-C8 cycloalkyl), -O-(C3-C8 halogenated cycloalkyl), N$R^{11}R^{12}$, halogen, -CN, 4-10 membered heterocyclyl containing 1-3 heteroatoms selected from N, O or S, -(C1-C6 alkylene)-LI and C(=O)$R^8$; wherein the 4-10 membered heterocyclyl may optionally have 1 to 3 substituents selected from the group consisting of C1-C3 alkyl, halogen, -OH, C1-C3 haloalkyl and C3-C4 cycloalkyl;
$R^{11}$ and $R^{12}$ are independently selected from H, C1-C6 alkyl, C1-C6 haloalkyl, C3-C6 cycloalkyl, C3-C6 halocycloalkyl, -CO(C2-C4 alkenyl), or -CO(C2-C4 alkynyl); or $R^{11}$ and $R^{12}$ together with an adjacent N constitute a 4-7 membered heterocyclyl containing 1-2 N atoms and 0-2 O or S atoms;
$R^{13}$ and $R^{14}$ are independently selected from substituted or unsubstituted C1-C6 alkylene or substituted or unsubstituted C2-C6 alkenylene.

2. The compound of formula I, or a stereoisomer, a geometric isomer, a tautomer thereof, a pharmaceutically acceptable salt thereof, a prodrug thereof and a hydrate or solvate thereof according to claim 1, wherein $R^1$ is a bicyclic group of the following formula:

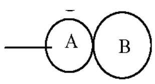
or
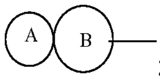
;

wherein ring A is a substituted or unsubstituted 5-membered heteroaryl ring; and ring B is a substituted or unsubstituted 6-membered heteroaryl ring or a substituted or unsubstituted phenyl.

3. The compound of formula I, or a stereoisomer, a geometric isomer, a tautomer thereof, a pharmaceutically acceptable salt thereof, a prodrug thereof and a hydrate or solvate thereof according to claim 1, wherein R1 is a substituted 5-14 membered heteroaryl or substituted 6-14 membered aryl.

4. The compound of formula I, or a stereoisomer, a geometric isomer, a tautomer thereof, a pharmaceutically acceptable salt thereof, a prodrug thereof and a hydrate or solvate thereof according to claim 1, wherein the compound of formula I is selected from the group consisting of:

| | | | |
|---|---|---|---|
| NO.1 | 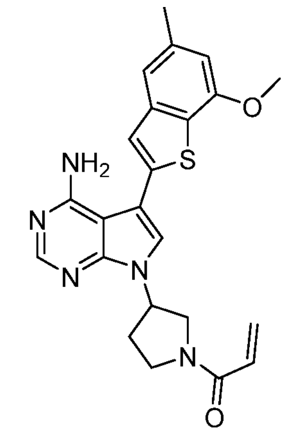 | NO.2 | 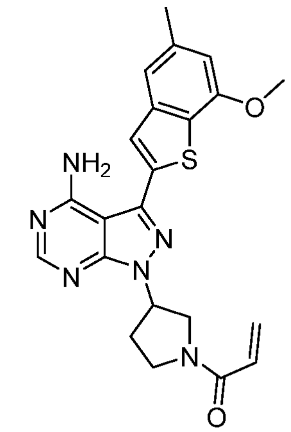 |
| NO.3 | 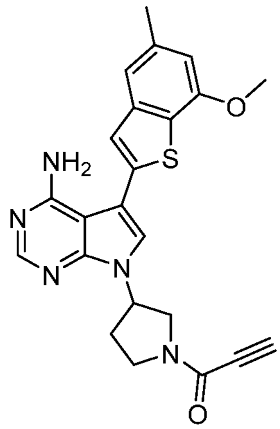 | NO.4 | 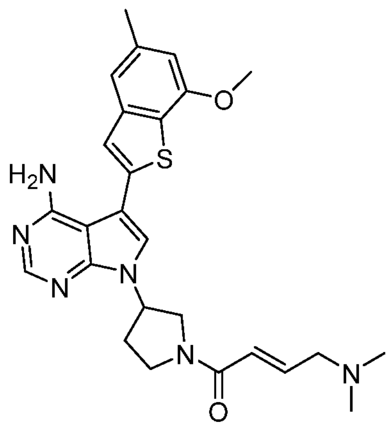 |
| NO.5 | 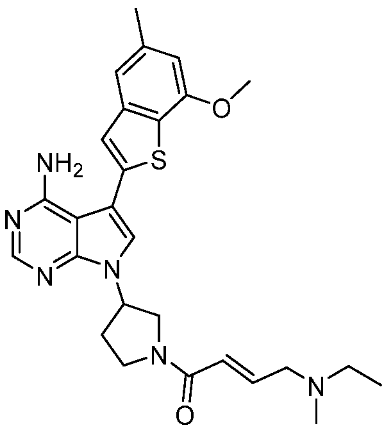 | NO.6 | 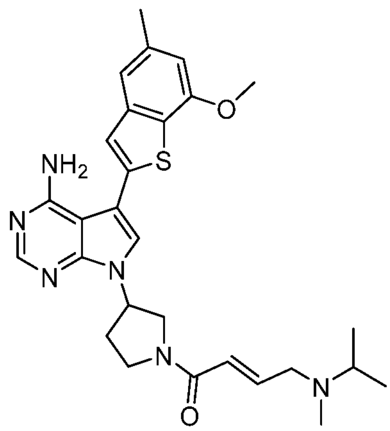 |

(continued)

| | | | |
|---|---|---|---|
| NO.7 | 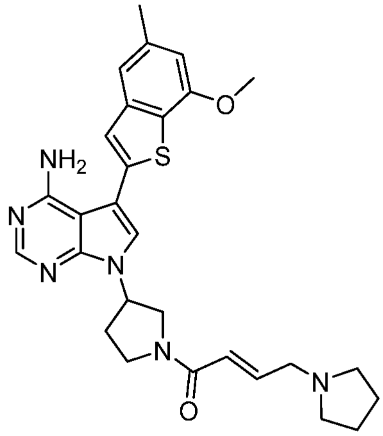 | NO.8 | 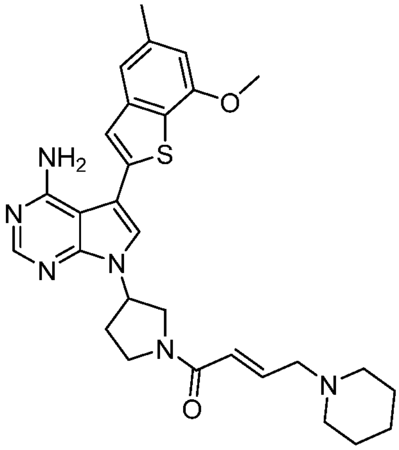 |
| NO.9 | 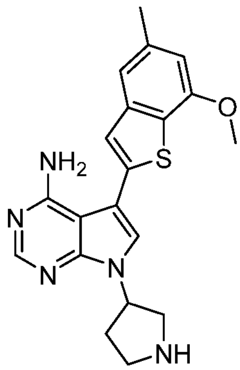 | NO.10 | 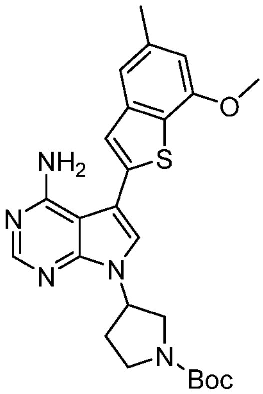 |
| NO.11 | 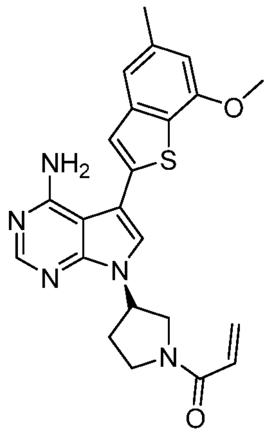 | NO.12 | 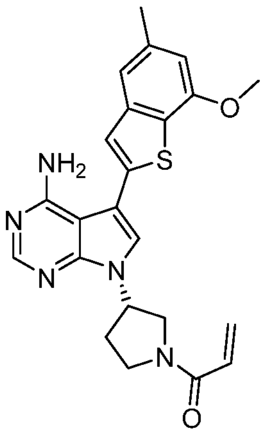 |

(continued)

| | | | |
|---|---|---|---|
| NO.13 | 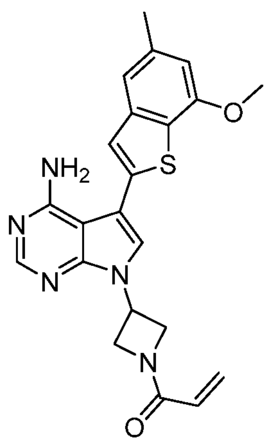 | NO.14 | 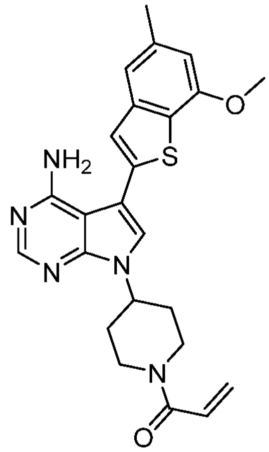 |
| NO.15 | 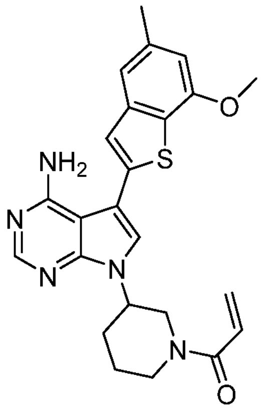 | NO.16 | 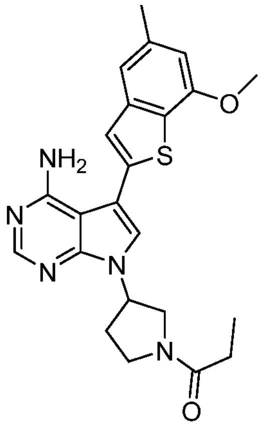 |
| NO.17 | 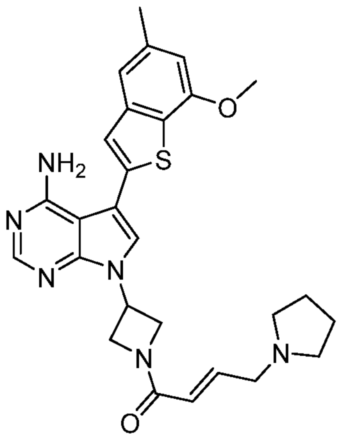 | NO.18 | 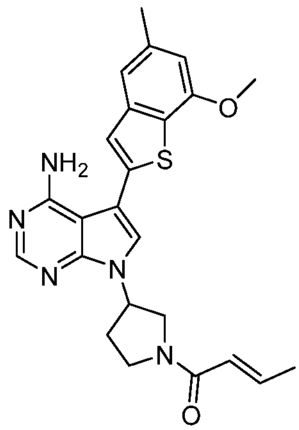 |

(continued)

| | | | |
|---|---|---|---|
| NO.19 | 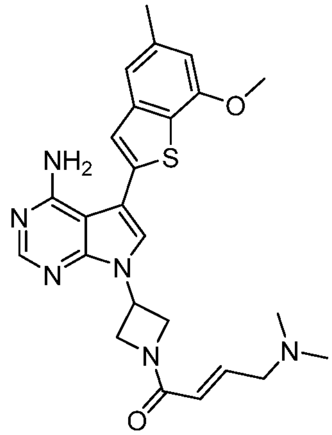 | NO.20 | 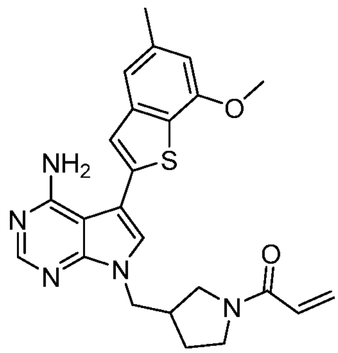 |
| NO.21 | 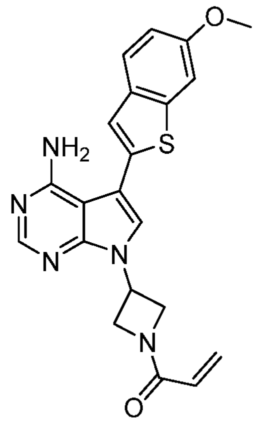 | NO.22 | 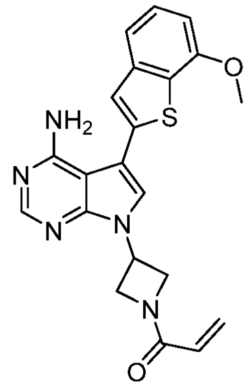 |
| NO.23 | 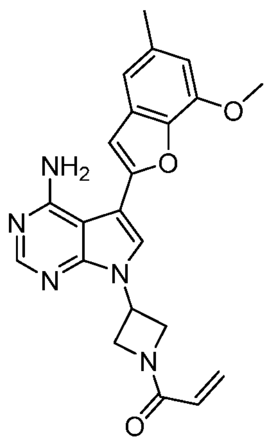 | NO.24 | 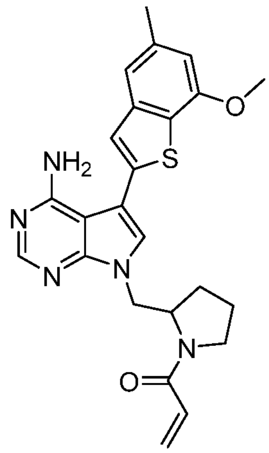 |

(continued)

| | | | |
|---|---|---|---|
| NO.25 | 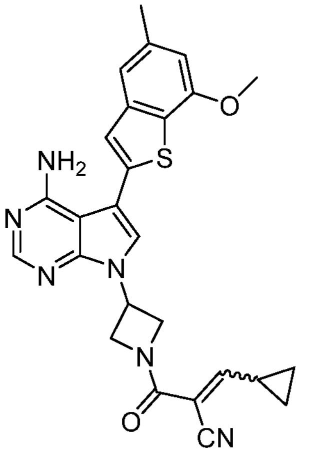 | NO.26 | 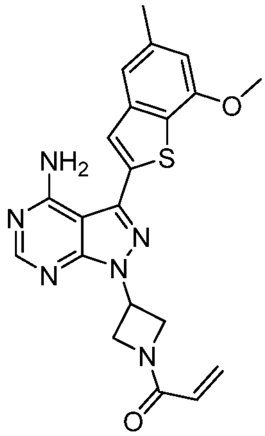 |
| NO.27 | 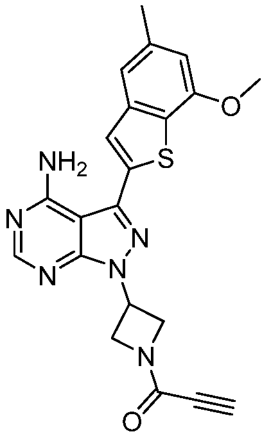 | NO.28 | 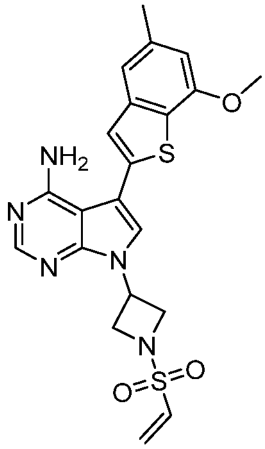 |
| NO.29 | 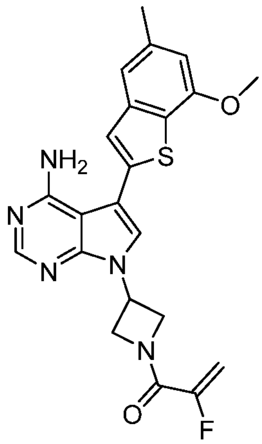 | NO.30 | 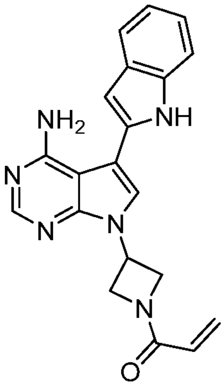 |

(continued)

| | | | |
|---|---|---|---|
| NO.31 | 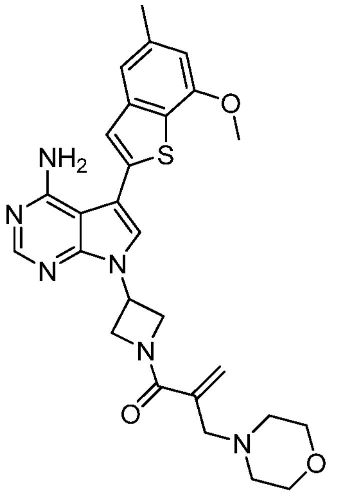 | NO.32 | 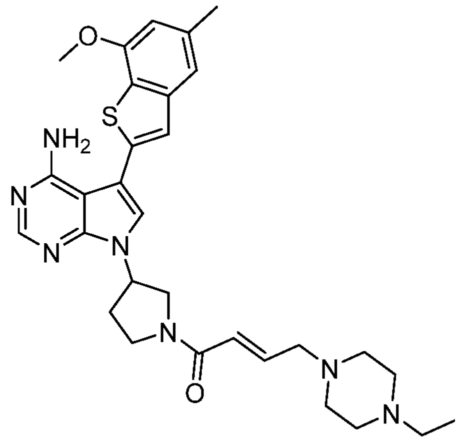 |
| NO.33 | 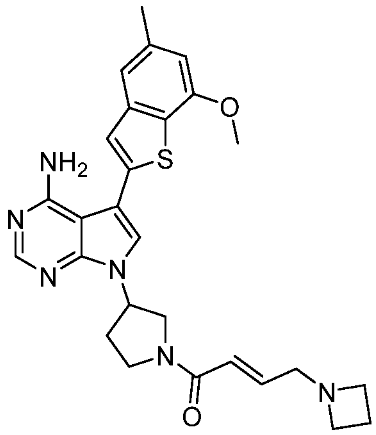 | NO.34 | 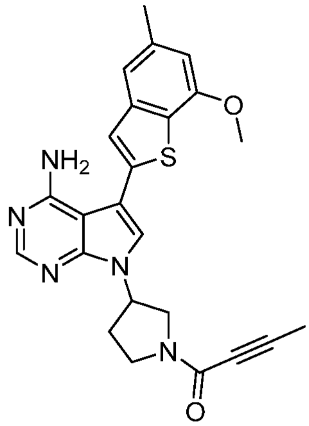 |
| NO.35 | 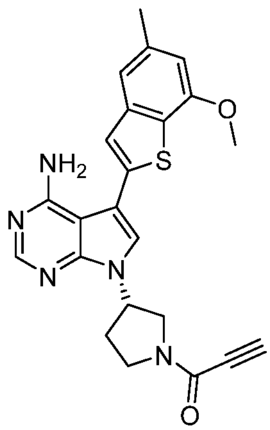 | NO.36 | 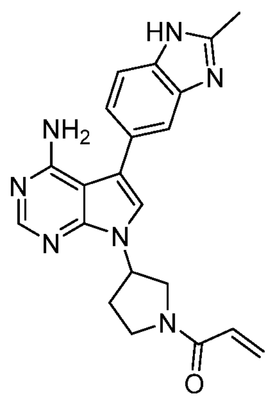 |

(continued)

| | | | |
|---|---|---|---|
| NO.37 | 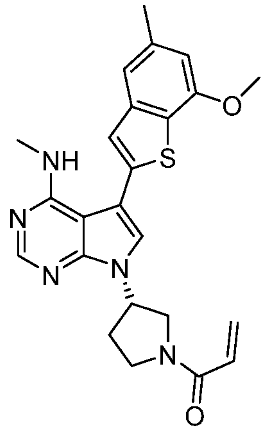 | NO.38 | 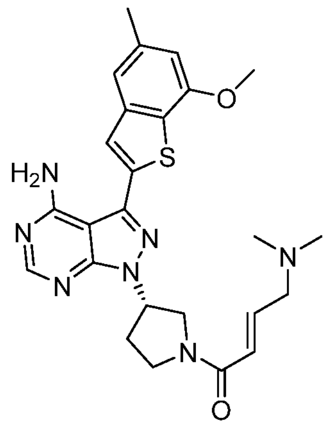 |
| NO.39 | 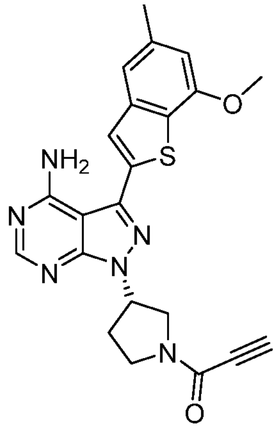 | NO.40 | 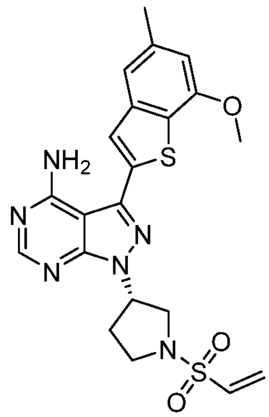 |
| NO.41 | 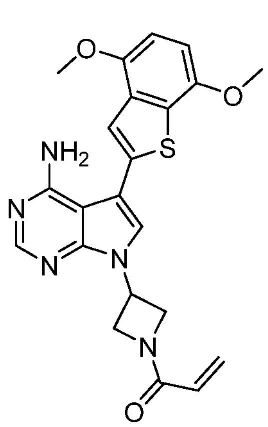 | NO.42 | 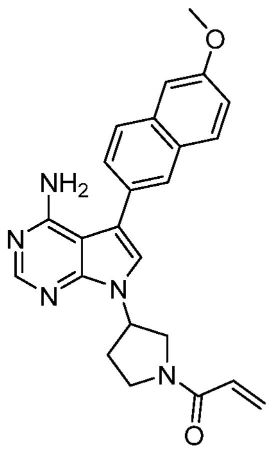 |

(continued)

| | | | |
|---|---|---|---|
| NO.43 | 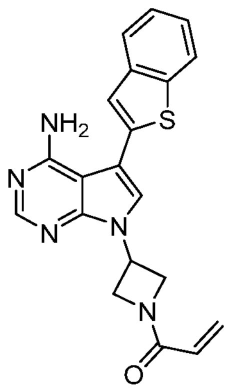 | NO.44 | 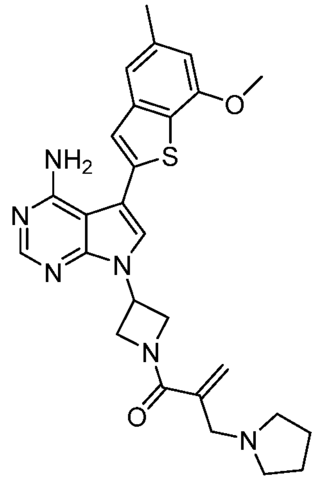 |
| NO.45 | 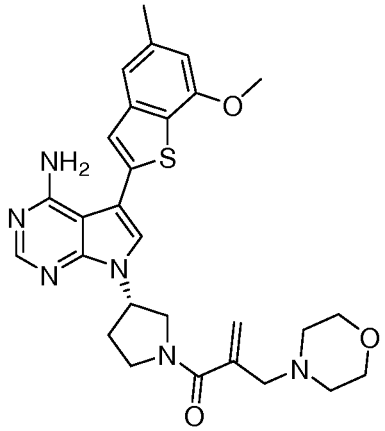 | NO.46 | 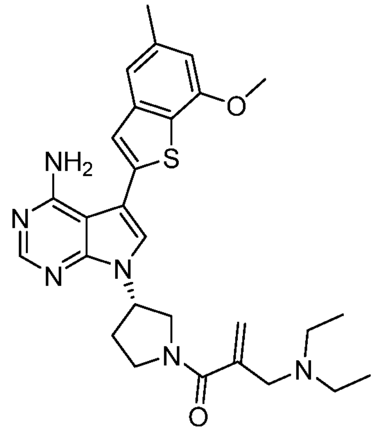 |
| NO.47 | 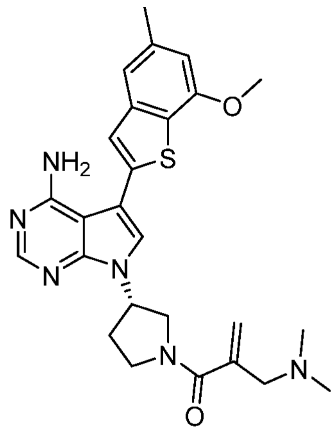 | NO.48 | 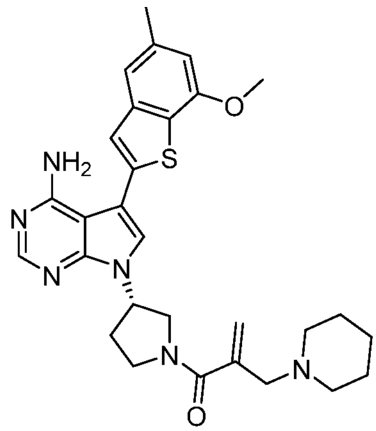 |

(continued)

| | | | |
|---|---|---|---|
| NO.49 | 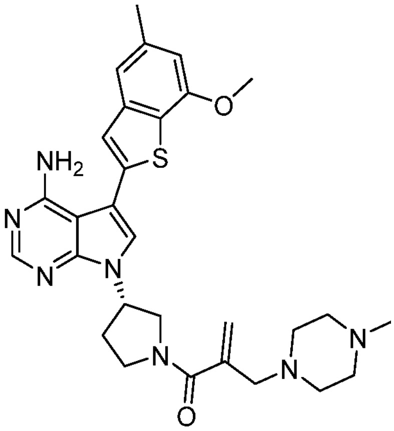 | NO.50 | 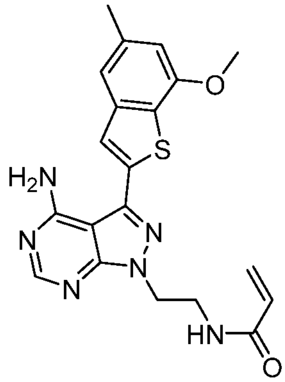 |
| NO.51 | 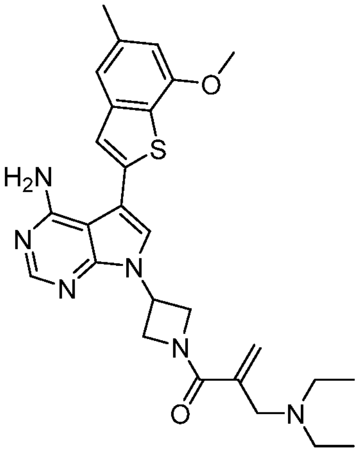 | NO.52 | 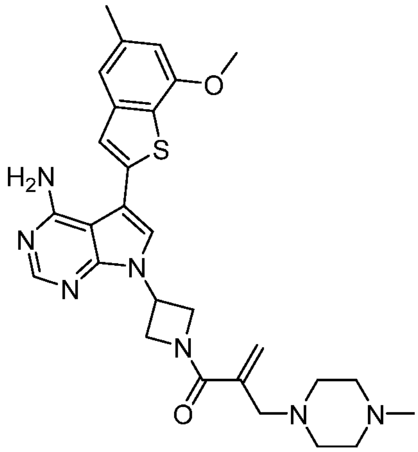 |
| NO.53 | 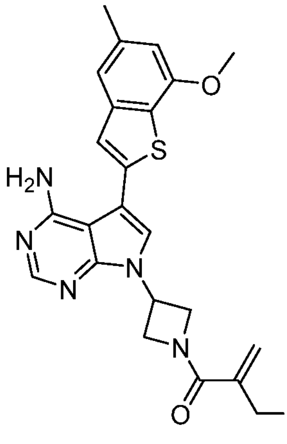 | NO.54 | 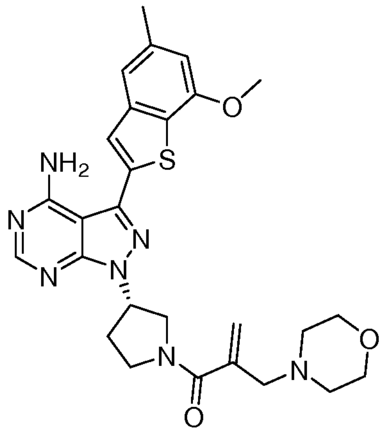 |

(continued)

| | | | |
|---|---|---|---|
| NO.55 | 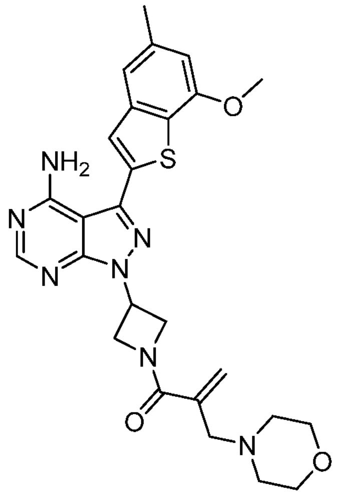 | NO.56 | 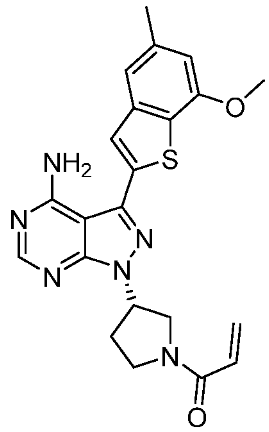 |
| NO.57 | 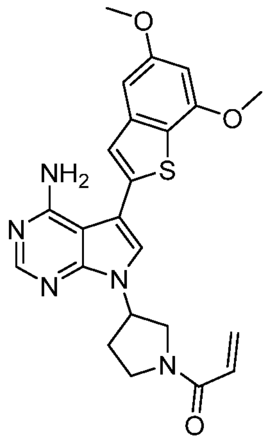 | NO.58 | 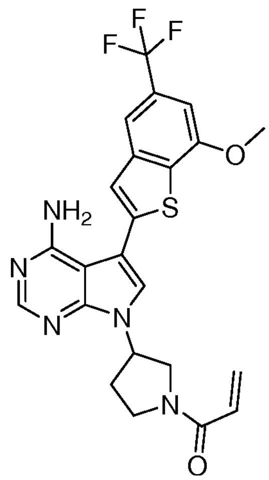 |
| NO.59 | 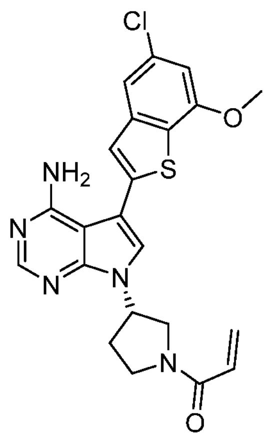 | NO.60 | 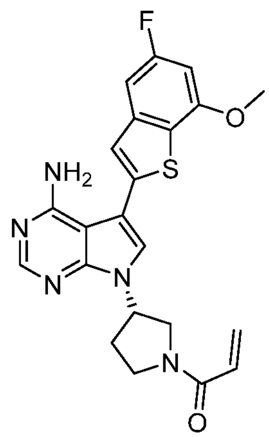 |

(continued)

| | | | |
|---|---|---|---|
| NO.61 | 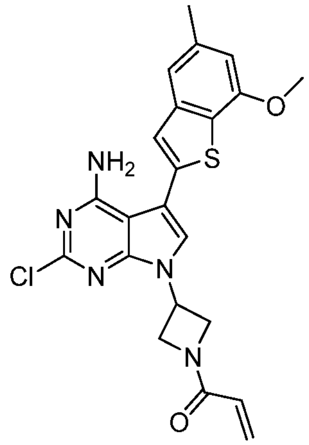 | NO.62 | 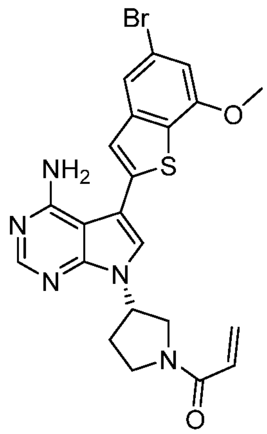 |
| NO.63 | 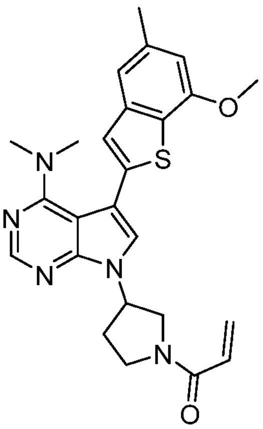 | NO.64 | 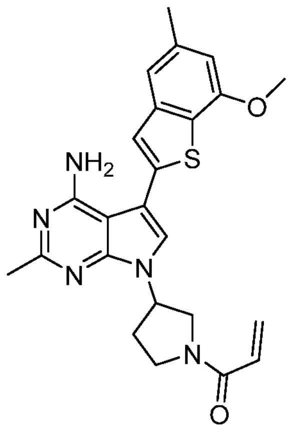 |
| NO.65 | 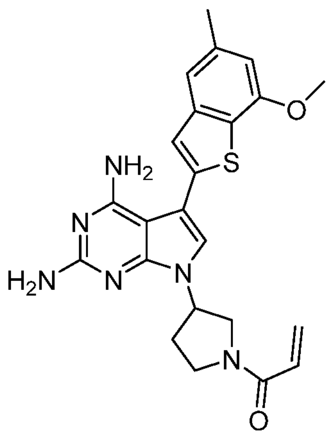 | NO.66 | 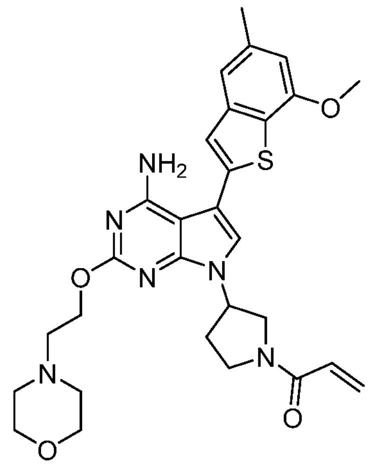 |

(continued)

| | | | |
|---|---|---|---|
| NO.67 | 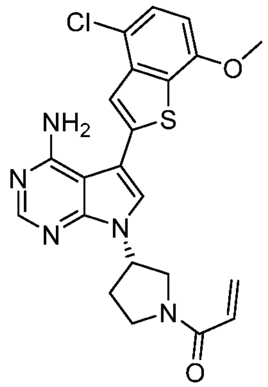 | NO.68 | 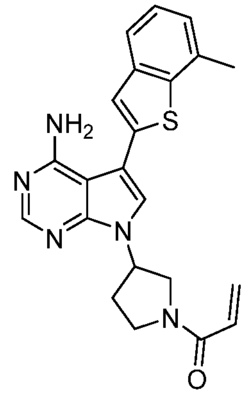 |
| NO.69 | 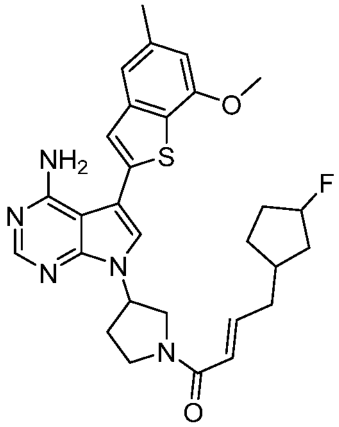 | NO.70 | 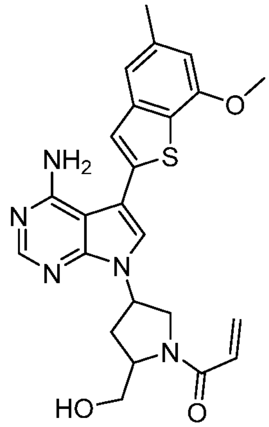 |
| NO.71 | 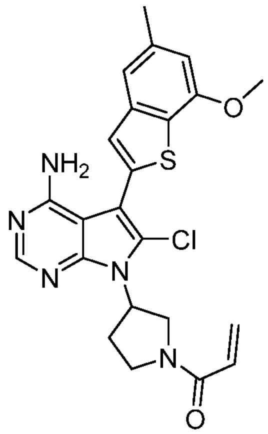 | NO.72 | 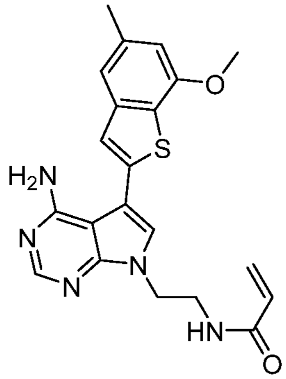 |

(continued)

| | | | |
|---|---|---|---|
| NO.73 | 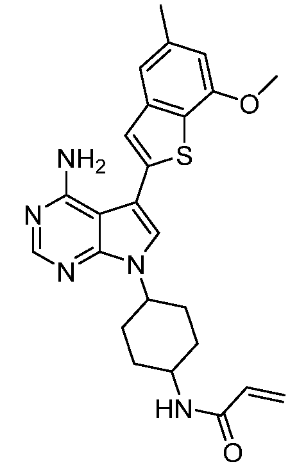 | NO.74 | 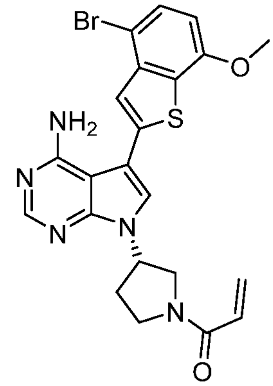 |
| NO.75 | 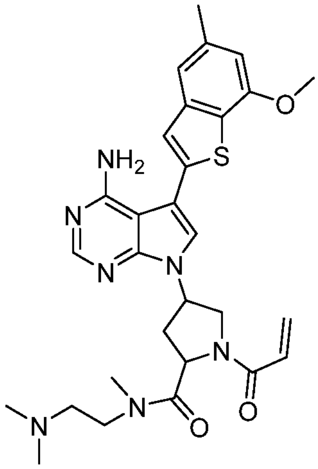 | NO.76 | 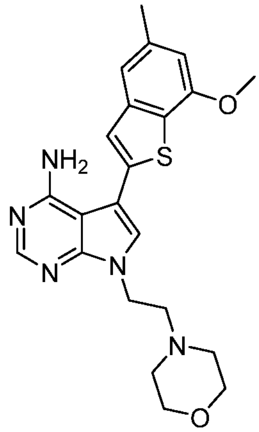 |
| NO.77 | 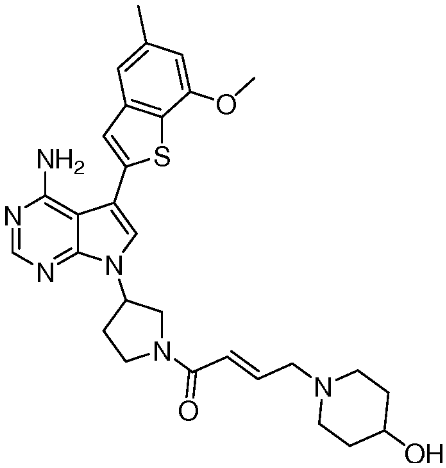 | NO.78 | 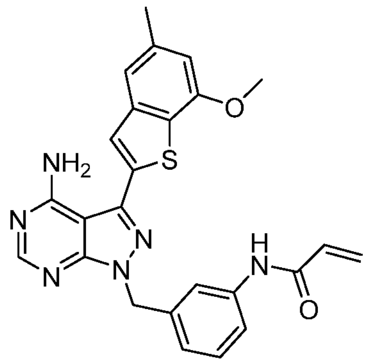 |

(continued)

| | | | |
|---|---|---|---|
| NO.79 | 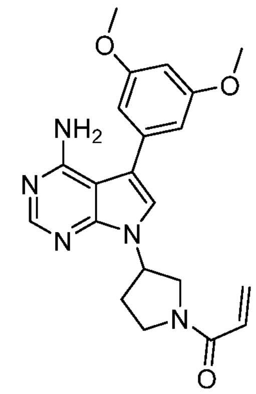 | NO.80 | 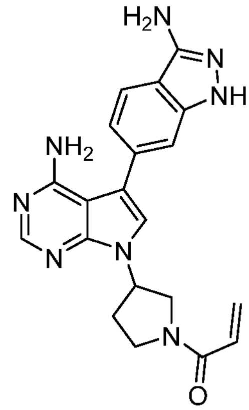 |
| NO.81 | 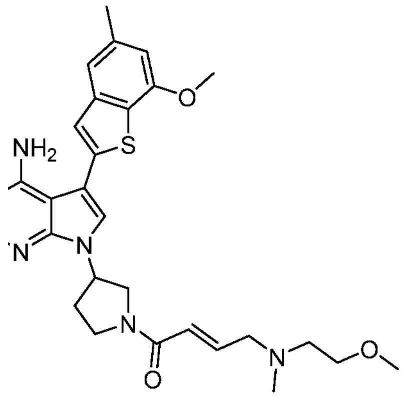 | NO.82 | 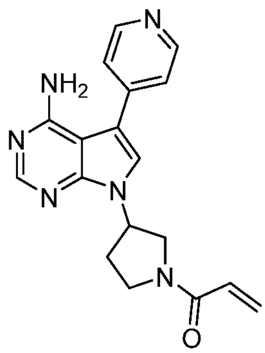 |
| NO.83 | 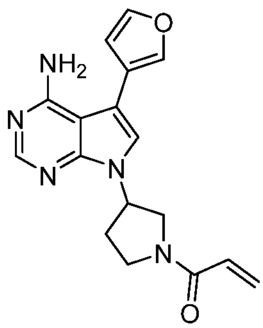 | NO.84 | 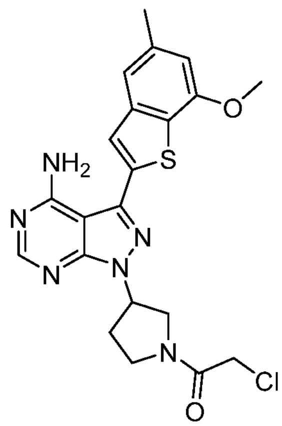 |

(continued)

| | | | |
|---|---|---|---|
| NO.85 | 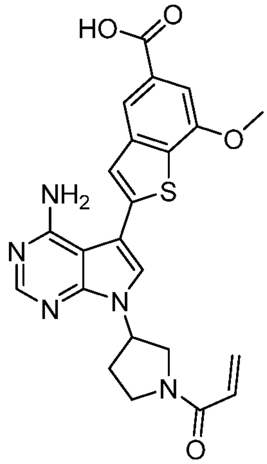 | NO.86 | 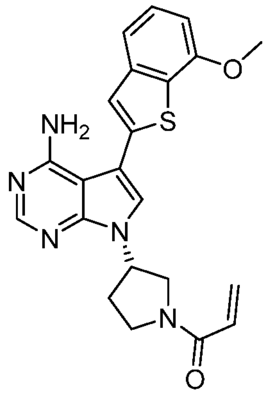 |

5. A pharmaceutical composition comprising a therapeutically effective amount of one or more of the compound or a stereoisomer, a geometric isomer, a tautomer thereof, a pharmaceutically acceptable salt thereof, a prodrug thereof and a hydrate or solvate thereof according to claim 1, and optionally a pharmaceutically acceptable carrier.

6. Use of the compound or a stereoisomer, a geometric isomer, a tautomer thereof, a pharmaceutically acceptable salt thereof, a prodrug thereof and a hydrate or solvate thereof according to claim 1 or the pharmaceutical composition according to claim 5 for the preparation of a medicament for preventing and/or treating a disease selected from the group consisting of:

a) tumor-related diseases; and
b) diseases associated with protein tyrosine kinase activity.

7. An FGFR inhibitor, wherein the FGFR inhibitor comprises an inhibitorically effective amount of one or more of the compound or a stereoisomer, a geometric isomer, a tautomer thereof, a pharmaceutically acceptable salt thereof, a prodrug thereof and a hydrate or solvate thereof according to claim 1.

8. A method for preparing the compound or a stereoisomer, a geometric isomer, a tautomer thereof, a pharmaceutically acceptable salt thereof, a prodrug thereof and a hydrate or solvate thereof according to claim 1, comprising the following step:

(i) in an inert solvent, subjecting a compound of formula 4 or a salt thereof and an acyl halide compound or an acid of formula 15 to a condensation reaction to form a compound of formula I;

| | | |
|---|---|---|
| Z-E-X 15 | + | 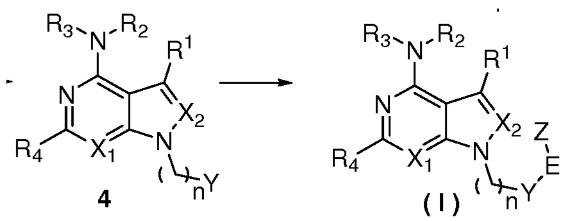 4 (I) |

in various formulas, $R^1$, $R^2$, $R^3$, $R^4$, n, $X_1$, $X_2$, Y, E and Z are as defined above;
X is selected from the group consisting of halogen and hydroxyl.

9. A method for preparing the pharmaceutical composition according to claim 5, comprising the step of: mixing a pharmaceutically acceptable carrier with the compound or a stereoisomer, a geometric isomer, a tautomer thereof, a pharmaceutically acceptable salt thereof, a prodrug thereof and a hydrate or solvate thereof according to claim 1 to form the pharmaceutical composition.

**10.** A method for non-diagnostically, non-therapeutically inhibiting FGFR activity, comprising the step of: administering to a patient in need thereof an inhibitory effective amount of the compound or a stereoisomer, a geometric isomer, a tautomer thereof, a pharmaceutically acceptable salt thereof, a prodrug thereof and a hydrate or solvate thereof according to claim 1 or the pharmaceutical composition according to claim 5.

# INTERNATIONAL SEARCH REPORT

International application No.
**PCT/CN2017/087222**

## A. CLASSIFICATION OF SUBJECT MATTER

C07D 487/04 (2006.01) i; A61K 31/519 (2006.01) i; A61K 31/5377 (2006.01) i; A61P 35/00 (2006.01) i

According to International Patent Classification (IPC) or to both national classification and IPC

## B. FIELDS SEARCHED

Minimum documentation searched (classification system followed by classification symbols)
C07D

Documentation searched other than minimum documentation to the extent that such documents are included in the fields searched

Electronic data base consulted during the international search (name of data base and, where practicable, search terms used)

CNABS, DWPI, REGISTY, CAPLUS, CNKI: SHANGHAI INSTITUTE OF MATERIA MEDICA, CHINESE ACADEMY OF SCIENCES; DUAN, Wenhu; GENG, Meiyu; WANG, Yuming; AI, Jing; FAN, Jun; DAI, Yang; DING, Jian; searching according to the structure of formula I, FDA, FGF, FGFR, pyrrolo [2, 3-d]pyrimidin+, benzothiophen

## C. DOCUMENTS CONSIDERED TO BE RELEVANT

| Category* | Citation of document, with indication, where appropriate, of the relevant passages | Relevant to claim No. |
|---|---|---|
| X | WO 0017202 A1 (BASF AG), 30 March 2000 (30.03.2000), a raw material in page 70, lines 9-10, embodiment 1, step f); a compound involved in page 182, paragraphs 21-22; an intermediate invovled in page 93, lines 26-27, embodiment 22, step e); and an intermediate invovled in claims 1 and 3, and page 96, lines 21-22, embodiment 23, step e) | 1, 3 |
| A | WO 0017202 A1 (BASF AG), 30 March 2000 (30.03.2000) | 2, 4-10 |
| X | WO 0172751 A1 (BASF AG), 04 October 2001 (04.10.2001), a raw material used in page 113, lines 12-14, embodiment 21; and a raw material used in 122, lines 21-22, embodiment 34, step a) | 1, 3 |
| A | WO 0172751 A1 (BASF AG), 04 October 2001 (04.10.2001) | 2, 4-10 |
| A | WO 02076986 A1 (ABBOTT GMBH & CO KG), 03 October 2002 (03.10.2002), claim 1, and embodiment | 1-10 |
| A | WO 2013087578 A1 (BAYER PHARMA AG), 20 June 2013 (20.06.2013), claim 1, and embodiment | 1-10 |
| X | WO 2005047289 A1 (PFIZER PROD INC.), 26 May 2005 (26.05.2005), page 48, lines 18-19, claim 7, the 3rd compound; and page 33, lines 36-37 | 1-3, 5 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 03 September 2017 (03.09.2017) | **14 September 2017 (14.09.2017)** |

| Name and mailing address of the ISA/CN: | Authorized officer |
|---|---|
| State Intellectual Property Office of the P. R. China<br>No. 6, Xitucheng Road, Jimenqiao<br>Haidian District, Beijing 100088, China<br>Facsimile No.: (86-10) 62019451 | **ZHAO, Zhenzhen**<br>Telephone No.: (86-10) **62086358** |

Form PCT/ISA/210 (second sheet) (July 2009)

## INTERNATIONAL SEARCH REPORT

International application No.
**PCT/CN2017/087222**

**C (Continuation). DOCUMENTS CONSIDERED TO BE RELEVANT**

| Category* | Citation of document, with indication, where appropriate, of the relevant passages | Relevant to claim No. |
|---|---|---|
| A | WO 2005047289 A1 (PFIZER PROD INC.), 26 May 2005 (26.05.2005) | 4, 6-10 |
| X | WO 2013070976 A1 (INTELLIKINE LLC), 16 May 2013 (16.05.2013), page 151, compound 9, page152, compound 8, page153, compound 26, page 154, compound 12, page 158, compound 26-27, page 159, compound 33, page 160, compound 36, page 161, compound 42, page 162, compound 48 and page 163, compound 51; and page 3, paragraph [0015] | 1-3, 5 |
| A | WO 2013087578 A1 (BAYER PHARMA AG), 20 June 2013 (20.06.2013) | 4, 6-10 |

Form PCT/ISA/210 (continuation of second sheet) (July 2009)

# INTERNATIONAL SEARCH REPORT
Information on patent family members

International application No.
**PCT/CN2017/087222**

| Patent Documents referred in the Report | Publication Date | Patent Family | Publication Date |
|---|---|---|---|
| WO 0017202 A1 | 30 March 2000 | CN 1326457 A | 12 December 2001 |
| | | ZA 200102201 B | 15 March 2002 |
| | | BR 9913888 A | 08 January 2002 |
| | | TR 200101395 T2 | 21 November 2001 |
| | | ZA 200102201 A | 15 March 2002 |
| | | DE 69928414 T2 | 03 August 2006 |
| | | IL 141867 D0 | 10 March 2002 |
| | | ID 28362 A | 17 May 2001 |
| | | HU 0200355 A2 | 29 June 2002 |
| | | EP 1114052 B1 | 16 November 2005 |
| | | JP 2002527359 A | 27 August 2002 |
| | | HU 0200355 A3 | 28 July 2004 |
| | | CZ 20010959 A3 | 12 December 2001 |
| | | AU 752474 B2 | 19 September 2002 |
| | | DE 69928414 D1 | 22 December 2005 |
| | | NO 20011357 D0 | 16 March 2001 |
| | | PL 347138 A1 | 25 March 2002 |
| | | AU 6047599 A | 10 April 2000 |
| | | NO 20011357 A | 14 May 2001 |
| | | SK 3852001 A3 | 04 March 2003 |
| | | CA 2344262 A1 | 30 March 2000 |
| | | AT 310001 T | 15 December 2005 |
| | | HK 1039325 A1 | 24 February 2006 |
| | | EP 1114052 A1 | 11 July 2001 |
| | | ES 2253930 T3 | 01 June 2006 |
| | | NZ 510587 A | 28 November 2003 |
| WO 0172751 A1 | 04 October 2001 | AU 4057000 A | 08 October 2001 |
| WO 02076986 A1 | 03 October 2002 | ZA 200306887 A | 13 September 2004 |
| | | EP 1379528 A1 | 14 January 2004 |
| | | ZA 200306887 B | 13 September 2004 |
| | | MX PA03008560 A | 30 June 2004 |
| | | CO 5570669 A2 | 31 October 2005 |
| | | NO 20034177 A | 21 November 2003 |
| | | CA 2440714 A1 | 03 October 2002 |
| | | BR 0205890 A | 29 June 2004 |
| | | EP 1379528 A4 | 07 December 2005 |
| | | NO 20034177 D0 | 19 September 2003 |
| | | CZ 20032837 A3 | 14 January 2004 |
| | | JP 4319412 B2 | 26 August 2009 |
| | | EC SP034773 A | 24 December 2003 |
| | | KR 20030088114 A | 17 November 2003 |
| | | US 7332497 B2 | 19 February 2008 |
| | | SK 13122003 A3 | 06 April 2004 |
| | | IL 157640 D0 | 28 March 2004 |
| | | PE 00472003 A1 | 07 February 2003 |
| | | CA 2440714 C | 10 August 2010 |
| | | PL 363946 A1 | 29 November 2004 |
| | | BG 108268 A | 30 April 2005 |
| | | US 2004006083 A1 | 08 January 2004 |
| | | UY 27222 A1 | 31 October 2002 |

Form PCT/ISA/210 (patent family annex) (July 2009)

**INTERNATIONAL SEARCH REPORT**
Information on patent family members

International application No.
**PCT/CN2017/087222**

| Patent Documents referred in the Report | Publication Date | Patent Family | Publication Date |
|---|---|---|---|
| | | JP 2005501811 A | 20 January 2005 |
| | | CN 101426792 A | 06 May 2009 |
| | | HU 0400267 A2 | 30 August 2004 |
| WO 2013087578 A1 | 20 June 2013 | PT 2791140 T | 20 September 2016 |
| | | GT 201400113 A | 25 August 2015 |
| | | CU 20140065 A7 | 27 November 2014 |
| | | US 9206184 B2 | 08 December 2015 |
| | | AU 2012350750 B2 | 03 August 2017 |
| | | TW 201339162 A | 01 October 2013 |
| | | SG 11201402325Q A | 26 September 2014 |
| | | CO 7030961 A2 | 21 August 2014 |
| | | HK 1205123 A1 | 11 December 2015 |
| | | HU E029582 T2 | 28 March 2017 |
| | | IN 4310CHN2014 A | 04 September 2015 |
| | | US 2014336173 A1 | 13 November 2014 |
| | | US 2013158000 A1 | 20 June 2013 |
| | | UY 34484 A | 31 July 2013 |
| | | PH 12014501355 A1 | 22 September 2014 |
| | | RS 55144 B1 | 30 December 2016 |
| | | CR 20140288 A | 14 July 2014 |
| | | EP 2791140 B1 | 15 June 2016 |
| | | CN 104245700 B | 16 November 2016 |
| | | DK 2791140 T3 | 19 September 2016 |
| | | HR P20161130 T1 | 18 November 2016 |
| | | CN 104245700 A | 24 December 2014 |
| | | DO P2014000127 A | 15 August 2014 |
| | | CL 2014001547 A1 | 24 October 2014 |
| | | KR 20140103328 A | 26 August 2014 |
| | | JP 6050829 B2 | 21 December 2016 |
| | | LT 2791140 T | 10 October 2016 |
| | | JP 2015500307 A | 05 January 2015 |
| | | IL 232611 D0 | 30 June 2014 |
| | | AU 2017206140 A1 | 03 August 2017 |
| | | CU 20160039 A7 | 31 August 2016 |
| | | ES 2591203 T3 | 25 November 2016 |
| | | CA 2859133 A1 | 20 June 2013 |
| | | AU 2012350750 A1 | 12 June 2014 |
| | | CY 1118112 T1 | 28 June 2017 |
| | | NZ 625073 A | 29 July 2016 |
| | | EA 201400707 A1 | 28 November 2014 |
| | | TW I567076 B | 21 January 2017 |
| | | SI 2791140 T1 | 28 October 2016 |
| | | EP 2791140 A1 | 22 October 2014 |
| | | PE 18552014 A1 | 26 November 2014 |
| | | AR 089207 A1 | 06 August 2014 |
| | | MX 2014006905 A | 08 September 2014 |
| WO 2005047289 A1 | 26 May 2005 | CA 2546192 A1 | 26 May 2005 |
| | | US 2005130994 A1 | 16 June 2005 |
| | | MX PA06005578 A | 11 August 2006 |
| | | EP 1687309 A1 | 09 August 2006 |

Form PCT/ISA/210 (patent family annex) (July 2009)

**INTERNATIONAL SEARCH REPORT**
Information on patent family members

International application No.
**PCT/CN2017/087222**

| Patent Documents referred in the Report | Publication Date | Patent Family | Publication Date |
|---|---|---|---|
| | | JP 2007511596 A | 10 May 2007 |
| | | BR PI0416656 A | 16 January 2007 |
| | | US 7435739 B2 | 14 October 2008 |
| | | CA 2546192 C | 06 April 2010 |
| WO 2013070976 A1 | 16 May 2013 | US 2017209448 A1 | 27 July 2017 |
| | | JP 2014532768 A | 08 December 2014 |
| | | EP 2776441 A1 | 17 September 2014 |
| | | US 2014377285 A1 | 25 December 2014 |
| | | JP 6114296 B2 | 12 April 2017 |
| | | CN 106924741 A | 07 July 2017 |
| | | CN 106994126 A | 01 August 2017 |
| | | HK 1201828 A1 | 11 September 2015 |
| | | EP 2776441 A4 | 08 April 2015 |
| | | CA 2854926 A1 | 16 May 2013 |
| | | CN 104080786 A | 01 October 2014 |
| | | US 2016287597 A1 | 06 October 2016 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011055215 A


### Non-patent literature cited in the description


- **SAMBROOK et al.** Molecular Cloning: Laboratory Manual. Cold Spring Harbor Laboratory Press, 1989
- *Adv. Synth. Catal.,* 2015, vol. 357, 2205-2212
- *J. AM. CHEM. SOC.,* 2007, vol. 129, 14092-14099